# EUROPÄISCHE PATENTSCHRIFT

(11) **EP 4 225 121 B1**
(45) Veröffentlichungstag und Bekanntmachung des Hinweises auf die Patenterteilung: **10.04.2024**
(21) Anmeldenummer: 22737410.5
(22) Anmeldetag: 15.06.2022
(51) Int. Cl.: A61B 1/005, A61B 1/018

(54) **ABKLAPPMECHANISMUS FÜR EIN ENDOSKOP**
FOLD-DOWN MECHANISM FOR AN ENDOSCOPE
MÉCANISME DE PLIAGE POUR ENDOSCOPE

(30) Priorität: 15.06.2021 DE 102021115475
(43) Veröffentlichungstag der Anmeldung: 16.08.2023
(73) Patentinhaber: Bob, Konstantin, 69469 Weinheim (DE)
(72) Erfinder: GRÜNDL, Andreas, 82319 Starnberg (DE); BOB, Konstantin, 69469 Weinheim (DE)
(74) Vertreter: Winter, Brandl - Partnerschaft mbB
(86) Internationale Anmeldenummer: PCT/EP2022/066401
(87) Internationale Veröffentlichungsnummer: WO 2022/263561

(56) Entgegenhaltungen:
- WO-A1-2018/130978
- US-A1- 2008 051 802
- US-A1- 2021 137 354

## Beschreibung

Die vorliegende Offenbarung betrifft einen Abklappmechanismus und insbesondere ein Endoskop mit einem solchen Abklappmechanismus zur abklapp- oder abwinkelbaren Halterung eines Endoskopkopfes am distalen (zum Körper eines Patienten hingewandten) Ende des Endoskops mit einer Anzahl axial aufeinanderfolgender und mittels zumindest eines Betätigungselements aktiv gegeneinander winkelverstellbarer, im Wesentlichen zylindrischer (oder elliptischer) Segmente, welche in der Seitenansicht zwei im Wesentlichen keilförmig zueinander ausgerichtete Stirnseiten und einen Zylindermantel aufweisen, der an einem Zylindermantelabschnitt mit maximaler axialer Länge einen Keilrückenabschnitt definiert, und welche in Endoskop-Längsrichtung zumindest einen Arbeitskanal für das Ein-/Hindurchführen eines chirurgischen Instruments ausbilden.

### Hintergrund der Offenbarung

Endoskope sind medizinische Arbeitsgeräte zur visuellen Exploration von Hohlräumen in einem Patientenkörper. Sie weisen grundsätzlich optische Einrichtungen am distalen, d.h. Anwender-abgewandten bzw. Patientenkörper-zugewandten Endoskopende (auch Endoskopkopf genannt) sowie optional einen Arbeitskanal auf, der sich ausgehend von einem proximalen (Anwender-zugewandten) Endoskopabschnitt oder extrakorporalen Endoskopgriff durch einen (daran sich anschließenden) flexiblen oder biegesteifen (insbesondere starren) Endoskopschaft hindurch bis zum Endoskopkopf erstreckt und das extrakorporale Einführen und Verwenden eines medizinischen Instruments wie z.B. einer Zange, Schere, Nadel, Schlinge, Messer und dergleichen ermöglicht.

Derartige Endoskope können wahlweise mit zusätzlichen Fähigkeiten versehen werden, etwa indem am distalen Endoskopende/Endoskopkopf eine Kappe oder Hülse auf den Endoskopkopf radial außenseitig aufgesetzt wird, die mit bestimmten Funktionen/Funktionselementen versehen oder ausgerüstet ist, wodurch das Endoskop nicht nur zur Exploration und/oder als Zugang für therapeutische Anwendungen sondern auch selbst als ein minimalinvasives Instrument zur Ausführung eines chirurgischen Vorgangs genutzt werden kann. Alternativ hierzu ist es aber auch vorgesehen, spezielle Endoskope für ganz bestimmte medizinische Anwendungen mit derartigen Fähigkeiten integral auszustatten, wobei derartige Spezialanfertigungen jedoch nur für diese besondere Anwendung geeignet sind.

Verschiedene diagnostische und/oder therapeutische Verfahren erfordern beispielsweise eine Bildgebung und/oder bei Bedarf therapeutische Techniken am Gallen- und/oder Bauchspeicheldrüsengang sowie den Lebergängen des Patienten. Da die Vatersche Papille, welche den gemeinsamen Austritt von Gallen- und Bauchspeicheldrüsengang in das Duodenum bildet, seitlich in das Duodenum hineinragt, sind herkömmliche prograde (in Endoskop-Längsrichtung blickende) Endoskope für das Heran- und Einführen von chirurgischen Instrumenten in den Gallenkanal ungeeignet, da nicht genügend Schwenkraum im engen Duodenum (Durchmesser 3 bis 4 cm) vorhanden ist, um deren prograde Optik sowie den Arbeitskanal in eine seitwärts blickende Position auszurichten, da ein typischer Biegeradius solcher Geräte bei etwa 6 cm liegt. Dieser Biegeradius bzw. diese Verschwenkung wird bei herkömmlichen prograden Endoskopen häufig durch sogenannte "Deflectings" (dem Endoskopkopf unmittelbar in Richtung proximal vorgeordnete, aktiv abkrümmbare Endoskopschaft-Abschnitte) erzielt.

### Stand der Technik

Aus dem Stand der Technik (bspw. der US 2010/228086 A) sind zu diesem Zweck speziell angefertigte Duodenoskope bekannt, welche eine laterale (seitwärts blickende) oder retrograde (rückblickende) Optik (auch "Seitoptik" genannt) sowie einen seitwärts gerichteten bzw. sich öffnenden Arbeitskanal aufweisen. Alternativ hier kann an einem prograden (in Axialrichtung sich öffnenden) Ausgang des Arbeitskanals solcher Duodenoskope ein sogenannter Albarranhebel vorgesehen sein, der durch manuell bewirktes Verschwenken ein gezieltes Führen/Umlenken eines im Arbeitskanal geführten Instruments ermöglicht. Durch die seitwärts gerichtete Anordnung der Funktionseinheiten insbesondere Optik und Beleuchtung am Endoskopkopf wird eine Bildgebung und Behandlung im Bereich des Duodenums unter optimaler Nutzung des zur Verfügung stehenden Raums ermöglicht.

Derartige Endoskope mit Seitoptik sind jedoch sehr aufwendig und teuer in ihrer Fertigung und werden deshalb bislang als wiederverwendbare Geräte entwickelt und hergestellt. Der gekrümmte Arbeitskanal solcher Endoskope sowie die komplexe und hinterschnittreiche Konstruktion des Albarranhebels haben sich in der Praxis zudem als schwer sterilisierbar erwiesen bzw. der Sterilisationsprozess hat sich als zu materialermüdend für die empfindlichen Geräte herausgestellt, sodass nach einem Eingriff mit einem solchen Duodenoskop nur eine Desinfektion möglich ist. Dies hat zur Folge, dass nach einem Eingriff ein Bakterienrasen (Biofilm) im Arbeitskanal und/oder parallel dazu angeordneten Hilfskanälen für Wasser, Energie, etc. des Endoskops bestehen bleiben kann. Löst sich dieser Biofilm dann bei einem darauffolgenden Eingriff, etwa, weil ein Instrument durch den Arbeitskanal geschoben wird, so kann er bspw. in den Gallengang und/oder Pankreasgang des nachfolgenden Patienten gelangen und dort schwerwiegende Entzündungen bis hin zu einer Sepsis beim Patienten verursachen.

Weiter haben solche Geräte den Nachteil, dass sie nur für wenige, sehr spezifische Eingriffe im Bereich des Duodenums eingesetzt werden können, da weder die Optik noch der Arbeitskanal in prograde Richtung gerichtet werden können. Zudem ist die Navigation im Körper mit seitwärts blickenden Endoskopen im Allgemeinen eher schwierig, da ein Vorausblicken immer ein Abkrümmen des dem Endoskopkopf unmittelbar vorgeordneten Deflectings um ca. 90° erfordert, wodurch wiederum mehr Platz in Endoskopquerrichtung benötigt wird, der nur im Magen vorhanden ist.

Des Weiteren ist aus DE 10 2018 110 620 A1 ein Abklappmechanismus zur abklapp- oder abwinkelbaren Halterung eines Endoskopkopfes bekannt, mit einer Anzahl axial aufeinanderfolgender und mittels eines Betätigungselements aktiv d.h. manuell kontrolliert gegeneinander winkelverstellbarer Segmente, die in Axialrichtung einen Arbeitskanal definieren. Die Segmente sind als Ellipsen- oder Zylinderausschnitte mit keilförmig zueinander ausgerichteten Stirnseiten ausgebildet, wobei jeweils zwei unmittelbar benachbarte Segmente derart zueinander ausgerichtet sind, dass sie sich an ihren jeweiligen Zylindermantelabschnitten maximaler Axiallänge axial abstützen oder aufeinander aufliegen, wodurch an der Abstütz- oder Auflagestelle ein Scharnier- oder Gelenkkontakt entsteht. Durch diese Konstruktion ist es möglich, den distalen Endoskopkopf in einem sehr engen Radius in eine Radialausrichtung zu verschwenken, derart, dass die distale Stirnseite des Endoskopkopfs nicht oder nur geringfügig über den Außenumfang des Endoskopschafts radial vorragt. Dabei sei darauf hingewiesen, dass der so aufgebaute Abklappmechanismus funktional auch als Bestandteil des Endoskopkopfs betrachtet werden kann und den Endoskopkopf somit in einen distalen und einen proximalen Kopfabschnitt unterteilt, wobei der proximale Kopfabschnitt (proximal letztes Segment) am Endoskopschaft (ggf. über ein zusätzliches Deflecting mit zum Abklappmechanismus unterschiedlichem aktivem Biegeradius) fest angeschlossen ist und der distale Kopfabschnitt (distal letztes Segment) zum proximalen Kopfabschnitt aktiv (manuell kontrolliert) verschwenkt/abgeklappt werden kann.

Der US2008/051802, die als nächstliegender Stand der Technik anzusehen ist, ist ein weiteres Endoskop zu entnehmen mit einem abwinkelbaren Schaft, der aus Segmenten gebildet ist und einen Arbeitskanal aufweist. Ein Verbindungsplatte oder ein Band, das eine gleitende Bewegung eines eingeschobenen Werkzeugs fördert, ist dieser Offenbarung nicht zu entnehmen.

Nachteilig an diesem Mechanismus ist jedoch, dass in den Arbeitskanal eingeschobene Instrumente innerhalb des Arbeitskanals insbesondere aufgrund des sehr engen Abklapp-/Abwinkelradius an den Segmenten anschlagen, sich verhaken und somit blockieren können und daher oft mehrmals vor- und zurückbewegt werden müssen, um durch den Arbeitskanal eingeführt zu werden. Dies erschwert die Nutzung des Endoskops.

### Zusammenfassung der Offenbarung

Die der Offenbarung zugrundeliegende Aufgabe ist es, Nachteile des Stands der Technik zu vermeiden oder zu reduzieren. Insbesondere soll ein Abklappmechanismus für ein Endoskop bereitgestellt werden, welcher auf eine Ausrichtung einer Endoskop-Optik im Endoskopkopf sowohl nach vorne als auch bei sehr geringem Raumbedarf nach lateral oder ggf. sogar rückwärtsgewandt ermöglicht und welcher ferner eine einfache Nutzung in Verbindung mit medizinischen Instrumenten sicherstellt.

Die der Offenbarung zugrundeliegende Aufgabe wird durch einen Abklappmechanismus und durch ein (medizinisches) Endoskop mit den Merkmalen der unabhängigen Ansprüche 1 und 12 gelöst.

Abklappmechanismen gemäß den unabhängigen Ansprüchen 1 und 12 44 beruhen, wie nachfolgend genauer erläutert, auf einer gemeinsamen erfinderischen Idee. Ein Abklappmechanismus wird bereitgestellt, der eine Anzahl von, mindestens ein, vorzugsweise mehrere, endoskoplängs zueinander angeordneten, keilartigen Segmenten (Abklappmechanismus-Wirbelkörper) hat, der/die zusammen einen durch den Abklappmechanismus verlaufenden Arbeitskanal ausbilden und der/die an einer Seite derart gelenkartig an der distalen Stirnseite des Endoskopschafts und/oder miteinander verbunden sind, dass die Anzahl der (insbesondere mehrerer) Segmente über ein entsprechendes Gelenk relativ zur distalen Stirnseite und/oder zueinander abwinkelbar sind, um den Abklappmechanismus (vergleichbar zu einem Finger einer menschlichen Hand) abzuklappen/zu krümmen. Ist der Abklappmechanismus abgeklappt/gekrümmt, liegen die Gelenke zwischen den jeweiligen Segmenten und/oder zur distalen Stirnseite des Endoskopschafts an einem äußeren Krümmungsradius des Abklappmechanismus und somit an einer Seite (Umfangsabschnitt des Endoskops), an welcher ein durch den Arbeitskanal vorgeschobenes Instrument an einer Wand des Arbeitskanals abgleitet / umgelenkt wird. Da das Instrument an Kanten oder Spalten in der Wand des Arbeitskanals, welche an Kontaktstellen zwischen benachbarten Segmenten vorliegen, hängen bleiben und blockieren kann oder zumindest nicht gleichmäßig (mit gleichmäßigem Kraftaufwand/Geschwindigkeit) eingeschoben werden kann, ist an der Seite (Umfangsabschnitt), an welcher das Instrument insbesondere im Fall einer Abkrümmung größer 0° hauptsächlich abgleitet (d.h. im Bereich der Gelenke) eine Abgleitplatte / eine Abgleitbandeinrichtung bereitgestellt, welche an der Seite (Umfangsabschnitt) des äußeren Krümmungsradius des Arbeitskanals angeordnet ist (eine Begrenzung / einen Wandabschnitt des durch die Segmente gebildeten Arbeitskanals bildet) und die Gelenke ersetzt/bildet oder überdeckt und ferner (zum Arbeitskanal hinweisende) Kanten und/oder Spalten zwischen den jeweils benachbarten Segmenten am äußeren Krümmungsradius des Abklappmechanismus minimiert oder überdeckt, um eine (in Endoskop-Längsrichtung) durchgängige, glatte Arbeitskanal-Abgleitfläche für das Instrument entlang des Abklappmechanismus bereitzustellen.

An dieser Stelle sei darauf hingewiesen, dass Endoskope der vorliegenden Gattung (so auch das vorliegende offenbarungsgemäße Endoskop) optional mit aktiv betätigbaren/abwinkelbaren distalen Schaftabschnitten, sogenannten Deflectings, ausgerüstet sein können, die in der Regel zwischen dem Endoskopkopf (also proximal zum Abklappmechanismus) und dem für gewöhnlich passiv abkrümmbaren (biegeflexiblen) Endoskopschaftabschnitt angeordnet sind. Zur Unterscheidung beider aktiv separat zueinander betätigbarer Mechanismen ist hauptsächlich der angestrebte, funktionsabhängige Abwinkelradius zu nennen. Während das Deflecting prinzipiell dazu dient, bei Einführen des Endoskops in ein Patientenhohlorgan dem Hohlorgan bzw, dem Verlauf des Organhohlraums (z.B. Darm, Speise-/Luftröhre, etc.) besser folgen zu können und ggf. eine Hohlorganwandung zu Behandlungszwecken anzunähern, ist der Abklappmechanismus dazu ausgelegt, innerhalb des Hohlorgans ein Abklappen des Endoskopkopfs oder eines distalen Teils davon quasi senkrecht zur Hohlorganwand zu ermöglich, ohne dass das Hohlorgan übergebührlich aufgeweitet werden muss. Letzteres bedingt einen gegenüber dem Deflecting (deutlich) kleineren Biege- /Abwinkelradius mit konstruktiven Konsequenzen wie z.B. größerer Spalte zwischen den Segmenten an der Abwinklungsaußenseite, die dann wieder zu deren Behebung zusätzliche konstruktive Maßnahmen ggf. erforderlich machen. Solche Maßnahmen sind bei Deflectings nicht erforderlich und werden durch Deflectings auch nicht angeregt. In anderen Worten kann ein Endoskop gemäß der Offenbarung den Abklappmechanismus mit einem ersten maximalen Abkrümm-/Abwinkelradius haben und kann proximal dazu ein Deflecting mit einem zweiten maximalen Abkrümm-/ Abwinkelradius bereitgestellt sein, welcher größer als der erste maximale Abkrümm-/ Abwinkelradius ist.

Optional kann (insbesondere in Hinblick auf die Anlenkung der Segmente aneinander und der dazwischen auftretenden Lücken) der Endoskopkopf oder Kopfabschnitt als ein (nicht unbedingt keilartig ausebildetes) Segment betrachtet werden und/oder kann ein distales Ende des dem Abklappmechanismus proximal vorgelagerten Endoskopabschnitts als am ein weitesten proximal angeordnetes (nicht unbedingt keilartig ausgebildetes) Segment betrachtet werden. In diesem Fall kann der Abklappmechanismus ein einzelnes keilartiges Segment aufweisen, wobei bevorzugt mehrere keilartige Segmente bereitgestellt sind.

Nachfolgend wird zunächst die Lösung der Aufgabe gemäß Anspruch 1 genauer beschrieben.

Genauer ausgedrückt, wird die Aufgabe durch einen Abklappmechanismus zur abklapp- oder abwinkelbaren Halterung eines Endoskopkopfes am distalen (vom Anwender abgewandten/ dem Patientenkörper zugewandten) Ende/Endabschnitt eines Endoskop(schaft)abschnitts gelöst, mit einer Anzahl (ein einzelnes oder mehrere) axial aufeinanderfolgender und mittels zumindest eines Betätigungselements aktiv (manuell kontrolliert) gegeneinander winkelverstellbarer/abklappbarer, im Wesentlichen zylindrischer (einschließlich ovaler/elliptischer) Segmente, welche jeweils zwei im Wesentlichen keilförmig zueinander ausgerichtete Stirnseiten und einen Zylindermantel aufweisen, der an einem Zylindermantelabschnitt mit maximaler axialer Länge einen Keilrückenabschnitt/Keilrückenbereich definiert, und welche in Endoskop-Längsrichtung zumindest einen (inneren) Arbeitskanal ausbilden, wobei der Arbeitskanal im Keilrückenabschnitt jedes Segments den Zylindermantel in Endoskop-Radialrichtung durchbricht bzw. im Keilrückenabschnitt jedes Segments radial nach außen offen liegt (der Zylindermantel ist im Keilrückenabschnitt bis in den Arbeitskanal in Axialrichtung aufgebrochen bzw. hat einen bis zum Arbeitskanal reichenden Längsschlitz), und wobei die Anzahl der Segmente im Keilrückenabschnitt durch eine einzige/gemeinsame flexible, vorzugsweise elastische Verbindungsplatte in Endoskop-Längsrichtung miteinander verbunden sind, welche am Außenumfang der Anzahl der Segmente nur über einen Teilumfangsabschnitt (etwas größer als die Schlitzbreite im Keilrückenabschnitt) an diese angeschlossen ist, um den Arbeitskanal jedes Segments radial nach außen zu verschließen, wobei zum einen durch die Verbindungsplatte eine im Arbeitskanal kontinuierlich verlaufende (die Segmente überbrückende) Abgleitfläche für eingeführte medizinische Instrumente und zum anderen Scharnierabschnitte zur Winkelverstellung zweier jeweils benachbarter Segmente gebildet wird.

Der Offenbarung liegt die Erkenntnis zugrunde, dass die aus dem Stand der Technik bekannten Segmente Kanten und zwischen benachbarten Segmenten jeweils Lücken bilden, welche quer zum Arbeitskanal verlaufen. Ist der Arbeitskanal abgekrümmt, wird ein Instrument, welches durch den Arbeitskanal geschoben wird, im Arbeitskanal umgeleitet und gleitet an einer Innenseite der Segmente ab (genauer, an der Seite der Segmente, an welcher diese den Gelenkkontakt bilden), sodass das Instrument an diesen Kanten und Lücken leicht anstoßen und sich verhaken kann. Ein ggf. zusätzlich innerhalb des Arbeitskanals geführter Schlauch kann dieses Problem, insbesondere im abgeklappten Zustand, nicht beheben. Die erfinderische Idee beruht zusammenfassend darauf, dass in dem Bereich, an welchem das Instrument hauptsächlich abgleitet, insbesondere im Bereich des Gelenkkontakts zwischen den Segmenten, eine kontinuierliche, flexible, sich über (insbesondere sämtliche) die Lücke zwischen benachbarten Segmenten des Abklappmechanismus erstreckende Abgleitplatte (Verbindungselement) oder Abgleitbandeinrichtung bereitgestellt ist, welche eine zumindest in Endoskop-Längsrichtung kontinuierliche Abgleitfläche für das Instrument bei dessen Vorschub durch den Arbeitskanal bildet. Somit wird ein Verhaken eines Instruments zwischen den Segmenten beim Vorschieben durch den Arbeitskanal vermieden.

In dem Bereich, an welchem das Instrument hauptsächlich abgleitet, bzw. im Bereich des Gelenkkontakts zwischen den Segmenten wird die durch die Segmente gebildete Arbeitskanalwand entfernt und der somit entstehende Durchbruch durch die Abgleitbandeinrichtung/-platte/Verbindungsplatte (vorzugsweise vollständig) überdeckt. Dadurch können zudem weitere überraschende Effekte erzielt werden. Zum einen kann aufgrund des Durchbruchs in den Segmenten ein Durchmesser des Arbeitskanals vergrößert und somit mehr Platz für benötigte Instrumente erreicht werden oder ist (bei einem unveränderten Arbeitskanaldurchmesser) in den Segmentquerschnitten Platz für weitere durch das Endoskop verlaufende Kanäle oder Bauteile bereitgestellt. Zum anderen wird erreicht, dass eine Scharniermechanik zwischen den einzelnen Segmenten sowohl in ihrem Aufbau als auch in der Herstellung/Montage besonders einfach und somit kostengünstig ist.

In anderen Worten ausgedrückt, weist der Abklappmechanismus für eine bzw. einer distale/n Endoskopspitze (Endoskop-Endabschnitt) eine Anzahl von im Wesentlichen zylindrischen bis ovalen, in Endoskop-Längsrichtung zueinander ausgerichteten Segmenten (Wirbelkörper) auf, die an einem Teilumfangsabschnitt (dem Keilrückenabschnitt) über ein(e) außenseitig an den Segmenten angebrachte(s)/fixierte(s) (gemeinsame(s)) flexible(s) Verbindungsplatte/ Rückenelement derart miteinander verbunden sind, dass sie zueinander über die als Scharnierabschnitt wirkende Verbindungsplatte verschwenkbar sind. Entsprechende Scharnierachsen sind jeweils zwischen zwei Segmenten angeordnet. Insbesondere wirkt die flexible Verbindungsplatte an den Scharnierabschnitten, d.h. zwischen jeweils zwei Segmenten, als ein Filmscharnier. Ferner verjüngen sich die Segmente zu einem anderen, der Verbindungsplatte diametral gegenüberliegenden Teilumfangsabschnitt hin (einem Keilspitzenabschnitt) derart, dass zwischen mehreren Segmenten ein Schwenkraum/Schwenkspalt bereitgestellt ist, der ermöglicht, eine Länge des Abklappmechanismus an dieser anderen Seite um maximal eine axiale Länge des Schwenkraums aktiv zu verkürzen, und zwar durch das Verschwenken der Segmente über die Verbindungsplatte/um die dadurch gebildeten Scharnierachsen. Ein maximales Abklappen ist vorzugsweise erreicht, wenn die (sämtliche) Segmente an den jeweils benachbarten Segmenten (bzw. am proximal und/oder distal dazu angrenzenden Endoskopabschnitt) anliegen/wenn deren Stirnflächen einander kontaktieren. Ein Arbeitskanal verläuft längs durch die Segmente und ist derart exzentrisch zum Segmentquerschnitt angeordnet, dass ein Außenumfang des Arbeitskanals und ein Außenumfang des Segments einander im Keilrückenabschnitt überschneiden, sodass am entsprechenden Überschneidungsbereich im Keilrückenabschnitt entlang des gesamten Segments in Endoskop-Längsrichtung ein Durchbruch gebildet ist. Dieser Durchbruch wird durch die radial außen an den Segmenten angebrachte Verbindungsplatte geschlossen. Die Verbindungsplatte stellt somit eine entlang des (gesamten) Abklappmechanismus verlaufende, kontinuierliche, glatte, kanten- und lückenfreie Fläche (d.h., die Abgleitfläche) dar, an welcher, insbesondere im abgeklappten Zustand des Abklappmechanismus, ein durch den Arbeitskanal geschobenes Instrument gleichmäßig abgleiten kann und gleichmäßig umgeleitet wird.

Die Segmente können beispielsweise einen kreiszylindrischen oder ovalen Querschnitt haben. Die Stirnseiten sind vorzugsweise eben, können aber beispielsweise auch konkav oder konvex gewölbt ausgebildet sein. Ferner wird dadurch, dass der Arbeitskanal zum Keilrückenabschnitt hin den Zylindermantel (die Mantelfläche) in Radialrichtung durchbricht, d.h., dadurch, dass der Arbeitskanal seitlich geöffnet ist, wird ein größerer Querschnitt des Arbeitskanals ermöglicht, wodurch das Einführen von Instrumenten vereinfacht ist. Optional kann ein dünnwandiger Innenschlauch bereitgestellt sein, welcher durch den durch die Segmente gebildeten Arbeitskanal verläuft, insbesondere um diesen (insbesondere zwischen den Segmenten) abzudichten.

Die kontinuierlich verlaufende Abgleitfläche, welche im Keilrückenabschnitt den Arbeitskanal begrenzt, ist insbesondere eine Fläche, die in einem Bereich, an welchem die Instrumente abgleiten, wenn der Abklappmechanismus abgeklappt/abgewinkelt ist, sowie in einer Vorschubrichtung der Instrumente durchgängig (durchbruchsfrei) und eben (kanten- und knickfrei) ausgebildet ist. Dies ermöglicht ein einfaches Abgleiten von eingeschobenen Instrumenten und vereinfacht dadurch ein Einschieben derselben in den Arbeitskanal.

Im Konkreten hat das offenbarungsgemäße Endoskop einen Endoskopkopf, der vorzugsweise über ein manuell aktuierbares/abkrümmbares (Endoskop-) Deflecting mit einem vorzugsweise flexiblen (passiv/selbsttätig biegbaren) Endoskopschaft gekoppelt ist, an dessen proximalem (dem Anwender zugewandten/ vom Patientenkörper abgewandten) Ende ein Handgriff angeordnet/anschließbar ist. Zwischen dem Endoskopkopf bzw. dem distalen (vom Anwender abgewandten/ dem Patientenkörper zugewandten) axialen Endabschnitt des Endoskopkopfs und dem Endoskopschaft oder vorzugsweise zwischen dem Endoskopkopf bzw. dem distalen axialen Endabschnitt des Endoskopkopfs und dem Endoskopfschaft oder Deflecting ist ein (zusätzlicher) Abklappmechanismus (mit einem zum Deflecting, falls vorhanden, unterschiedlichen/kleineren Abklappradius) zwischengeordnet, mit zumindest einem oder mehreren wirbelartigen Segmenten, die in Seitenansicht betrachtet keilförmig gebildet sind. Jedes Segment ist mit wenigstens einer Längs-Durchgangsöffnung (oder einer Längs-Durchgangsbohrung) versehen, die einen Axialabschnitt eines (gemeinsamen) Arbeitskanals ausbildet. Die Längs-Durchgangsöffnung ist dabei zur Mittlachse des betreffenden Segments radial nach außen versetzt, derart, dass der Durchmesser der Durchgangsöffnung im Segmentumfangsbereich mit infolge der Keilform maximaler axialer Segmentlänge über den Außenumfang des Segments hinausreicht und dadurch ein Längsschlitz oder Längsspalt entsteht. Dadurch erhält das Segment in Draufsicht bevorzugt eine sichelförmige Kontur. Schließlich ist eine Verbindungsplatte/ ein Rückenelement vorgesehen, die/das an den Seitenumfangsbereich mit maximaler axialer Segmentlänge angepasst ist, derart, dass sie bei deren Befestigung an der Außenseite des Segments lediglich diesen Seitenumfangsbereich (also nicht vollumfänglich) abdeckt und somit den Längsschlitz nach Außen verschließt. Die Verbindungsplatte ist ferner zumindest am Endoskopkopf bzw. am distalen Kopfabschnitt und vorzugsweise an einem weiteren proximalen Segment gleichen Aufbaus (falls vorhanden) außenseitig befestigt und verbindet so diese Elemente miteinander. Dabei ist die Verbindungsplatte in einem Axialabschnitt zwischen den hiermit verbundenen Elementen mit einer Einschnürung in Umfangsrichtung versehen, wodurch ein (Falt-) Scharnier gebildet wird, um welches die hiermit verbundenen Elemente schwenkbar/abklappbar sind.

Vorteilhafte Ausgestaltungen der Offenbarung sind Gegenstand der Unteransprüche und werden nachfolgend genauer beschrieben. Diese können in beliebiger Kombination oder ggf. auch gesondert, d.h. in jeweils separater Weise, beansprucht werden.

Vorzugsweise deckt die Verbindungsplatte den Arbeitskanal im Keilrückenabschnitt zumindest teilweise (nämlich im Bereich des Längsschlitzes) ab und ist insbesondere darüber gewölbt.

Die Scharnierabschnitte haben vorteilhafterweise eine Ausdehnung in Endoskop-Längsrichtung, insbesondere derart, dass die an der Verbindungsplatte angeordneten oder befestigten Segmente voneinander beabstandet sind. Dadurch ist ein Biegeradius der Scharnierabschnitte erhöht und ein Übergang zwischen den abgeklappten Segmenten besser ausgerundet, d.h. besser für das Abgleiten der Instrumente geeignet. Insbesondere kann eine Ausdehnung jedes Scharnierabschnitts in Endoskop-Längsrichtung 30 bis 100% (vorzugsweise 40-70%) einer Ausdehnung der Verbindungsplatte im Bereich der (benachbarten) Segmente (d.h. in Halteabschnitten der Verbindungsplatte, an welchen die Segmente gehalten oder befestigt sind) betragen.

Alternativ oder zusätzlich kann die von der Verbindungsplatte gebildete Abgleitfläche im Bereich der Halteabschnitte entlang der Endoskop-Längsrichtung nach radial außen gewölbt / konkav ausgerundet sein. In anderen Worten ausgedrückt, bildet die Abgleitfläche (vorzugsweise nur) im Bereich der Halteabschnitte eine/n sich in der Längsrichtung nach radial außen ausrundende Mulde oder einen (innenseitigen) Bogen. Ist die Dicke der Verbindungsplatte auch im gewölbten Bereich konstant, wölbt sich somit im unabgeklappten/gestreckten Zustand des Abklappmechanismus eine radiale Außenseite der Verbindungsplatte im entsprechenden Bereich (bogen-/beulenartig) entlang der Endoskop-Längsrichtung nach außen. Dadurch kann eine Abgleitfläche bereitgestellt werden, die im abgeklappten Zustand des Abklappmechanismus, d.h., wenn die Instrumente besonders stark umgeleitet werden und eine besonders starke Radialkraft an der Abgleitfläche erzeugen, entlang ihrer (gesamten) Erstreckung in der Endoskop-Längsrichtung, d.h. in Abgleichrichtung, gekrümmt ist und somit ein besonders ebenes und stabiles Abgleiten ermöglicht.

Alternativ oder zusätzlich können sich die Halteabschnitte in der Endoskop- oder Segment-Umfangsrichtung über den sich im Keilrückenabschnitt öffnenden Arbeitskanal nach radial außen wölben. Insbesondere folgt die Wölbung jedes Halteabschnitts im Wesentlichen einem Außendurchmesser des jeweiligen Segments oder nähert diesen (z.B. polygonal) an. Der Arbeitskanal wird somit durch die Verbindungsplatte nicht verengt und hat viel Platz für einzuschiebende Instrumente. Ferner wird durch die Wölbung in Umfangsrichtung ein Führungskanal gebildet, welcher eingeschobene Instrumente in Endoskop-Längsrichtung führt und ein Hin- und Her-Rutschen derselben vermeidet.

Vorzugsweise ist die Verbindungsplatte zumindest im Bereich der Abgleitfläche kratzbeständig ausgebildet, um zu vermeiden, dass durch die daran abgleitenden Instrumente die Oberfläche beschädigt wird und dadurch ggf. schwierig zu desinfizieren ist. Beispielsweise kann die Oberfläche behandelt, z.B. gehärtet oder beschichtet sein, oder kann die Verbindungsplatte vollständig oder im Bereich der Abgleitfläche aus einem kratzfesten Material, vorzugsweise einem Metall, gefertigt sein.

Nach einer bevorzugten Ausführungsform bildet die Verbindungsplatte vorzugsweise laschenförmige oder ohrenförmige Halteabschnitte aus, welche jeweils zur Befestigung oder zum Halten an einem der Segmente und/oder am distalen Ende des Endoskopschafts/ Deflectings dienen. Insbesondere sind die Halteabschnitte und die Scharnierabschnitte wechselweise zueinander angeordnet. An den Halteabschnitten können die Segmente oder das distale Ende des Endoskopschafts/Deflectings sicher gehalten werden. Beispielsweise können die Segmente an den Halteabschnitten durch Kleben, Schweißen oder formschlüssig befestigt werden. Die Form der Halteabschnitte kann zudem an die Segmente angepasst werden, um einen besonders sicheren Halt zu ermöglichen. Zumindest einer der Halteabschnitte an einem proximalen Ende der Verbindungsplatte ist zur Befestigung/zum Halten von einem distalen Ende des Endoskopschafts, oder eines Endoskop-Deflectings, ausgebildet. Weiter distal liegende Halteabschnitte sind vorzugsweise an die Segmente des Abklappmechanismus angepasst.

Von besonderem Vorteil ist es, wenn die Verbindungsplatte zumindest an den Scharnierabschnitten, optional entlang ihrer gesamten Erstreckung, federelastisch ausgebildet ist. Die Verbindungsplatte (insbesondere die Scharnierabschnitte) kann somit als ein Rückstellelement dienen, welches dazu ausgebildet ist, den Abklappmechanismus aus einer abgeklappten Stellung in eine im Wesentlichen nach vorne ausgerichtete / gestreckte (die Endoskop-Längsachse gerade verlängernd oder einen stumpfen Winkel dazu bildende) Stellung zurückzustellen.

In anderen Worten ausgedrückt, kann die Verbindungsplatte, zumindest im Bereich der Scharnierabschnitte, als eine Biegefeder oder eine Blattfeder ausgebildet sein, welche den Abklappmechanismus in die im Wesentlichen nach vorne ausgerichtete / gestreckte Stellung vorspannt. Die nach vorne ausgerichtete / gestreckte Stellung ist eine Stellung, in welcher der Endoskopkopf / ein am weitesten distal angeordnetes Segment des Abklappmechanismus im Wesentlichen nach vorne, in einer Endoskop-Längsrichtung oder Endoskop-Vorschubrichtung, ausgerichtet ist (falls ein Endoskop-Deflecting vorgesehen ist, in einer ggf. durch das Deflecting vorgegebenen Endoskop-Vorschubrichtung), derart dass eine darin vorgesehene Optik einen Blick nach vorne ermöglicht. Durch eine solche federelastische Verbindungsplatte ist nur zum Abklappen des Abklappmechanismus eine Betätigung durch den Nutzer notwendig, da die Rückstellung aus der abgeklappten Stellung durch die Verbindungsplatte erzeugt wird. Somit kann beispielsweise eine Anzahl von Betätigungszügen reduziert werden und wird die Nutzung für einen Anwender vereinfacht.

Besonders bevorzugte Effekte können erzielt werden, wenn eine Federelastizität der Verbindungsplatte entlang von ihrer Längserstreckung variabel, insbesondere kontinuierlich ab- oder zunehmend ist. Die Federelastizität kann z.B. durch eine Behandlung der Verbindungsplatte, durch ein Bereitstellen von schwächenden oder stärkenden Strukturen durch eine sich verändernde Materialdicke oder dergleichen erreicht werden. Im nachfolgenden Beispiel wird eine bestimmte Ausgestaltung der Verbindungsplatte beschrieben, durch welche eine Federelastizität der Verbindungsplatte entlang ihrer Axialerstreckung wahlweise einstellbar ist.

Besonders vorteilhaft ist es ferner, wenn die Scharnierabschnitte derart in Umfangsrichtung (insbesondere nach radial außen) gewölbt/gekrümmt sind, dass jeder Scharnierabschnitt eine in Endoskop-Längsrichtung verlaufende Rinne mit einem bestimmten, konstanten oder variablen, Rinnenradius (Krümmungsradius/Wölbung/Wölbungsgrad) bildet. Vorzugsweise ist der Rinnenradius/Krümmungsradius bzw. die Wölbung/der Wölbungsgrad abhängig von einer erwünschten Rückstellkraft angepasst/ausgewählt. In anderen Worten ausgedrückt, ist die Rückstellkraft der als Rückstellelement dienenden (federelastischen) Scharnierabschnitte u. A. abhängig von deren Wölbung/Krümmung entlang der Umfangsrichtung einstellbar/eingestellt. Die Rückstellkraft ist eine Kraft, mit welcher der Scharnierabschnitt (aufgrund seiner Federelastizität) den Abklappmechanismus zurück in die unabgeklappte oder gestreckte Stellung drängt. Das heißt, die Rückstellkraft, wirkt entgegen dem zum Abklappen dienenden Betätigungselement und stellt optional auch das Betätigungselement in eine Ausgangslage zurück, wenn dieses durch den Nutzer nicht mehr betätigt wird. Die Rückstellkraft ist stärker, je kleiner der Rinnenradius bzw. je größer/enger die Wölbung des Scharnierabschnitts ist.

Funktional ausgedrückt, werden beim Biegen oder Abklappen der Scharnierabschnitte an den Rillenrändern/im Rillenrandbereich (elastisch, ggf. auch plastisch) gestaucht und/oder in einem (in Umfangsrichtung betrachtet) mittigen Bereich/Rillenmittenbereich (elastisch, ggf. auch plastisch) gestreckt. Da jedoch die Rillenränder/die gekrümmten Scharnierabschnittsränder dieser Stauchung entgegenwirken (diese weitgehend blockieren), werden diese (insbesondere elastisch) verformt, genauer, nach radial außen gedrückt. Das heißt, sie verformen sich derart, dass der jeweilige Scharnierabschnitt sich entlang seiner Scharnierachse einer im Wesentlichen ebenen oder geraden (d.h., ungewölbten oder ungekrümmten) Form nähert (flacht sich ab). Genauer ausgedrückt, flacht sich der Scharnierabschnitt bei einem geringfügigen Abklappen des Abklappmechanismus zunächst im Rillenmittenabschnitt ab, d.h., der Scharnierabschnitt wird entgegen der Krümmung gebogen, um das Biegen des Scharnierabschnitts zu ermöglichen. Wird der Abklappmechanismus stärker abgeklappt/in Richtung der abgeklappten Stellung betätigt, vergrößert/verbreitert sich der im Wesentlichen abgeflachte Bereich in Umfangsrichtung, d.h. hin zu den Rillenrändern, um die stärkere Biegung des Scharnierabschnitts zu ermöglichen. Dabei wirkt der Verformungswiderstand/die Elastizität des Materials der Biegung/dem Abklappen entgegen. Um ein gleichmäßiges Abklappen des Abklappmechanismus zu gewährleisten, ist es von Vorteil, wenn die Wölbung/die Rinne spiegelsymmetrisch zu einer durch die Rinnenmitte und längs der Endoskop-Längsachse verlaufenden Spiegelebene ist. In anderen Worten ausgedrückt, ist ein Verlauf der Wölbung/Rinne/Krümmung ausgehend von der Rinnenmitte hin zu den Rinnenrändern zu beiden Seiten hin gleich.

Ist der Rillenradius/die Wölbung (zwischen der Rinnenmitte zum Rinnenrand) hin variabel, kann insbesondere eine Rückstellkraft eingestellt werden, welche abhängig von einem Abklapp-/Abwinkelradius (einem Betätigungsgrad) des Abklappmechanismus variiert. D.h., die Rückstellkraft ist über die Rinnenkrümmung/den Rinnenradius/die Wölbung modifizierbar. Insbesondere ist die Rückstellkraft durch den Rillen-/Krümmungsradius und/oder durch einen Krümmungsverlauf (also die Änderung des Rillen-/Krümmungsradius) entlang der Endoskop-/Segment-Umfangsrichtung derart eingestellt, dass ein kleinerer Rillen-/Krümmungsradius einer größeren Rückstellkraft entspricht bzw. dass eine Position eines eingestellten Krümmungsradius entlang der Endoskop-/Segment-Umfangsrichtung einem Abklappwinkel/Abklappgrad des Abklappmechanismus entspricht, wobei ein maximaler Abklappwinkel/Abklappgrad einer Position der Krümmung am Rillenrand entspricht und ein minimaler Abklappwinkel/Abklappgrad einer Position der Krümmung in der Rillenmitte entspricht.

Beispielsweise verringert sich der Rinnenradius hin zu den in Umfangsrichtung äußeren Scharnierabschnittsrändern bzw. Rillenrändern. D.h., der Rillen-/Krümmungsradius ist im Rillenmittenbereich groß oder sogar gar nicht gekrümmt (d.h. gerade) und im Rillenrandbereich klein (d.h. stark gekrümmt) um zu gewährleisten, dass im stark abgeknickten Zustand des Abklappmechanismus eine höhere Rückstellkraft wirkt als im wenig abgeknickten Zustand. Dies kann beispielsweise von Vorteil sein, da der Nutzer dadurch ein verbessertes sensorisches Feedback darüber erhält, wie stark der Abklappmechanismus abgeklappt ist. In anderen Worten ausgedrückt, kann der Rinnenradius/Krümmungsradius, entlang der Umfangsrichtung betrachtet, mittig an dem Scharnierabschnitt relativ groß sein und zu den (seitlichen/freien) Scharnierabschnittsrändern bzw. Rillenrändern hin abnehmen, bzw. kann die Wölbung/der Wölbungsgrad mittig relativ gering sein und zu den Rändern hin stärker/enger werden. Wenn der Rinnenradius zum Rand hin abnimmt bzw. die Wölbung zum Rand hin zunimmt, ist die Rückstellkraft umso größer, je stärker der Abwinkelmechanismus abgeklappt/abgewinkelt ist.

Alternativ oder zusätzlich kann der Rillen-/ Krümmungsradius in dem Rillenmittenbereich und/oder in einem Zwischenbereich zwischen der Rillenmitte und dem Rillenrand stärker als entlang des übrigen Rinnenquerschnitts gekrümmt sein. Somit ist entsprechend in einem nur geringfügig (insbesondere minimal) abgeklappten Zustand und/oder einem mittleren Abklappzustand eine große Rückstellkraft bereitstellbar. Ist die Rille/der Scharnierabschnitt im Rillen- / Scharnierabschnittsrandbereich nur vergleichsweise schwach oder gar nicht gekrümmt, ist die Rückstellkraft im maximal abgeklappten Zustand gering. Dies ist vorteilhaft, da ein Anwender in diesem Fall im maximal abgeklappten/maximal betätigten Zustand des Abklappmechanismus weniger Betätigungskräfte aufbringen muss. Ein einfaches (wenig Bedienkraft benötigendes) Halten des Abklappmechanismus im maximal abgeklappten Zustand durch den Nutzer wird somit erleichtert.

Zusammenfassend kann die Rückstellkraft im maximal abgeklappten/abgewinkelten Zustand des Abklappmechanismus vergrößert (maximiert) sein, wenn der Rinnenradius sich unmittelbar am Randbereich (maximal) verringert und/oder kann die Rückstellkraft in einem zwischenliegenden Bereich vergrößert (maximiert) sein, wenn der Rinnenradius sich an einem zwischenliegenden Bereich (zwischen der Mitte und dem Rand der Rinne) (maximal) verringert und/oder kann die Rückstellkraft im nur wenig (minimal) abgeklappten/abgewinkelten Zustand (im gestreckten Zustand) des Abklappmechanismus vergrößert (maximiert) werden, wenn der Rinnenradius sich im Rinnenmittenbereich (maximal) verringert.

Die Verbindungsplatte kann entweder derart ausgebildet sein, dass jeder Scharnierabschnitt gleich ist, oder die Verbindungsplatte kann mehrere unterschiedliche, insbesondere unterschiedlich gekrümmte, Scharnierabschnitte aufweisen. Z.B. kann der am weitesten proximal liegende, erste Scharnierabschnitt im Scharnier-/Rillenrandbereich stärker gekrümmt sein als im übrigen Scharnier- /Rillenbereich, und kann der am weitesten distal liegende, letzte Scharnierabschnitt im Scharnier-/Rillenmittenbereich stärker gekrümmt sein. Die zwischenliegenden Scharnierabschnitte entlang der Endoskop-Längsachse können ausgehend von dem ersten Scharnierabschnitt hin zum letzten Scharnierabschnitt jeweils einen etwas weiter mittig liegenden stark gekrümmten Bereich aufweisen. D.h., entlang der Endoskop-Längsachse wird scharnierabschnittsweise die Krümmung graduell weiter zum Rillenmittenbereich gerückt bzw. ausgehend von dem ersten Scharnierabschnitt mit der starken Krümmung im Rillenrandbereich wird ein gradueller Übergang der Position der starken Krümmung bis zu dem letzten Scharnierabschnitt mit der starken Krümmung im Rillenmittenbereich bereitgestellt. Auf diese Weise wird sichergestellt, dass der Abklappmechanismus zuerst an seinem proximalen Ende / zuerst an dem am weitesten proximal liegenden Scharnierabschnitt abklappt, dann die weiteren Segmente/die weiteren Scharnierabschnitte in Endoskop-Längsrichtung nacheinander abklappen und zuletzt das am weitesten distal liegende Segment / der daran angeordnete Endoskopkopf abklappt oder der Abklappmechanismus zuletzt an seinem distalen Ende abklappt. Die Scharnierabschnitte können auch in anderer Reihenfolge angeordnet sein, z.B. umgekehrt zu der vorstehend beschriebenen Reihenfolge und somit zunächst am distalen Ende des Abklappmechanismus abklappen und zuletzt am proximalen Ende abklappen.

Es ist insbesondere von Vorteil, wenn die Verbindungsplatte (insbesondere Scharnierabschnitte der Verbindungsplatte) im distalen Bereich eine geringere Federelastizität aufweist als im proximalen Bereich (insbesondere im Bereich der am weitesten proximal liegenden Segmente des Abklappmechanismus oder des Deflectings oder im Bereich der am weitesten proximal liegenden Segmente des Deflectings). Dadurch wird erreicht, dass das Endoskop sich von seinem distalen Ende ausgehend aufrollt. Vorteilhafter Weise kann hierdurch ein laterales Ausschwenken des distalen Endoskopkopfes beim Abkrümmen verringert werden. Somit benötigt das vorliegende Endoskop weniger Platz beim Abklappen des Endoskopkopfes über den Abklappmechanismus und ggf. das Deflecting. Bevorzugt verbindet das Verbindungselement sowohl die Segmente des Abkappmechanismus und des Deflectings. Weiter bevorzugt nimmt eine Steifigkeit des Verbindungselements entlang seiner Axialerstreckung in Richtung distal vorzugsweise kontinuierlich bzw. gleichmäßig ab.

Ferner hat es sich als zweckmäßig erwiesen, wenn die Verbindungsplatte an den Scharnierabschnitten (in Umfangsrichtung) verjüngt ist (die vorstehend genannten Einschnürungen bildet). Dadurch wird die Flexibilität / Biegbarkeit der Scharnierabschnitte erhöht. Optional können die Scharnierabschnitte dennoch eine ausreichende Torsionssteifigkeit aufweisen. Ferner sollten die Scharnierabschnitte breit genug ausgebildet sein, um als Teil der Abgleitfläche zu dienen.

Bevorzugt kann die Verbindungsplatte einen in Endoskop-Längsrichtung durchgehenden, glatten Mittenabschnitt bilden, welcher die Abgleitfläche bildet, von dem symmetrisch (an den Halteabschnitten) beiderseits quer oder schräg zur Endoskoplängsrichtung oder in Endoskop-Umfangsrichtung laschenförmige Befestigungsbereiche oder Laschen abstehen, welche zur Befestigung der Segmente dienen und bezüglich des Mittenabschnitts aufeinander zu gebogen sind.

Vorzugsweise können die Befestigungsbereiche in Endoskop-Längsrichtung breit (insbesondere mehr als viermal, bevorzugt mehr als fünfmal, weiter bevorzugt mehr als zehnmal so breit) im Verhältnis zu Schlitzen oder Zwischenräumen sein, die zwischen den jeweils benachbarten Befestigungsbereichen ausgebildet sind. Vorzugsweise haben die Befestigungsbereiche im Wesentlichen eine Breite in Endoskop-Längsrichtung, die jeweils zumindest einer maximalen Ausdehnung von einem der Segmente in Endoskop-Längsrichtung entspricht, weiter vorzugsweise der maximalen Ausdehnung von einem der Segmente in Endoskop-Längsrichtung und einem Abstand zwischen zwei benachbarten Segmenten entspricht. Die Schlitze können bevorzugt im Wesentlichen linienförmig/ eindimensional sein. In anderen Worten ausgedrückt, kann die Verbindungsplatte an beiden Seiten mit (parallelen) Einschnitten versehen sein, welche jeweils zwei benachbarte Befestigungsbereiche voneinander trennen. Somit können die benachbarten (blechartigen) Befestigungsbereiche dachziegelartig/ schuppenartig übereinander gleiten, wenn der Abklappmechanismus abgekrümmt wird. Somit ist eine Abdeckung eines Spalts zwischen den Segmenten/ Kippkörpern (zumindest im Keilrückenbereich) unabhängig von der Krümmung des Abklappmechanismus gewährleistet, selbst wenn der Mittenbereich der Verbindungsplatte schmal ausgebildet ist. Ein Verhaken des Instruments während des Vorschiebens durch den Arbeitskanal wird somit noch besser unterbunden.

Alternativ oder zusätzlich kann zusätzlich zu der (ersten) vorstehend beschriebenen Verbindungsplatte eine zweite Verbindungsplatte bereitgestellt sein, welche einen sich in Endoskop-Längsrichtung erstreckenden zweiten Mittenabschnitt und davon symmetrisch quer zum Mittenabschnitt (in Endoskop-Umfangsrichtung) vorstehende Laschen aufweist. Die zweite Verbindungsplatte ist insbesondere Endoskop-radial innerhalb (bezogen auf einen montierten Zustand des Abklappmechanismus) der ersten (radial äußeren) Verbindungsplatte angeordnet. Diese zwei (erste und zweite) Verbindungsplatten können zueinander in der Endoskop-Längsrichtung verschiebbar gleitend angeordnet sein, insbesondere indem eine der beiden Verbindungsplatten nur an einer Stelle in Endoskop-Längsrichtung relativ zu der anderen der Verbindungsplatten fixiert und ansonsten frei verschiebbar ist.

Ferner können die zwei Verbindungsplatten derart in Längsrichtung versetzt angeordnet/ aufeinandergelegt sein, dass die Laschen der einen Verbindungsplatte die Zwischenräume zwischen den Laschen der anderen Verbindungsplatte zumindest teilweise, vorzugsweise vollständig, überdecken. Bevorzugt sind die Laschen der einen Verbindungsplatte in Endoskop-Längsrichtung zumindest so breit wie, insbesondere breiter als, die Zwischenräume zwischen den Laschen der jeweils anderen Verbindungsplatte. Die Mittenabschnitte der ersten und zweiten Verbindungsplatte liegen vorzugsweise übereinander, liegen weiter vorzugsweise in Umfangsrichtung positionsgleich/ deckungsgleich übereinander. Somit ist eine Abdeckung eines Spalts zwischen den Segmenten/ Kippkörpern (zumindest im Keilrückenbereich) unabhängig von der Krümmung des Abklappmechanismus gewährleistet. Ein Verhaken des Instruments während des Vorschiebens durch den Arbeitskanal wird somit noch besser unterbunden.

Weiter bevorzugt sind die Segmente (insbesondere alle Segmente) an der ersten, radial äußeren Verbindungsplatte wie vorstehend beschrieben montiert. Die Laschen der zweiten, radial inneren Verbindungsplatte dienen in diesem Fall zum Abdecken der Zwischenräume zwischen den Laschen der radial inneren Verbindungsplatte und bilden somit Abdeckbereiche. In anderen Worten ausgedrückt, sollte die zweite, radial innere Verbindungsplatte/ Abdeckplatte (kurz: Platte), die die Öffnungen der ersten Platte abdeckt, zwischen der ersten, radial äußeren Platte und den Segmenten/ Kippelementen liegen. Die zweite Platte ist insbesondere an nur einer Stelle in Endoskop-Längsrichtung zu der ersten Platte lagefestgelegt (d.h. an (genau) einem der Segmente oder (genau) einem der Wirbelkörper des Deflectings oder dem Endoskopkopf/ distalen Kopfabschnitt und/oder an der ersten Platte befestigt). Insbesondere kann sich die zweite Platte somit gegen die erste Platte verschieben und ist trotzdem eingesperrt, d.h. im Abklappmechanismus sowohl im neutralen als auch abgeklappten/ abgekrümmten Zustand entlang der Endosdoskop-Längsrichtung geführt (derart, dass sie nicht radial nach außen über die Segmente hinaus abstehen kann). Demgemäß können sich bei einem Abkrümmen des Abklappmechanismus die Laschen der radial äußeren, zweiten Verbindungsplatte sich schuppenartig über die Laschen der radial inneren, ersten Verbindungsplatte schieben.

Vorzugsweise sind die Laschen der radial äußeren Verbindungsplatte in der Endoskop-Längsrichtung breiter als die Laschen der radial inneren Verbindungsplatte. Weiter vorzugsweise sind die Laschen der zweiten, inneren Platte in der Endoskop-Längsrichtung schmaler als ein Abstand zwischen zwei benachbarten Segmenten, derart, dass sie beim Abklappen/ Abkrümmen des Abklappmechanismus nicht gegen die Segmente stoßen.

Alternativ können die Segmente wechselweise an der ersten, radial inneren und der zweiten, radial äußeren Verbindungsplatte angebracht sein.

Insbesondere sind die erste und die zweite Verbindungsplatte jeweils nur halb so dick in ihrer Plattendicke/ Blechdicke (d.h. im montierten Zustand in Endoskop-Radialrichtung betrachtet), verglichen mit einer Anordnung, bei welcher eine einzelne Verbindungsplatte vorgesehen ist.

Insbesondere ist der offenbarungsgemäße Abklappmechanismus zur Halterung des Endoskopkopfes am distalen Ende eines (Endoskop-) Deflectings vorgesehen und ausgebildet. Dies ermöglicht es, den Endoskopkopf zum einen durch den Abklappmechanismus stark/ eng abzuklappen, um auch bei Untersuchungen von sehr engen Körperhöhlen eines Patienten einen Blick nach hinten oder zur Seite zu ermöglichen, und zum anderen ein weitläufiges Abkrümmen des Endoskopkopfs durch das Deflecting zu erreichen, um auch in größeren Körperhöhlen eine sichere Navigation bereitzustellen. Das heißt, bevorzugt ist ein Biegeradius des Abklappmechanismus, wenn dieser vollständig abgeklappt ist, kleiner als ein Biegeradius des Endoskop-Deflectings, wenn dieses vollständig abgekrümmt ist. Das Endoskop ist vorzugsweise als Duodenoskop geeignet oder ausgebildet, wobei die rückwärtsgewandte oder laterale Ausrichtung einer Optik des Endoskopkopfs durch den Abklappmechanismus bereitgestellt wird.

Ferner ist es von Vorteil, wenn das offenbarungsgemäße Endoskop einen Hüllschlauch oder Endoskopschlauch (einen sogenannten "Overtube" / Schlauchtülle / Ummantelung; kurz: Schlauch) aufweist, der separat zu dem Abklappmechanismus und insbesondere diesen in Umfangsrichtung (im Wesentlichen vollständig) umgebend/umschließend bereitgestellt ist. Der Endoskopschlauch dient dazu, bei einer Nutzung des Endoskops Patientengewebe vor einer Verletzung durch den Abklappmechanismus, z.B. durch Einklemmen, zu schützen. Ferner verhindert der Endoskopschlauch ein Verrutschen oder Verdrehen der Segmente (und ggf. des Endoskopkopfs) zueinander um die Scharnierabschnitte. D.h., der Schlauch hält die Segmente (und ggf. den Endoskopkopf) in einer zueinander fluchtenden Lage. Bei ähnlichen Systemen werden üblicherweise Spanndrähte verwendet, auch welche verzichtet werden kann und somit die Herstellungskosten, insbesondere Montagekosten, gesenkt werden können. In anderen Worten ausgedrückt bildet der Schlauch eine, insbesondere zumindest in Umfangsrichtung geschlossene, Hülle um den Abklappmechanismus. Der Schlauch kann ferner an einem distalen (dem Patienten zugewandten) Ende geschlossen sein und über den Endoskopkopf bzw. das letzte Segment des Abklappmechanismus gestülpt sein. Alternativ oder zusätzlich kann der Schlauch am Endoskopkopf fixiert sein.

Vorzugsweise ist ein Kanaleinstellmechanismus bereitgestellt, welcher es ermöglicht, einen Durchmesser des Arbeitskanals zu vergrößern und/oder zu verkleinern. Dies ist von Vorteil, da somit der Arbeitskanal schnell und einfach für die Verwendung mit größeren oder kleineren Instrumenten anpassbar ist, ohne dass dafür zusätzliche Werkzeuge benötigt werden oder unterschiedlich große Segmente hergestellt werden müssen. D. h. eine Stückzahl der herzustellenden Segmente ist erhöht und die Fertigungskosten sind entsprechend gesenkt, ohne dass eine Variabilität von gewünschten Arbeitskanaldurchmessern eingebüßt wird. In anderen Worten ausgedrückt, ist ein Kanaleinstellmechanismus bereitgestellt, der zur Vergrößerung und/oder Verkleinerung eines Arbeitskanaldurchmessers konfiguriert ist, und der dadurch bereitgestellt wird, dass die die Segmente im Keilrückenabschnitt sowie die Verbindungsplatte nach radial innen und/oder außen bezüglich der Endoskop-Längsrichtung aufbiegbar und/oder zubiegbar konfiguriert sind.

Der Kanaleinstellmechanismus wird dadurch bereitgestellt, dass Segmentflügel der Segmente, welche den Arbeitskanal zumindest teilweise umgreifen, (Enden der Sichelform der Segmente/dem Keilrückenabschnitt bildende Segmentteile) derart ausgebildet sind, dass sie elastisch und/oder plastisch radial nach außen aufbiegbar und/oder radial nach innen zubiegbar sind. In anderen Worten ausgedrückt, sind die Segmentflügel derart biegbar, dass deren Spitzen voneinander weg bzw. aufeinander zu verbogen werden können.

Ferner weist die Verbindungsplatte (Rückenelement) bevorzugt Kanaleinstellstrukturen auf, welche die Biegesteifigkeit der Verbindungsplatte lokal verringern, derart, dass die Verbindungsplatte definiert nach radial innen und/oder außen bezüglich der Endoskop-Längsrichtung aufbiegbar und/oder zubiegbar konfiguriert ist. In anderen Worten ausgedrückt, weist die Verbindungsplatte (auch bezeichnet als Rückenelement) zumindest an den Halteabschnitten (insbesondere an einem Übergang zu zwischen den jeweiligen Befestigungs- und Deckbereichen oder einem dem Deckbereich zugewandten Teil des Befestigungsbereichs), optional auch an den Deckbereichen und/oder an den Scharnierabschnitten, Kanaleinstellstrukturen auf. Vorzugsweise sind die Kanaleinstellstrukturen ausschließlich an den Befestigungsbereichen, insbesondere nur an einem Übergang zu den jeweiligen Deckbereichen, vorgesehen. Jeder Halteabschnitt der Verbindungsplatte ist mit zumindest einer Kanaleinstellstruktur versehen. Die Kanaleinstellstrukturen ermögliche oder vereinfachen ein Auf- und Zubiegen der Halteabschnitte, und ggf. auch der Deck- und Scharnierabschnitte (des Mittenabschnitts), nach radial außen bzw. innen bezüglich der Endoskop-Längsachse. Das heißt, die Kanaleinstellstrukturen stellen Soll-Biegestellen dar, die ein definiertes, einfaches Biegen des Verbindungselements, insbesondere von dessen Befestigungsbereichen relativ zu dessen Deckbereichen/Mittenabschnitt, zulässt, um dessen Wölbung in Endoskop-Umfangsrichtung anzupassen.

Die Kanaleinstellstrukturen können beliebige Strukturen sein, die eine Biegesteifigkeit des Rückelements definiert und lokal verringern. Bevorzugt sind entlang der Verbindungsplatte (ausschließlich) gleiche Kanaleinstellstrukturen vorgesehen. Es ist jedoch auch denkbar, an unterschiedlichen Halte- und/oder Deck- und/oder Scharnierabschnitten unterschiedliche Kanaleinstellstrukturen bereitzustellen. Insbesondere ist es von Vorteil, an den am weitesten proximal und/oder an am weitesten distal liegenden Halteabschnitten, die zur Befestigung am Endoskopschaft bzw. -deflecting bzw. -kopf dienen, unterschiedliche Kanaleinstellstrukturen vorzusehen. Ferner ist es denkbar, an den Befestigungsbereichen unterschiedliche Kanaleinstellstrukturen als am Mittenabschnitt bereitzustellen.

Vorzugsweise sind die Kanaleinstellstrukturen (alle oder ein Teil davon) über Schlitze in der Verbindungsplatte bereitgestellt, die so dünn sind, dass diese ein Abgleiten des chirurgischen Instruments am Rückenpanzer nicht beeinträchtigen, selbst wenn die Schlitze in den Deckbereichen und Scharnierabschnitten eingebracht sind. Ein Schlitz durchdringt die Verbindungsplatte und öffnet sich sowohl in einer radial außenals auch innenliegenden Verbindungsplattenfläche. Jeder Halteabschnitt oder jeder Befestigungsbereich der Verbindungsplatte kann mit zumindest einem Schlitz als einer Kanaleinstellstruktur versehen sein. Vorzugsweise ist ein Raster von Schlitzen als eine Kanaleinstellstruktur vorgesehen. Die Schlitze sind vorzugsweise V-förmig und derart ausgerichtet, dass eine Spitze der V-Form in Endoskop-Umfangsrichtung nach außen oder innen zeigt. Alternativ können die Schlitze geradlinig in Endoskop-Längsrichtung verlaufen und nach Art einer Perforierung nacheinander angeordnet sein, sodass entlang der Perforierung die Biegesteifigkeit der Verbindungsplatte reduziert ist und eine Längserstreckung der Perforierung die Biegeachse definiert. Alternativ können die Schlitze in Endoskop-Umfangsrichtung verlaufen, sodass zumindest ein Abschnitt der Halteabschnitte/der Befestigungsbereiche lamellenartig oder gitterrostartig ausgebildet ist. Ferner können diagonale Schlitzverläufe vorgesehen sein.

Alternativ oder zusätzlich können die Kanaleinstellstrukturen (alle oder ein Teil davon) über Variationen eines Querschnittsprofils der Verbindungsplatte bereitgestellt sein. Beispielsweise kann die Materialdicke der Verbindungsplatte falzartig verringert sein, sodass eine oder mehrere Rille(n) bereitgestellt ist/sind, insbesondere derart, dass diese sich in Endoskop-Längsrichtung über jeden Halteabschnitt/jeden Befestigungsbereich erstreckt/erstrecken. Die jeweiligen Rillen können nur an einer radialen Außenseite oder nur an einer radialen Innenseite der Verbindungsplatte vorgesehen sein. Alternativ können die jeweiligen Rillen sowohl radial innen als auch radial außen derart eingebracht sein, dass sie einander jeweils gegenüberliegen oder dass sie zueinander (insbesondere in Endoskop-Umfangsrichtung) versetzt sind. Ähnlich wie die Schlitze können auch die Rillen in Endoskop-Längsrichtung oder in Endoskop-Umfangsrichtung oder diagonal dazu verlaufen. Alternativ oder zusätzlich kann ein Querschnittsprofil eines der Halteabschnitte, insbesondere am Übergang zwischen den Befestigungs- und Deckbereichen, gewellt sein, um eine Kanaleistellstruktur bereitzustellen. Die zumindest eine Welle erstreckt sich in Endoskop-Längsrichtung über den gesamten Halteabschnitt/Befestigungsbereich, sodass entlang der Welle die Biegesteifigkeit des Halteabschnitts/Befestigungsbereich reduziert ist, wobei die Wellenlängserstreckung die Biegeachse definiert.

Alternativ oder zusätzlich kann als die Kanaleinstellstruktur(en) ein Abschnitt mit verringerter Materialfestigkeit relativ zu der restlichen Verbindungsplatte sein, sodass in diesem Bereich die Biegesteifigkeit reduziert ist. Beispielsweise ist dieser Abschnitt an einem Übergang zwischen den Befestigungsbereichen und den Deckbereichen vorgesehen. Vorzugsweise sind ein oder mehrere in Endoskop-Längsrichtung verlaufende Abschnitte mit verringerter Materialfestigkeit an jedem Halteabschnitt/Befestigungsbereich bereitgestellt. Diese können beispielsweise aus einem unterschiedlichen Material hergestellt sein oder können durch eine Materialbehandlung bereitgestellt sein. Ggf. können die gesamten Befestigungsbereiche aus einem Material mit geringerer Biegesteifigkeit als die Scharnierabschnitte oder der gesamte Mittenabschnitt der Verbindungsplatte hergestellt sein. Z.B. können die Befestigungsbereiche aus einem Kunststoff hergestellt sein und zumindest die Scharnierabschnitte, vorzugsweise auch die Deckbereiche (d.h. der gesamte Mittenabschnitt), aus einem Federstahl gefertigt sein.

Nach einer weiten vorteilhaften Ausgestaltung der Offenbarung ist das Segment in zwei (vorzugsweise separate) Teilsegmente geteilt, die über laschenartige Befestigungsbereiche der Verbindungsplatte an dieser angebracht und miteinander verbunden sind. Zwischen den Teilsegmenten an einer dem Arbeitskanal abgewandten Seite (insbesondere dem Keilspitzenabschnitt) des Zylindermantels des Segments ist ein (Radial-) Spalt ausgebildet. Die Verbindungsplatte / die Befestigungsbereiche wirkt/en als eine Spreizfeder, welche die zwei Teilsegmente spreizt, um den Spalt zu erweitern (derart, dass sich zwischen den Teilsegmenten ein Winkel öffnet), um innerhalb des gespreizten Spalts einen Raum für einen zusätzlichen Arbeitskanal bereitzustellen. Die Befestigungsbereiche umgreifen die Teilsegmente zumindest teilweise und werden in einem Auslieferungszustand des Endoskops über ein Riegelelement in einem elastisch nach radial innen verformten Zustand gehalten, in welchem die Teilsegmente nahe zueinander, insbesondere im Wesentlichen aneinander anliegend (ggf. mit einer zwischenliegenden Auskleidung oder einem zwischenliegenden Zusatzschlauch, wie nachfolgend genauer beschrieben) positioniert sind. In diesem Zustand ist die Spreizfeder vorgespannt und der Spalt im Wesentlichen geschlossen. Bei einem Entfernen des Riegelelements wird der Spalt durch die Vorspannung der Spreizfeder geöffnet. Das Riegelelement kann ein schnurartiges Element wie ein Riegeldraht sein, der durch Ösen der Befestigungsbereiche gefädelt ist und nach Richtung proximal abgezogen werden kann, wenn ein zusätzlicher Arbeitskanal benötigt wird. Alternative Riegelelemente, wie z.B. ein abziehbarer Kompressions-Schlauch o.Ä. sind denkbar.

Der Zusatzarbeitskanal bzw. der Spalt ist vorzugsweise mit einer Schlauchfolie ausgekleidet, welche im geschlossenen Zustand des Raums / des Zusatzarbeitskanals gefaltet in dem Spalt liegt und in Endoskop-Längsrichtung durch den Abklappmechanismus verläuft, um den Spalt abzudichten. Die Auskleidung ist vorzugsweise aus einem dünnen EPTFE- oder TPU-Material gefertigt. Wird der Spalt geöffnet, faltet sich die Auskleidung auf, um den Spalt am Keilspitzenabschnitt zu überbrücken / zu schließen. Alternativ oder zusätzlich kann ein elastischer Zusatzschlauch bereitgestellt sein, der derart zwischen den Teilsegmenten in dem Schlitz angeordnet (vorzugsweise beidseitig an den Teilsegmenten angeklebt) ist, dass dieser durch das Spreizen der Teilsegmente entfaltet wird, um als Zusatzarbeitskanal zu dienen. In dem Zusatzschlauch bzw. innerhalb der Auskleidung kann bevorzugt eine entfernbare Folie o.Ä. bereitgestellt sein, um ein Verkleben des Schlauchs oder der Auskleidung im geschlossenen Zustand zu verhindern.

Vorzugsweise ist jedes Segment spiegelsymmetrisch bezüglich einer durch den Arbeitskanal und die Endoskop-Längsachse verlaufende Ebene in zwei separate Hälften geteilt und wird demgemäß durch zwei (im Wesentlichen halbzylindrische) Teilsegmente gebildet, derart, dass jedes Teilsegment einen Teil-Zylindermantel bildet. In diesem Fall hat jedes Teilsegment am Keilrückenabschnitt einen Rücksprung, derart, dass die Rücksprünge der beiden aneinander angeordneten Teilsegmente einander gegenüberliegen und zusammen den Arbeitskanal bilden (d.h., jedes Teilsegment bildet eine Teil-Umfangswand des Arbeitskanals). Radial außenliegende Abschnitte der Rücksprünge sind voneinander beabstandet, sodass im Bereich des Keilrückenabschnitts der Zylindermantel durch die Rücksprünge bzw. den durch diesen gebildeten Arbeitskanal durchbrochen ist.

Alternativ wird die der Offenbarung zugrundeliegende Aufgabe durch einen Abklappmechanismus und insbesondere durch ein (medizinisches) Endoskop mit den Merkmalen des Anspruchs 14 gelöst.

Genauer ausgedrückt, wird ein einen Endoskopschaft aufweisendes Endoskop mit einem distalen, manuell aktuierbaren Abklappmechanismus zur kontrolliert abklapp- oder abwinkelbaren Halterung eines Endoskopkopfes oder distalen Kopfabschnitts an einem dem Abklappmechanismus proximal vorgelagerten Endoskopabschnitt bereitgestellt. Der Abklappmechanismus hat eine Anzahl axial aufeinanderfolgender und mittels zumindest eines gemeinsamen Betätigungselements aktiv gegeneinander winkelverstellbarer, im Wesentlichen zylindrischer Segmente, welche jeweils zwei im Wesentlichen keilförmig zueinander ausgerichtete axiale Stirnseiten und einen Zylindermantel aufweisen, der an einem Zylindermantelabschnitt mit maximaler axialer Länge einen Keilrückenabschnitt definiert, an welchem die Anzahl der Segmente aneinander (bzw. am Endoskopkopf/Kopfabschnitt und/oder am proximal vorgelagerten Endoskopabschnitt) angelenkt sind, und welche in Endoskop-Längsrichtung jeweils zumindest eine Durchgangsöffnung aufweisen, derart, dass die Anzahl der Segmente gemeinschaftlich einen Arbeitskanal ausbildet (wobei jedes Segment einen insbesondere umlaufenden / in Umfangsrichtung durchgehenden bzw. geschlossenen Arbeitskanalwandabschnitt hat). In dem Arbeitskanal ist ein(e) flexible(s), sich in der Endoskop-Längsrichtung entlang der Anzahl der (insbesondere mehreren) Segmente, vorzugsweise entlang des gesamten Abklappmechanismus, erstreckende/s Abgleitband/ Abgleitbandeinrichtung/ Abgleitplatte derart eingelegt, dass das/die Abgleitband/ Abgleitbandeinrichtung/ Abgleitplatte einen keilrückenseitigen Abschnitt einer Arbeitskanalwand des Arbeitskanals (d.h., den Arbeitskanalwandabschnitt der jeweiligen Segmente) (zumindest teilweise) bedeckt (und einen zwischen den Segmenten vorliegenden Bereich, z.B. Lücken/Kanten/Winkel) und eine durchgängige, glatte Abgleitfläche für in den Arbeitskanal eingeführte medizinische Instrumente bildet.

In anderen Worten ausgedrückt, entspricht der Abklappmechanismus in seinem grundlegenden Aufbau dem Abklappmechanismus nach der vorstehend beschriebenen Lösung nach Anspruch 1, mit entlang der Endoskop-Längsrichtung zueinander angeordneten, zylindrischen Segmenten, die in einer Seitenansicht keilförmig ausgebildet sind und die am Keilrückenabschnitt aneinander angelenkt sind, um zueinander über eine Betätigung eines Betätigungselements winkelverstellbar/abklappbar zu sein, derart, dass eine Lücke zwischen den Keilspitzenabschnitten der Segmente beim Abklappen geschlossen wird. Im Unterschied zu Anspruch 1 hat bei der Lösung nach Anspruch 14 jedes Segment eine Durchgangsöffnung/-bohrung mit einer durchgehenden/umlaufenden/geschlossenen Umfangswand (Arbeitskanalwandabschnitt), wobei die Durchgangsöffnungen der Segmente gemeinsam einen Arbeitskanal definieren. Eine flexible, vorzugsweise elastische, langgestreckt rechteckige Abgleitplatte/-bandeinrichtung ist in den Arbeitskanal keilrückenseitig (an einer dem Keilrückenabschnitt zugewandten Seite / an der Seite, wo die Segmente aneinander angelenkt sind) eingelegt und erstreckt sich durch mehrere, insbesondere alle Segmente des Abgleitmechanismus sowie über zwischen den Segmenten gebildete Lücken oder kanten/Winkel. Zusammenfassend sind die Segmente über separat zu der Abgleitplatte/-bandeinrichtung ausgebildete Gelenke miteinander verbunden und die Abgleitplatte/-bandeinrichtung überdeckt die Gelenke.

Beim Abkrümmen des Abklappmechanismus werden die Segmente zueinander abgewinkelt, derart, dass die Keilspitzenabschnitte aufeinander zubewegt werden, ggf. aneinander zur Anlage kommen. Somit sind auch die Arbeitskanalwandabschnitte benachbarter Segmente winklig zueinander, ggf. zusätzlich mit Lücken zwischen den Segmenten, insbesondere im nicht vollständig abgeklappten Zustand. Die Abgleitplatte-/bandeinrichtung überbrückt die Winkel und ggf. die Lücken und wölbt sich darüber, sodass durch die Abgleitplatte-/bandeinrichtung eine glatte, gerundete Abgleitfläche am äußeren Krümmungsradius (entlang der Keilrückenabschnitte der zueinander abgeklappten Segmente) bereitgestellt wird. Die Abgleitplatte/-bandeinrichtung hat eine Härte oder die Abgleitfläche ist gehärtet, derart, dass ein gleichmäßiges, glattes Abgleiten des Instruments ermöglicht ist (ohne dass die Abgleitfläche wesentlich, d.h., über oberflächliche Längskratzer hinaus, beschädigt wird).

In anderen Worten ausgedrückt, bildet die Abgleitplatte/ Abgleitbandeinrichtung ein Versteifungselement aus, welches an einem keilrückenseitigen Bereich in den Arbeitskanal (insbesondere zwischen den Innenschlauch des Arbeitskanals und die Innenwand der Segmente/ Kippkörper) eingelegt ist.

Vorzugsweise ist die Abgleitbandeinrichtung an einem distalen Endbereich des Abklappmechanismus, insbesondere (ausschließlich) an dem Endoskopkopf oder distalen Kopfabschnitt oder an einem (einzelnen) am weitesten distal angeordneten Segment (optional stattdessen an einem (einzelnen) beliebigen anderen Segment, insbesondere am ersten, zweiten oder dritten Segment vom distalen Kopfabschnitt oder Endoskopkopf aus gezählt), beispielsweise klebend/geschweißt/gelötet fixiert (dreh- und axialfest / ohne Freiheitsgrade).

Weiter vorzugsweise liegt die Abgleitbandeinrichtung in der Endoskop-Längsrichtung verschiebbar in den Durchgangsöffnungen einer Anzahl von weiter proximal angeordneten (oder im Falle der Befestigung an einem anderen Segment, in den distal und/oder proximal dazu angeordneten) Segmenten (vorzugsweise in allen weiteren), derart, dass die Abgleitbandeinrichtung beim Abkrümmen des Abklappmechanismus in dem Arbeitskanal verrutschen kann, um eine Dehnung oder Stauchung der Abgleitbandeinrichtung in Endoskop-Längsrichtung im Wesentlichen zu vermeiden. In anderen Worten ausgedrückt ist die Abgleitbandeinrichtung an einem (einzelnen) Element (aus den Segmenten und dem distalen Kopfabschnitt oder dem Endoskopkopf) des Abklappmechanismus befestigt und verläuft lose durch die übrigen Elemente, sodass die Abgleitplatte/-bandeinrichtung auch bei einer Längenänderung des Arbeitskanals aufgrund des Abklappens des Abklappmechanismus sich in Längsrichtung längenausgleichend verschiebt und sich nicht wellt, d.h., dabei gerade und glatt bleibt.

Ist die Abgleitbandeinrichtung/die Abgleitplatte an der Fixierstelle lagefest angebracht und liegt ansonsten lose in dem Arbeitskanal, wölbt sich beim Abklappen des Abklappmechanismus die Abgleitplatte/ Abgleitbandeinrichtung der Krümmung des Abklappmechanismus im Wesentlichen folgend, derart, dass sie die Wände (mehrere oder alle) der Durchgangsöffnungen (d.h. die Arbeitskanalwandabschnitte, die durch die einzelnen Segmente gebildet werden) lediglich tangiert. Somit werden im abgeklappten Zustand des Abklappmechanismus ein Winkelunterschied und/oder Lücken durch die Abgleitplatte/ Abgleitbandeinrichtung überbrückt, die somit eine glatte, (im Wesentlichen gleichmäßig) gebogene Abgleitfläche für das Instrument beim Vorschieben durch den Arbeitskanal bereitstellt.

Ferner ist es von Vorteil, wenn ein proximales (loses/unbefestigtes) Ende der Abgleitplatte/-bandeinrichtung in der Endoskop-Längsrichtung verschiebbar in den dem Abklappmechanismus proximal vorgelagerten Endoskopabschnitt hineinragt. Somit wird erreicht, dass das lose Ende der Abgleitbandeinrichtung/Abgleitplatte sich nicht zwischen Segmenten verhaken kann und z.B. ein Abklappen des Abklappmechanismus blockieren oder diesen beschädigen kann. D.h., das lose Ende ist sicher in den Arbeitskanal eingesteckt.

Darüber hinaus ist es bevorzugt, wenn die Abgleitbandeinrichtung elastisch ist und in einer Ruhestellung im Wesentlichen gerade verläuft, sodass die Abgleitbandeinrichtung beim Rückstellen des Abklappmechanismus aus einer abgeklappten Stellung in eine im Wesentlichen nach vorne ausgerichtete oder gestreckte Stellung in die gerade, ungebogene Ruhestellung strebt. Insbesondere ist es von Vorteil, wenn die Abgleitbandeinrichtung / die Abgleitplatte in der Endoskop-Längsrichtung derart steif ist, dass Kräfte, die während einer Nutzung des Abklappmechanismus auf die Abgleitplatte/ Abgleitbandeinrichtung wirken, stets zu Wölbungen der Abgleitplatte mit großem Radius führen und z.B. eine lokale Wellenbildung oder ein Verschieben oder Verdrehen der Abgleitbandeinrichtung in dem Arbeitskanal vermieden werden. Ferner ist es bevorzugt, wenn die Abgleitbandeinrichtung in Endoskop-Längsrichtung im Wesentlichen undehnbar ist.

Vorzugsweise ist die Abgleitbandeinrichtung insgesamt bandförmig/ plattenförmig (d.h. eine Abgleitplatte) und bildet eine (insbesondere rechteckige) geschlossene, durchbruchsfreie Fläche.

Alternativ bevorzugt kann die Abgleitbandeinrichtung mehrere Längselemente aufweisen, welche sich in Endoskop-Längsrichtung, insbesondere parallel zu einander, erstrecken. Die Längselemente können in Form von Längsstreifen ausgebildet sein, zwischen welchen Längsschlitze vorgesehen sind. Insbesondere können die Längsstreifen sich relativ zueinander bewegen. Demgemäß können unterschiedliche (Längs-) Verschiebungen der Längsstreifen über die Breite der Abgleitbandeinrichtung (d.h., abhängig von Umfangsposition der Längsstreifen am Abklappmechanismus bezogen auf die Gelenke zwischen den Segmenten) bereitgestellt werden, um vorteilhafterweise auszugleichen, dass der Abklappmechanismus sich an einer Keilrückenseite der Segmente (im Bereich der Gelenke) nicht verkürzt, an einer Keilspitzenseite jedoch stark verkürzt. Beispielsweise kann die Abgleitbandeinrichtung gabelförmig ausgebildet sein, d.h. sie kann mehrere zinkenartige Längsstreifen aufweisen, die an ihrem distalen Ende über einen Quersteg verbunden sind. Am Quersteg kann die Abgleitbandeinrichtung an einem distalen Bereich des Abklappmechanismus oder am Endoskopkopf befestigt sein, derart, dass die Längsstreifen sich in proximaler Richtung durch den Abklappmechanismus erstrecken. Die Längsstreifen sind in diesem Fall im Arbeitskanal (zwischen der Segment-Innenwand/ Arbeitskanalwand der Segmente und einem Innenschlauch des Arbeitskanals) entlang des Abklappmechanismus frei gleitend / längsverschiebbar zu den Segmenten angeordnet.

Alternativ oder zusätzlich bevorzugt kann die Abgleitbandeinrichtung als Längselemente mehrere in Endoskop-Längsrichtung verlaufende Drähte aufweisen. Die Drähte sind in den Arbeitskanal (zwischen den Innenschlauch und die Segmentinnenwand/ Arbeitskanalwand der Segmente) eingelegt und sind optional in Rillen an der Arbeitskanalwand geführt. Die Drähte können an ihren distalen Endbereichen, insbesondere ihren distalen Enden, an dem distalen Bereich des Abklappmechanismus oder am Endoskopkopf befestigt sein und ansonsten frei gleitend im Arbeitskanal liegen. Somit können die unterschiedlichen Verschiebungen der Abgleitbandeinrichtung über die Breite der Abgleitbandeinrichtung besonders einfach ermöglicht werden.

Insbesondere kann die vorstehend beschriebene Lösung nach Anspruch 1 modifiziert werden, indem das Endoskop zusätzlich zu der Verbindungsplatte/ dem Rückenelement mit einer vorstehend beschriebenen Abgleitbandeinrichtung versehen wird. D.h., zum einen sind die Segmente im Keilrückenabschnitt durch ein/e flexible/s, vorzugsweise elastische/s, Verbindungsplatte/ Rückenelement in Endoskop-Längsrichtung miteinander verbunden, welche unter Abdeckung der jeweiligen Längsschlitze an der jeweiligen Segmentaußenseite fixiert ist. Zum anderen ist in dem Arbeitskanal eine flexible, sich in der Endoskop-Längsrichtung entlang mehrerer Segmente, vorzugsweise entlang des gesamten Abklappmechanismus, erstreckende Abgleitbandeinrichtung (kann auch als zusätzliches Rückenelement betrachtet werden) derart eingelegt, dass sie die Verbindungsplatte und/oder die jeweiligen Segmente im Bereich des Keilrückenabschnitts zumindest teilweise bedeckt. In anderen Worten ausgedrückt kann das Endoskop ferner mit einer Abgleitbandeinrichtung versehen sein, welche flexible ist, sich in der Endoskop-Längsrichtung entlang mehrerer Segmente, vorzugsweise entlang des gesamten Abklappmechanismus, erstreckt und derart in den Arbeitskanal eingelegt ist, dass die Abgleitbandeinrichtung die Verbindungsplatte und/oder die jeweiligen Segmente am Keilrückenabschnitt zumindest teilweise bedeckt.

Insbesondere ist die Abgleitbandeinrichtung an einem Übergangsbereich zwischen den Segmenten und der Verbindungsplatte angeordnet und bedeckt bevorzugt den Übergangsbereich. Vorteilhafterweise kann die Abgleitbandeinrichtung unmittelbar an einem am weitesten keilrückenseitig liegenden Ende (in Umfangsrichtung betrachtet) der Segmente (d.h. in Verlängerung der sichelförmigen Kontur/ der Sichelspitzen der Segmente) angeordnet sein. In andere Worten ausgedrückt, kann eine Abgleitbandeinrichtung als ein oder mehrere elastische Verstärkungsprofile in den Arbeitskanal eingelegt werden. Insbesondere kann die Abgleitbandeinrichtung in zumindest einen Freiraum/ Raum zwischen dem Innenschlauch des Arbeitskanals und dem Keilrückenabschnitt der Kippkörper/ Segmente (bei einem geschlossenen Arbeitskanal) bzw. der Verbindungsplatte (bei einem Arbeitskanal, der die Zylinderwand des Segments keilrückenseitig durchbricht) eingelegt werden.

Nachfolgend wird eine weitere bevorzugte, ggf. unabhängig beanspruchbare Ausführungsform der vorliegenden Offenbarung beschrieben. Diese betrifft ein Endoskop mit einem mehrere (insbesondere rauten- oder keilförmige) aneinander verkippbar angelenkte Wirbelkörper aufweisenden (insbesondere vorstehend beschriebenen) Deflecting und einem proximal zum Deflecting angeordneten Endoskopschaft. Das Endoskop hat einen Betätigungsmechanismus mit einer Betätigungsfeder/ Spiralfeder (kurz: Feder), welche mit ihrem distalen Ende an einem distalen Bereich, insbesondere an einem am weitesten distal angeordneten Wirbelkörper des Deflectings, fixiert ist, sich durch das Deflecting und den Endoskopschaft erstreckt, und in Endoskop-Längsrichtung betätigbar ist, um ein Abkrümmen des Deflectings zu steuern.

Ein distaler Federbereich der Betätigungsfeder hat eine größere Steigung (Abstand zwischen zwei Windungen der Feder) als ein proximaler Federbereich, in welchem die Windungen vorzugsweise aneinander anliegen. Insbesondere liegt ein Übergangsbereich zwischen dem proximalen und dem distalen Federbereich endoskoplängs betrachtet im Bereich des Deflectings. Am distalen Ende der Feder bzw. des distalen Federbereichs ist ein Sperrdraht angebracht, welcher sich durch das Deflecting und den Endoskopschaft erstreckt und in Endoskop-Längsrichtung betätigbar ist. Vorzugsweise ist der Sperrdraht innerhalb der Feder geführt. Vorzugsweise ist der distale Federbereich in einer neutralen (durch den Betätigungsmechanismus unbetätigten/ unabgewinkelten) Stellung des distalen Endoskopbereichs (aufweisend zumindest das Deflecting) durch den Sperrdraht vorgespannt, was vorteilhafterweise eine Stabilität des Betätigungsmechanismus / des distalen Endoskopendes erhöht.

Bei einer Betätigung (einem Zug) des Sperrdrahts in proximaler Richtung in einem ersten Betätigungsbereich wird zunächst der distale Federbereich komprimiert bis die Feder blockiert (die Windungen der Feder im distalen Federbereich in Anlage kommen). Dadurch werden zunächst die am weitesten distal angeordneten Wirbelkörper des Deflectings, welche insbesondere distal von dem Übergangsbereich zwischen dem proximalen und distalen Federbereich angeordnet sind, relativ zueinander abgewinkelt. Der Sperrdraht kann im ersten Betätigungsbereich in der proximalen Richtung betätigt werden. Die Betätigungsfeder ist vorzugsweise an einem proximalen Ende durch eine Stützfeder (Druckfeder) gestützt, welche eine vorbestimmte Steifigkeit hat, die konfiguriert ist, um eine Bewegung der Betätigungsfeder in der proximalen Richtung während der Betätigung im ersten Betätigungsbereich des Sperrdrahts zu minimieren oder zu verhindern.

Wird der Sperrdraht und/oder die (blockierte) Betätigungsfeder nach der Betätigung im ersten Betätigungsbereich weiter in proximaler Richtung betätigt/ gezogen, wird eine Betätigung in einem zweiten Betätigungsbereich bereitgestellt. In diesem Fall bewegt sich der Sperrdraht zusammen mit der Betätigungsfeder (ggf. gegen die Stützfeder/ die Stützfeder komprimierend) in proximaler Richtung, um auch die proximal angeordneten Wirbelkörper des Deflectings abzuwinkeln, welche sich insbesondere proximal von dem Übergangsbereich befinden. Somit kann das Deflecting, welches zur Steuerung/ Navigation des Endoskops in einem Patientenhohlraum dient, in mehrstufigen Betätigungsbereichen abgewinkelt werden, was eine besonders feinfühlige Steuerung ermöglicht.

Ferner kann das Endoskop einen (insbesondere vorstehend beschriebenen) distalen Abklappmechanismus/ Kippkopf mit mehreren keilförmigen, aneinander verkippbar angelenkten Segmenten aufweisen, welcher distal zum Deflecting angeordnet ist und in einem kleineren Winkel abkrümmbar ist als das Deflecting. Der Betätigungsmechanismus kann einen Steuerdraht (Zug-Druck-Draht / Bowdenzug) aufweisen, welcher an einem distalen Bereich des Abklappmechanismus oder eines distal dazu angeordneten Elements (z.B. einem Endoskopkopf) fixiert ist, sich durch den Abklappmechanismus, das Deflecting und den Endoskopschaft erstreckt, und in Endoskop-Längsrichtung betätigbar ist, um eine Abkrümmung des Abklappmechanismus zu steuern. Insbesondere ist der Steuerdraht durch die Betätigungsfeder geführt. Somit können der Steuerdraht und die Betätigungsfeder optimal gegeneinander vorgespannt sein/ gegeneinander arbeiten, was eine Stabilität des Betätigungsmechanismus/ des distalen Endoskopbereichs (aufweisend zumindest das Deflecting und den Abklappmechanismus) erhöht.

In der neutralen Stellung des distalen Endoskopbereichs ist das Deflecting vorzugsweise durch den Betätigungsmechanismus unbetätigt/ unabgekrümmt und ist die Betätigungsfeder, wie vorstehend beschrieben, durch den Sperrdraht vorgespannt. Wird der Steuerdraht ausgehend von der neutralen Stellung des distalen Endoskopbereichs in proximaler Richtung betätigt (gezogen), werden die Segmente des Abklappmechanismus relativ zueinander abgewinkelt. Somit kann z.B. eine Abwinkelung des Abklappmechanismus um 120° zur Endoskop-Längsachse/ zum proximal unmittelbar dazu vorgelagerten Endoskopabschnitt (dem Deflecting) erreicht werden. Insbesondere erreicht der Abklappmechanismus einen besonders kleinen Krümmungsradius und ist somit besonders geeignet, um in einem engen Raum eine laterale oder retrograde Ausrichtung eines Endoskopkopfes/ eines distalen Kopfabschnitts zu ermöglichen.

Die Vorspannung des distalen Federbereichs der Betätigungsfeder und ggf. eine Vorspannung der Stützfeder ist vorzugsweise derart ausgelegt/ eingestellt, dass eine Abkrümmung des Deflectings durch die Betätigung des Steuerdrahts minimiert oder verhindert wird. Somit kann die Betätigung des Abklappmechanismus zuverlässig von der Betätigung des Deflectings entkoppelt werden.

Vorzugsweise werden der Steuerdraht sowie der Sperrdraht und ggf. die Betätigungsfeder motorisch angetrieben, um diese unabhängig voneinander in Längsrichtung betätigen zu können. Wird das Deflecting im ersten oder zweiten Betätigungsbereich betätigt, wird vorzugsweise gleichzeitig der Steuerdraht zur Betätigung des Abklappmechanismus betätigt, um eine Längenänderung des Steuerdrahts, welche durch das Abkrümmen des Deflectings bedingt ist, auszugleichen, um die Stellung des Abklappmechanismus beizubehalten. Alternativ kann der Steuerdraht betätigt werden, um eine gewünschte Krümmung von beispielsweise bis zu 180° des distalen Endoskopbereichs als Ganzes (d.h. des Abklappmechanismus und des Deflectings) einzustellen. Somit können zur Steuerung/Navigation des Endoskops in dem Patientenhohlraum der Abklappmechanismus/ Kippkopf und das Deflecting gemeinsam verwendet werden und bildet der Abklappmechanismus z.B. bei der Steuerung um Kurven kein ungelenkes, ausholendes Hindernis.

Durch die Motorsteuerung/ den motorischen Antrieb ist es beispielsweise auch möglich, dass parallel mit der Abwinkelung der am weitesten distal angeordneten Deflectingelemente (d.h. die entsprechenden Wirbelkörper des Deflectings) auch die Kippkopfelemente (d.h. die Segmente des Abklappmechanismus) abgewinkelt werden. Bei geeigneter Ansteuerung können die Kippkopfelemente auf den gleichen Biegeradius ausgesteuert werden wie die Deflectingelemente, so dass sich ein gleichmäßiger Biegeradius ergibt.

Vorzugsweise sind die Wirbelkörper des Deflectings durch ein weiteres Deflecting-Verbindungs-/Rückenelement (Rückenpanzer/ Verbindungsplatte) miteinander verbunden, welches im Wesentlichen gleich ausgebildet ist, wie das Verbindungs-/Rückenelement (Rückenpanzer/ Verbindungsplatte), über das die Segmente des Abklappmechanismus miteinander verbunden sind, wobei Abmessungen des Deflecting-Verbindungs-/Rückenelements (insbesondere eine Ausdehnung der Halte-/ bzw, Befestigungsabschnitte in der Endoskop-Längsrichtung und/oder - Umfangsrichtung und/oder ein Abstand zwischen den Halte- bzw. Befestigungsabschnitten) an die Wirbelkörper des Deflectings angepasst sind. Das Deflecting-Verbindungs-/Rückenelement kann integral mit dem Verbindungs-/Rückenelement ausgebildet sein, welches die Segmente des Abklappmechanismus verbindet. In anderen Worten ausgedrückt, kann das Deflecting ebenfalls eine Rückenfeder/ Verbindungs-/Rückenelement haben. Beide Bereiche (d.h. der Abklappmechanismus und das Deflecting) können mit einer (insbesondere einzelnen/ gemeinsamen) Feder (Rückenfeder/ Verbindungs-/Rückenelement) (d.h. am Keilrückenbereich) abgedeckt sein, die eine unterschiedliche Teilung (insbesondere zwischen Halte- und Scharnierbereichen) im Kippkopfbereich und Deflectingbereich aufweist.

Insbesondere sind die Wirbelkörper des Deflectings in einer Seitenansicht keilförmig und in einer Draufsicht zylindrisch ausgebildet, sind jedoch in Längsrichtung länger als die Segmente des Abklappmechanismus. Ein Keilrückenabschnitt des Deflectings kann an einer gleichen Umfangsposition angeordnet sein, wie der Keilrückenabschnitt der Segmente des Abklappmechanismus. Die (Wirbelkörperseitigen) Keilrückenabschnitte des Deflectings können durch das Deflecting-Verbindungs-/Rückenelement verbunden sein/ aneinander angelenkt sein. Ggf. kann zudem ein wie vorstehend bereits beschriebenes, in Längsrichtung zum Deflecting-Verbindungs-/Rückenelement versetztes zweites Verbindungs-/Rückenelement bereitgestellt sein, welches zum Verbindungs-/Rückenelement des Abklappmechanismus und/oder zum Deflecting radial nach innen versetzt angeordnet ist.

Nach einem ggf. unabhängig beanspruchbaren Aspekt der vorliegenden Anmeldung wird ein Steuerungsverfahren zum Steuern eines Endoskops mit einem distalen Endoskopkopf, einem proximal dazu angeordneten Abklappmechanismus zum Abklappen des Endoskopkopfs, und einem proximal zum Abklappmechanismus angeordneten Deflecting, insbesondere eines vorstehend beschriebenen Endoskops, bereitgestellt, wobei zumindest der Abklappmechanismus unabhängig zum Deflecting antreibbar ist.

Das Steuerungsverfahren kann bevorzugt das Endoskop in einen ersten Modus schalten, in welchem nur der Abklappmechanismus unabhängig vom Deflecting abklappbar ist oder abgeklappt wird. Weiter bevorzugt kann das Steuerungsverfahren das Endoskop in einem zweiten Modus schalten, in welchem das Deflecting abkrümmbar ist oder abgekrümmt wird, z.B. nachdem eine bestimmte, insbesondere vollständig abgeklappte, Stellung des Abklappmechanismus durch eine Steuerung im ersten Modus erreicht wurde.

Weiter bevorzugt kann das Steuerungsverfahren das Endoskop in einen dritten Modus schalten, in welchem der Abklappmechanismus und das Deflecting, insbesondere im Wesentlichen gleichzeitig, weiter insbesondere mit einem entlang des Abklappmechanismus und des Deflectings im Wesentlichen konstanten Krümmungsradius, gekrümmt bzw. abgeklappt werden oder abkrümmbar bzw. abklappbar sind.

Weiter bevorzugt kann das Steuerungsverfahren das Endoskop in einen vierten Modus schalten, in welchem der Abklappmechanismus weiter abklappbar ist oder abgeklappt wird, z.B. nachdem das Deflecting durch eine Steuerung im dritten Modus eine bestimmte, insbesondere maximale, Krümmung erreicht hat. Letztendlich ist es aber auch möglich, das Deflecting und/ oder den Abklappmechanismus im Rahmen ihrer jeweiligen geometrischen Möglichkeiten vorzugsweise wechselweise entgegengesetzt zu krümmen, um so eine S- bzw. umgekehrte S-Form zu realisieren (d.h., das Deflecting und/oder der Abklappmechanismus können überstreckt bzw. radial zu der Umfangs-Seite hin gekrümmt werden, an welcher das Rückenelement daran angebracht ist).

Bevorzugt kann das Steuerungsverfahren Daten über eine Position des Endoskopkopfs innerhalb eines Patientenkörpers abrufen oder empfangen. Diese Daten können von einem Positionserfassungsmittel wie z.B. einem Sensor am Endoskopkopf, einer gespeicherten Behandlungsplanung, einem Sensorsystem oder dergleichen stammen, welche bevorzugt Teil eines Behandlungssystems mit dem Endoskop sind. Weiter bevorzugt kann das Steuerungsverfahren das Endoskop abhängig von den ermittelten Daten über eine Position des Endoskops im Patientenkörper in den ersten, zweiten, dritten oder vierten Modus schalten. Beispielsweise kann das Steuerungsverfahren das Endoskop im ersten Modus betreiben, wenn der Endoskopkopf sich im Dünndarm in der Nähe der Papille befindet oder wenn es sich im Dickdarm befindet. Auf diese Weise kann zielgerichtet und einfach eine rückwärtsgewandte Sicht zur Suche nach der Papille bzw. nach Darmpolypen oder dergleichen bereitgestellt werden. Weiter beispielsweise kann das Steuerungsverfahren das Endoskop im dritten Modus betreiben, wenn der Endoskopkopf sich im Magen befindet. Dort ist viel Platz zur Navigation und die Lenkung des Endoskops kann somit optimiert werden. Wird ermittelt, dass der Endoskopkopf einen Endbereich des Magens erreicht, kann das Steuerungsverfahren beispielsweise das Endoskop in den vierten Modus schalten. Dadurch kann ein Ausgang des Magens einfacher zu finden sein und kann das Endoskop genauer gesteuert werden, um sich zum/durch den Magenausgang zu bewegen.

Das Endoskop kann ferner zumindest einen Antrieb zum Antreiben des Deflectings und einen Antrieb zum Antreiben des Abklappmechanismus haben. Die Steuervorrichtung kann dazu eingerichtet sein, die Antriebe und je nach Modus unterschiedlich schnell und unabhängig voneinander anzutreiben. D.h., das Steuerungsverfahren steuert die verschiedenen Modi insbesondere dadurch, dass die Antriebe, z.B. Elektromotoren oder Schrittmotoren unterschiedliche angesteuert werden, d.h. je nach Modus unterschiedlich schnell betrieben werden. Somit kann ggf. ein Einfluss einer Stellung/Bewegung von einem von dem Deflecting und dem Abklappmechanismus auf die Steuerung des jeweils anderen kompensiert werden.

Ferner betrifft ein weiterer Aspekt der vorliegenden Anmeldung ein Behandlungssystem mit einem vorstehend (insbesondere in Hinblick auf das Steuerungsverfahren) beschriebenen Endoskop und einer Rechnereinheit oder Steuerungsvorrichtung, welche zur Ausführung des vorstehenden Steuerverfahrens konfiguriert ist und welche insbesondere dazu eingerichtet ist, die Daten zur Position des Endoskopkopfs abzufragen und zu verarbeiten und / oder Antriebe zum Antreiben des Deflectings und des Abklappmechanismus über die Steuerdrähte zu steuern.

### Figurenbeschreibung

Nachfolgend wird die vorliegende Offenbarung anhand einer bevorzugten Ausführungsform beschrieben. Diese sind jedoch nur veranschaulichender Natur und sollen den Schutzumfang der vorliegenden Offenbarung nicht einschränken. Ferner werden bei der Beschreibung der verschiedenen Ausführungsformen für gleiche Bauteile dieselben Bezugszeichen verwendet.
Fig. 1 zeigt eine perspektivische Darstellung eines Endoskopkopfs eines offenbarungsgemäßen Endoskops nach einer bevorzugten Ausführungsform in abgewinkelter und gerader Stellung.
Fig. 2 zeigt eine Draufsicht auf den Endoskopkopf der bevorzugten Ausführungsform.
Fig. 3 zeigt ein Rückenelement des Endoskops nach einer ersten bevorzugten Ausführungsform und eine Modifikation des Rückenelements.
Fig. 4 zeigt das Rückenelement der ersten Ausführungsform in einer perspektivischen Darstellung.
Fig. 5 zeigt eine Querschnittsansicht des Rückenelements durch einen rillenartig gekrümmten Scharnierabschnitt gemäß einer Modifikation der ersten Ausführungsform.
Fig. 6 zeigt eine Draufsicht auf den Endoskopkopf zur Veranschaulichung einer Funktionsweise eines Kanaleinstellmechanismus der ersten Ausführungsform.
Fig. 7 und Fig. 8 zeigen das Rückenelement der bevorzugten Ausführungsform mit Kanaleinstellstrukturen des Kanaleinstellmechanismus gemäß mehreren Modifikationen.
Fig. 9 und Fig. 10 zeigen einen Querschnitt durch einen Abklappmechanismus nach einer weiteren Ausführungsform.
Fig. 11 zeigt Seitenansicht eines Abklappmechanismus nach einer alternativen Ausgestaltung der Offenbarung im gestreckten und abgeklappten Zustand.
Fig. 12 und Fig. 13 zeigen zwei bevorzugte Ausführungsformen der alternativen Ausgestaltung.
Fig. 14 stellt schematisch eine weitere bevorzugte Ausführungsform des Abklappmechanismus nach der vorliegenden Offenbarung dar.
Fig. 15 illustriert einen Aspekt einer Modifikation der Ausführungsform nach einer der Figuren 1 bis 8.
Fig. 16 A bis Fig. 16 C zeigen eine Betätigungsabfolge eines Betätigungsmechanismus zur Betätigung eines Endoskops nach einer der Ausführungsformen der Figuren 1 bis 15.
Fig. 17 zeigt schematisch eine Modifikation des Endoskops nach Fig. 1.
Fig. 18 zeigt ein Endoskop, insbesondere nach einer der vorstehenden Figuren, in verschiedenen einstellbaren Betriebszuständen.

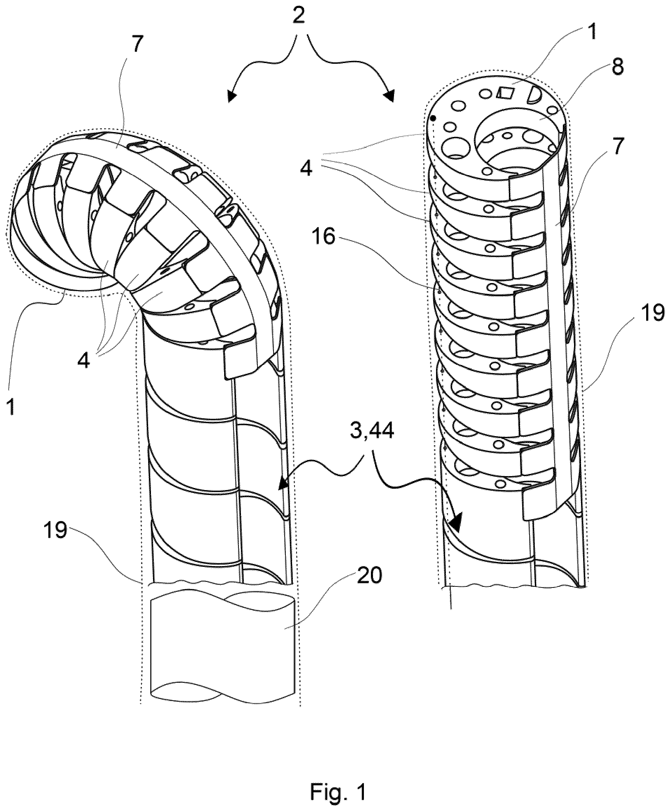

Fig. 1 zeigt einen Endoskopkopf 1 des offenbarungsgemäßen Endoskops nach einer bevorzugten Ausführungsform in perspektivische Darstellung. Der Endoskopkopf 1 hat einen Abklappmechanismus 2 (als Bestandteil des Endoskopkopfs 1 selbst oder als hierzu separate Einheit), der links in Fig. 1 abgewinkelt/abgeklappt dargestellt ist und rechts in Fig. 1 gestreckt dargestellt ist. Insbesondere ist das Endoskop ein Duodenoskop und der Abklappmechanismus 2 ist bevorzugt (aber nicht unbedingt erforderlich) am distalen Ende eines Deflecting-Abschnitts 3 des Endoskops angebracht. Der Deflecting-Abschnitt 3 ist ein flexibler, unabhängig vom Abklappmechanismus 2 manuell (kontrolliert) betätigbarer/abwinkelbarer Abschnitt, durch welchen der Endoskopkopf 1 vorzugsweise zur Navigation des Endoskops verschwenkbar (d.h. mit einem in Bezug auf den Abklappmechanismus 2 großen Biegeradius neigbar) ist. Der Deflecting-Abschnitt 3 - falls vorhanden - ist am distalen Ende eines vorzugsweise biegeflexiblen Endoskopschafts 20 fixiert. Der Endoskopschaft 20 ist in diesem Fall passiv abkrümmbar, d.h. nicht manuell aktuier- /kontrollierbar, um beispielsweise Biegungen eines Colons zu folgen. Ferner ist ein hier gestrichelt dargestellter Endoskopschlauch oder Overtube 19 bereitgestellt, welcher über den Abklappmechanismus 2 geschoben und insbesondere am Endoskopkopf 1 fixiert wird, um bei einer Verwendung des entsprechenden Endoskops Patientengewebe vor einer Verletzung (z.B. durch Einklemmen) durch die Mechanik des Abklappmechanismus 2 und/oder des Endoskops zu schützen. Der Endoskopschlauch oder Overtube 19 ist separat zu dem Abklappmechanismus 2 und diesen in Umfangsrichtung (vollständig) umgebend bereitgestellt.

Der Abklappmechanismus 2 kann gegenüber dem Deflecting-Abschnitt 3 separat abgeklappt werden (mit einem in Bezug auf den Deflecting-Abschnitt 3 kleinen Biegeradius des Abklappmechanismus 2), um so zumindest einen distalen Endabschnitt des Endoskopkopfs 1 (einschließlich Optik und Arbeitskanal-Ausgang) oder den gesamten Endoskopkopf 1 in Richtung radial zu kippen und so einen seitlichen (radialen) oder rückwärtsgewandten Blick einer am Endoskopkopf, an der Spitze des Abklappmechanismus 2 angeordneten Optik zu ermöglichen.

Der Abklappmechanismus 2 weist hierfür eine Anzahl von (wirbelkörperartigen) Segmenten 4 auf (mindestens ein Segment), die im Wesentlichen zylindrisch bis oval ausgebildet sind, mit demzufolge runden oder ovalen axialen Stirnseiten, welche den Stirnseiten benachbarter Segmente 4 in einer Endoskop-Längsrichtung zugewandt sind bzw. dem distal zum Abklappmechanismus angeordneten Endoskopkopf bzw. dem distalen Kopfabschnitt und dem proximal zum Abklappmechanismus angeordneten Deflecting / Endoskopschaft zugewandt sind. In einem Längsschnitt entlang der Endoskope-Längsrichtung ist jedes Segment 4 keilförmig (keilförmige Scheibe) ausgebildet, d. h., die Stirnseiten des jeweiligen Segments 4 sind in einem spitzen Winkel zueinander ausgerichtet. Die keilförmigen Segmente 4 sind in einer Umfangsrichtung zueinander derart angeordnet, dass Keilspitzenabschnitte 5 der Segmente 4 (d. h., Mantelflächen, welche in der Endoskop-Längsrichtung eine durch die Keilform bedingte minimale Ausdehnung/Länge aufweisen) jeweils an der gleichen Position in Umfangsrichtung/Umfangsposition liegen. Demzufolge liegen die Umfangsabschnitte der Segmente 4 mit maximaler Axiallänge (nachfolgend als Keilrückenabschnitt/-bereich 6 bezeichnet) ebenfalls in der gleichen Umfangsposition axial nahe beieinander, derart, dass im unbetätigten Zustand des Abklappmechanismus 2 (prograde Ausrichtung in Konstruktionslage) die keilförmigen Segmente 4 vergleichbar zu den Zähnen einer Zahnstange im Wesentlichen rechtwinklig zur Endoskopachse abstehen.

Wird der so aufgebaute Abklappmechanismus 2 betätigt (beispielsweise mittels eines im Keilspitzenabschnitt 5 platzierten nicht weiter dargestellten Zugseils), werden die Keilspitzenabschnitte 5 der Segmente 4 aufeinander (axial) zubewegt, sodass eine Länge des Endoskopkopfs 1 an der Seite der Keilspitzenabschnitte 5 verkürzt wird, wohingegen sich die Segmente im gegenüberliegenden Keilrückenabschnitt 6 gegeneinander (direkt oder indirekt) abstützen und dadurch das Abklappen erzeugt wird.

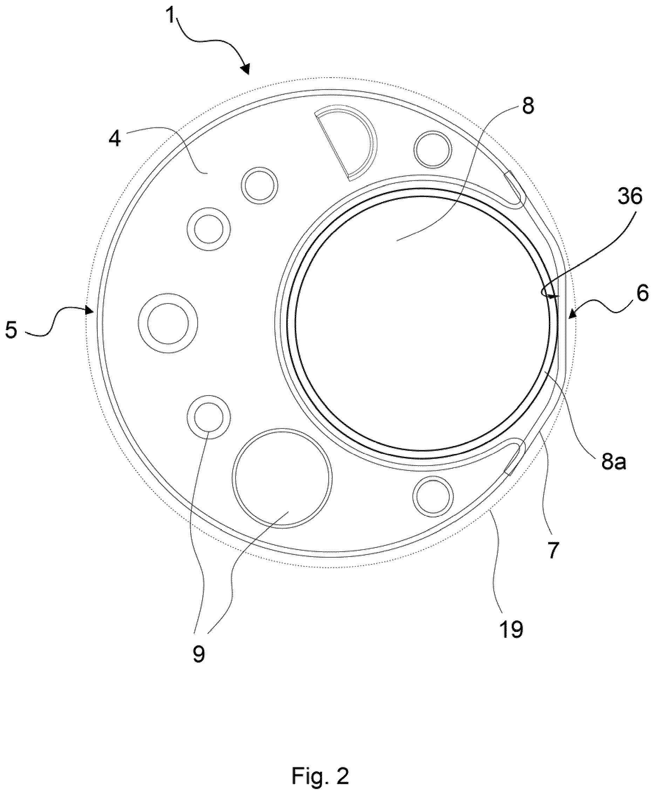

An dem dem jeweiligen Keilspitzenabschnitt 5 gegenüberliegenden Keilrückenabschnitt 6 der Segmente 4 (d. h., Mantelflächen, welche in der Endoskop-Längsrichtung eine durch die Keilform bedingte maximale axiale Ausdehnung aufweisen) sind die Segmente 4 durch ein Verbindungs-/Rückenelement 7 (Rückenpanzer/ Verbindungsplatte) miteinander verbunden. Das Rückenelement 7 ist eine separat zu den Segmenten 4 ausgebildete flexible, insbesondere elastische (dünnwandige) Verbindungsplatte vorzugsweise aus einem metallischen Werkstoff. Fig. 2 zeigt eine Draufsicht auf den Endoskopkopf 1 bzw. auf die Spitze des Abklappmechanismus 2. Das Endoskop bzw. jedes Segment 4 weist einen Arbeitskanalabschnitt 8 auf, derart, dass die Arbeitskanalabschnitte 8 der Segmente 4 in ihrer Gesamtheit einen Arbeitskanal definieren, der sich zu dem Keilrückenabschnitt 6 hin längsschlitzförmig öffnet. D. h., die Mantelfläche des/jedes Segments 4 hat an der Umfangsposition des Keilrückenabschnitts 6 einen schlitz- oder spaltförmigen Durchbruch in radialer Richtung, der sich axial über die gesamte Segmentlänge erstreckt. Das Rückenelement 7 erstreckt sich oder wölbt sich über den Durchbruch und ist beidseitig davon in Umfangsrichtung an dem Segment 4 an dessen Außenumfang befestigt, ohne dabei das Segment 4 vollumfänglich zu umschließen. Somit ist der von einem Segment 4 gebildete Arbeitskanalabschnitt 8 auf der Seite des Keilrückenabschnitts 6 durch das Rückenelement 7 begrenzt/zumindest teilweise, vorzugsweise vollständig verschlossen. Optional liegt innerhalb des Arbeitskanals, d.h. in Endoskop-Längsrichtung durch (sämtliche) Arbeitskanalabschnitte 8 verlaufend, ein Innenschlauch S, der den Arbeitskanal, insbesondere zwischen benachbarten Segmenten 4 und benachbarten weiteren Elementen, abdichtet.

In Draufsicht auf ein solches Segment 4 gemäß der Fig. 2 ist eine axiale Durchgangsöffnung/Durchgangsbohrung zu erkennen, welches den Arbeitskanalabschnitt 8 dieses Segments 4 bildet. Diese Durchgangsöffnung ist dezentral in Richtung hin zum Keilrückenabschnitt 6 versetzt angeordnet, derart, dass der Bohrungs-/Öffnungsdurchmesser über den Außenumfang des Segments 4 hinausreicht und somit die Mantelfläche des Segments aufbricht. Dadurch entsteht eine Art Sichelform für das Segment 4 mit einer im Wesentlichen hufeisenförmigen Ausbuchtung als Arbeitskanalabschnitt, wobei die Verbindungsplatte 7 lediglich einen Teilumfangsbereich des Segments 4 im Keilrückenabschnitt 6 außenseitig abdeckt und - in Umfangsrichtung gesehen - beidseits des spaltförmigen Aufbruchs an der Außenseite des Segments 4 fixiert ist. Dadurch bildet eine radiale Innenseite der Verbindungsplatte 7 einen Teil einer Innenwand des Arbeitskanalabschnitts 8.

Ferner sind in dem Endoskop/in jedem Segment 4 insbesondere in jenem Sichelbereich, der durch das radiale Versetzen der Durchgangsöffnung entstanden ist, Neben-/Versorgungskanäle 9 angeordnet, beispielsweise zur Führung von Bowdenzügen, Spülleitungen, und Ähnlichem, die einen gegenüber dem Arbeitskanal 8 kleineren Durchmesser aufweisen. Die Nebenkanäle 9 und der Arbeitskanal 8 sind in jedem Segment 4 derart angeordnet, dass diese parallel zueinander verlaufen.

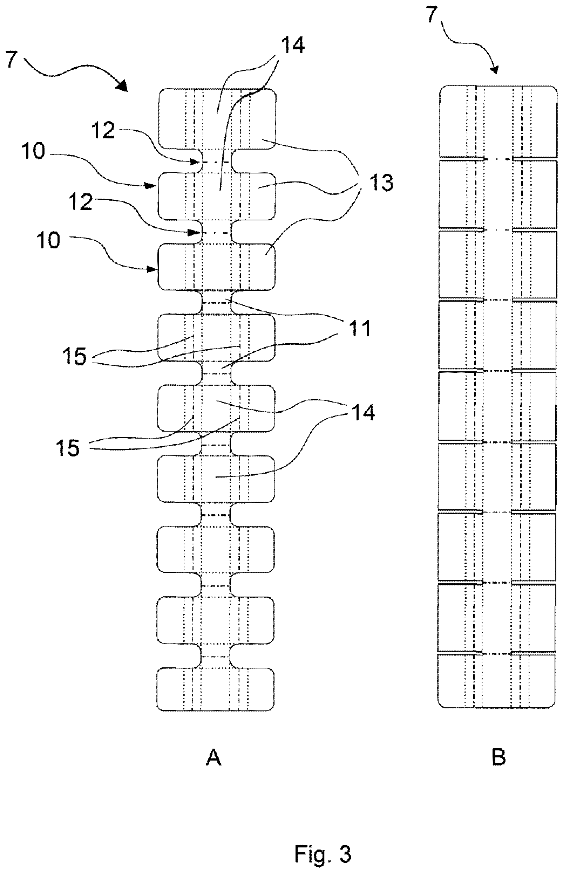

Das Rückenelement/die Verbindungsplatte 7, welches mit Verweis auf Fig. 3 (links, bezeichnet mit "A") und 4 genauer beschrieben wird, hat mehrere axial beabstandete Halteabschnitte 10, an welchen jeweils ein Segment 4 angeordnet ist oder wird, sowie axial zwischen den Halteabschnitten 10 angeordnete Scharnierabschnitte 11, welche entsprechend zwischen zwei Segmenten 4 angeordnet sind und derart biegbar/flexibel ausgebildet sind, um als Scharnier für das Abklappen des Endoskopkopfs 1 durch den Abklappmechanismus 2 zu dienen. Dies ist in Fig. 3 durch Scharnierachsen 12 veranschaulicht. Vorzugsweise ist das Rückenelement/die Verbindungsplatte 7 im Bereich der Scharnierabschnitte 11 in Umfangsrichtung gesehen verjüngt/eingeschnürt. Weiter vorzugsweise ist das Rückenelement/die Verbindungsplatte 7 zumindest im Bereich der Scharnierabschnitte 11 aus einem biegeelastischen Material, zum Beispiel Federstahl, gefertigt.

Ferner weisen die/jeder der Halteabschnitte 10 insbesondere zwei flügelartig voneinander in Umfangsrichtung abstehende Befestigungsbereiche 13 auf, die sich (laschenartig) seitwärts/in Umfangsrichtung gegenläufig erstrecken und zur Befestigung des Rückenelements 7 an den Segmenten 4 (beidseits des jeweiligen Längsschlitzes) ausgebildet sind. Zwischen zwei Befestigungsbereichen 13 ist ein Deckbereich 14 definiert, der über dem Durchbruch/Längsschlitz des betreffenden Segments 4 angeordnet/anordenbar ist, welcher durch den Arbeitskanal 8 in der Mantelfläche des entsprechenden Segments 4 gebildet wird, wie dies vorstehend beschrieben wurde. Die Deckbereiche 14 und die Scharnierabschnitte 11 bilden gemeinsam eine kontinuierliche (durchgehend glatte) radial innere Abgleitfläche 36 des Rückenelements 7, zum Führen und zum Abgleiten von medizinischen Instrumenten, die in den Arbeitskanal 8 eingeschoben werden, insbesondere, wenn der Abklappmechanismus 2 abgeklappt ist (siehe Fig. 1, linke Darstellung). Darüber hinaus ist einer der Halteabschnitte 10 an einem ersten (proximalen) Ende des Rückenelements 7 zur Befestigung an dem Endoskopschaft 20, insbesondere an dem (optionalen) Deflecting-Abschnitt 3 - falls vorhanden - , ausgebildet und kann hierfür beispielsweise in Endoskop-Längs- und/oder -Umfangsrichtung relativ zu den übrigen (Segment-) Halteabschnitten 10 verbreitert sein.

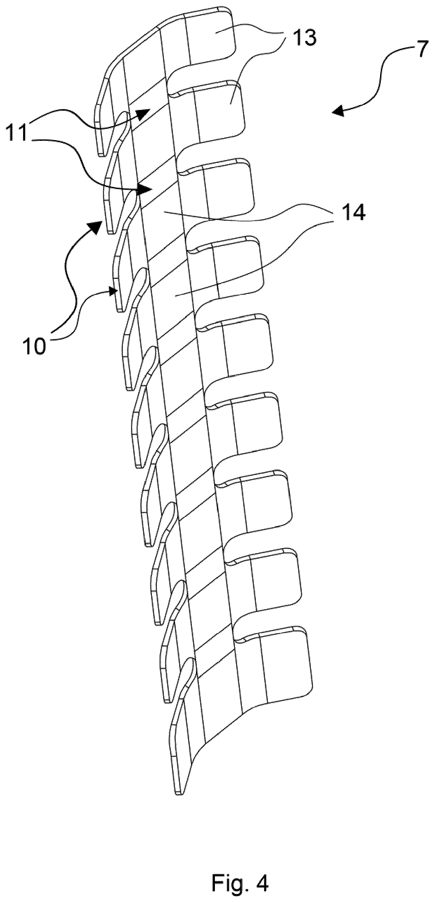

Ferner wird/ist das Rückenelement 7 bevorzugt aus einer einzigen Platte oder einem Blech gefertigt, beispielsweise gestanzt, wie in Fig. 3 zu sehen ist, und werden anschließend die Halteabschnitte 10 gebogen, um eine Wölbung des Rückenelements 7 zu erzielen, welche im Wesentlichen einem Außenumfang der Segmente 4 entspricht bzw. polygonal annähert. Insbesondere werden die Befestigungsbereiche 13 bezüglich den Deckbereichen 14 um eine Biegeachse 15 gebogen, um die den Segmenten 4 angepasste rinnenartige Wölbung zu erzeugen. Das fertige Rückenelement 7 ist in Fig. 4 in perspektivische Darstellung zu sehen.

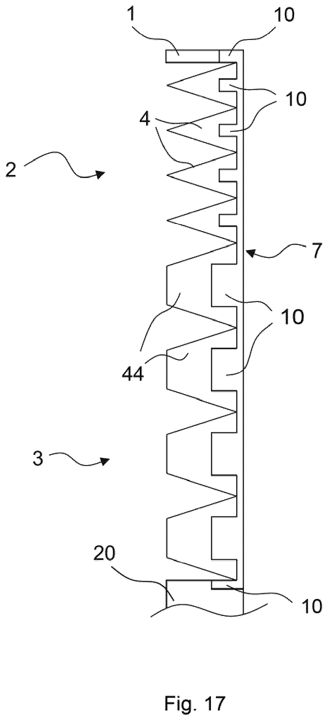

Bevorzugt kann das Rückenelement 7 sich entlang sowohl des Abklappmechanismus 2 als auch des Deflectings 3 erstrecken und, wie in Fig. 17 genauer dargestellt, über entsprechende Halteabschnitte 10 den Endoskopkopf bzw. den distalen Kopfabschnitt 1, die Segmente 4 des Abklappmechanismus 2, die Wirbelkörper 44 des Deflectings 3 und den (passiven) Endoskopschaft 20 miteinander verbinden. D.h. das Rückenelement 7 kann einen Deflecting-Abschnitt (Deflecting-Verbindungselement/ -Rückenelement) aufweisen, welcher die Wirbelkörper 44 des Deflectings 3 verbindet. Ferner können Wirbelkörper 44 des Deflectings 3, wie in Fig. 1 dargestellt, an einer Umfangsposition bezüglich des Endoskopschafts, welche der Umfangsposition der Keilrückenabschnitte 6 des Abklappmechanismus 2 und des Rückenelements 7 entspricht, jeweils einen schlitz- oder spaltförmigen Durchbruch in radialer Richtung haben, der sich über die gesamte Länge des jeweiligen Wirbelkörpers 44 erstreckt und der vorzugsweise einen sich durch die Wirbelkörper 44 erstreckenden Arbeitskanal mit einer Außenseite der Wirbelkörper 44 verbindet. Der Deflecting-Abschnitt des Rückenelements 7 kann die schlitz- oder spaltförmigen Durchbrüche in den Wirbelkörpern 44 des Deflectings überdecken.

Rechts in Fig. 3 (Bezeichnet mit "B") ist eine Modifikation des Rückenelements/ der Verbindungsplatte 7 dargestellt, wobei aus Übersichtlichkeitsgründen bezüglich der Bezugszeichen auf die Darstellung "A" verwiesen wird. Gemäß dieser Modifikation sind die laschenförmigen Befestigungsbereiche 13 breit im Vergleich zu den Zwischenräumen zwischen den Befestigungsbereichen 13 bzw. zu Scharnierabschnitten 11. Insbesondere sind die Zwischenräume schmale Schlitze bzw. Einschnitte. Die Befestigungsbereiche 13 stehen nach dieser Modifikation in der Endoskop-Längsrichtung über die Segmente 4 hinaus und überlappen einander bei einem Abkrümmen des Abklappmechanismus 2.

Die Funktion eines solchen Abklappmechanismus 4 lässt sich wie folgt zusammenfassen:
Zunächst ist, wie vorstehend ausgeführt wurde, der Endoskopkopf 1 oder der distale Endoskopkopfabschnitt über wenigstens ein oder mehrere Segmente 4 mit dem Endoskopschaft 20 oder mit dem optionalen Deflecting 3 am distalen Ende des Endoskopschafts 20 verbunden. Das Rückenelement/die Verbindungplatte 7 ist hierfür an ihren axial beabstandeten Halteabschnitten 10 am Endoskopkopf/distalen Kopfabschnitt, an dem wenigstens einen Segment 4 und am Endoskopschaft 20 oder Deflecting 3 fixiert und hält somit diese wenigstens drei Elemente axial aneinander. Gleichzeitig überdeckt das Rückenelement 7 den Längsspalt am zumindest einen Segment 4 und bildet so die Abgleitfäche zum Innern des von diesem Segment 4 teilweise umgebenen Arbeitskanalabschnitt 8.

Sobald ein Abklappmechanismus-spezifisches Betätigungselement (Zugseil) manuell aktuiert wird, klappt der Endoskopkopf/distale Kopfabschnitt an dem Filmscharnier, welches vom Rückenelement 7 zwischen dem Kopf und dem unmittelbar proximal dazu angeordneten Segment 4 ausgebildet ist, sowie an dem Filmscharnier, welches vom Rückenelement 7 zwischen dem Segment 4 und dem unmittelbar proximal dazu angeordneten Deflecting/Endoskopschaft 20 ausgebildet ist, entsprechend der Keilform des zumindest einen Segments 4 ab, bis die jeweiligen Stirnseiten der Segmente 4 mit einer nebenliegenden Fläche oder Stirnseite in Anlage kommen, wodurch der von den drei genannten Elementen gemeinsam gebildete Arbeitskanal 8 gekrümmt wird.

Wird in dieser Abklappposition ein minimalinvasives Instrument ausgehend vom proximalen Ende des Endoskopschafts 20 durch den Arbeitskanal 8 in Richtung distal vorgeschoben, stößt die Instrumentenspitze im Bereich der drei vorstehend genannten abgeklappten Elemente zwangsläufig an die durchgehend glatte (stufenlose) Innenseite des Rückenelements 7 ohne dass diese am zumindest einen Segment 4 vorschubblockierend anschlägt. Dadurch kann eine Vorschubbewegung auch bei extrem kleinen Abklappwinkel im Bereich des Abklappmechanismus problemlos ausgeführt werden. Entscheidend hierfür ist der Längsschlitz-artige Ausbruch des zumindest einen Segments 4, sowie dessen Überdeckung durch das Rückenelement 7, welches gleichzeitig das oder die Abklappscharniere zwischen den Elementen des Abklappmechanismus ausbildet.

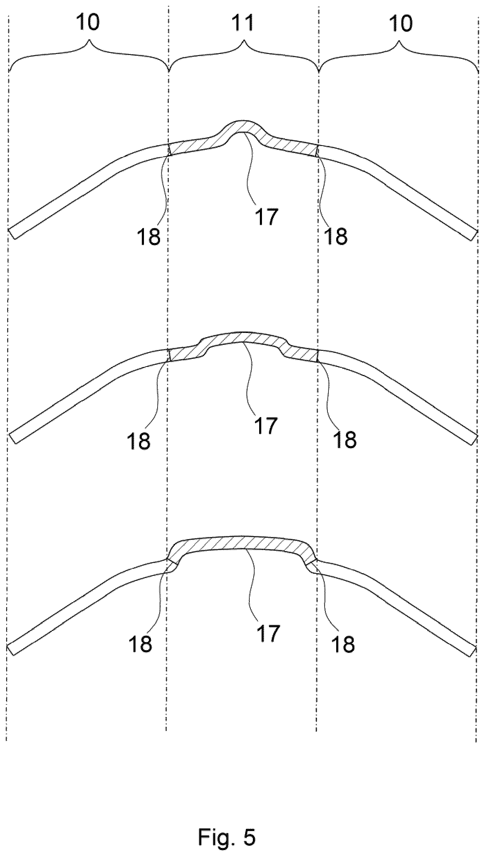

Fig. 5 zeigt die mehrere optionale Modifikationen der Verbindungsplatte/des Rückenelements 7 (Verbindungsplatte) im Querschnitt durch den Scharnierabschnitt 11. Der Scharnierabschnitt 11 ist jeweils entlang der Endoskop-/Segment-Umfangsrichtung nach radial außen gewölbt und formt somit eine (radial innenliegende) Rinne. Die Rinne hat einen insbesondere variablen Rinnenradius oder Krümmungsradius. Das Rückenelement 7 kann entlang der Endoskop-Längsachse gleiche oder unterschiedlich modifizierte Scharnierabschnitte aufweisen. In letzterem Fall ist Fig. 5 derart zu verstehen, dass darin mehrere unterschiedliche Scharnierabschnitte 11 eines einzelnen Rückenelements 7 dargestellt sind.

In der ersten Modifikation (oben in Fig. 5) ist der Rinnen-/Krümmungsradius im Bereich einer Rinnenmitte 17 (Zentrum der Rinne in Umfangsrichtung betrachtet) eng bzw. klein (die Wölbung groß) und vergrößert sich hin zu einem Rinnenrand 18. Der Rinnenradius ist also im Bereich des Rinnenrands 18 groß (nur relativ geringfügig gewölbt/mit kleiner Wölbung versehen). Bei dieser Modifikation ist eine Rückstellkraft in eine gestreckte Stellung des Abklappmechanismus, die der Scharnierabschnitt 11 im stark (insbesondere maximal) gebogenem / abgeknickten Zustand des Abklappmechanismus 2 ausübt, klein, und ist die Rückstellkraft in einem geringfügig (insbesondere minimal) abgeklappten Zustand des Abklappmechanismus 2 groß (im Verhältnis zu der kleinen Rückstellkraft im stark gebogenen Zustand).

In der zweiten Modifikation (mittig in Fig. 5) ist der Rinnen-/Krümmungsradius im Bereich der Rinnenmitte 17 sowie im Bereich des Rinnenrands 18 groß und ist in einem Zwischenbereich zwischen der Rinnenmitte 17 und dem Rinnenrand 18 eng bzw. klein. Bei dieser Modifikation ist die Rückstellkraft in die gestreckte Stellung, die der Scharnierabschnitt 11 im stark (insbesondere maximal) und im geringfügig (insbesondere minimal) gebogenem / abgeknickten / abgeklappten Zustand des Abklappmechanismus 2 ausübt, klein, und ist die Rückstellkraft in einem mittleren/zwischenliegenden abgeklappten Zustand des Abklappmechanismus 2 groß (im Verhältnis zu der kleinen Rückstellkraft).

In der dritten Modifikation (unten in Fig. 5) ist der Rinnen-/Krümmungsradius im Bereich der Rinnenmitte 17 groß und ist im Bereich des Rinnenrands 18 eng bzw. klein. Bei dieser Modifikation ist die Rückstellkraft in die gestreckte Stellung, die der Scharnierabschnitt 11 im stark (insbesondere maximal) gebogenem / abgeknickten / abgeklappten Zustand des Abklappmechanismus 2 ausübt, groß, und ist die Rückstellkraft im geringfügig (insbesondere minimal) gebogenen / abgeknickten / abgeklappten Zustand des Abklappmechanismus 2 klein (im Verhältnis zu der großen Rückstellkraft).

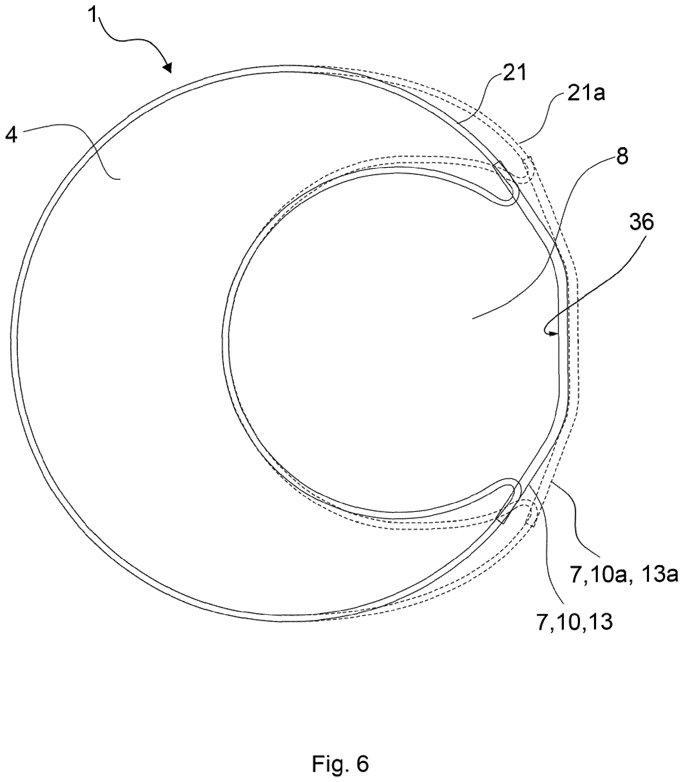

Fig. 6 zeigt eine Draufsicht auf den Endoskopkopf 1 zur Veranschaulichung einer Funktionsweise eines optionalen Kanaleinstellmechanismus der bevorzugten Ausführungsform. Der Endoskopkopf 21 ist gegenüber der Darstellung in Fig. 2 im Sinne einer besseren Übersichtlichkeit vereinfacht dargestellt und zeigt nur die für den Kanaleinstellmechanismus relevanten Elemente. Die Segmente 4 haben, wie vorstehend beschrieben, einen sichelförmigen Querschnitt, innerhalb dessen der Arbeitskanal 8 gebildet ist. Die Enden der Sichel bilden Segmentflügel 21, die sich aufeinander zu krümmen und Seitenwände des Arbeitskanals 8 bilden, d.h., die den Arbeitskanal 8 zumindest teilweise umgreifen. Radial außen an den Segmentflügeln 21 ist die Verbindungsplatte 7 jeweils über ihren Halteabschnitt 10, genauer, den Befestigungsbereich 13 desselben, befestigt. Die Segmentflügel 21 können nach radial außen oder innen elastisch und/oder plastisch aufgebogen/aufgeweitet bzw. zusammengebogen/verengt werden, um einen Durchmesser des Arbeitskanals 8 zu vergrößern bzw. zu verkleinern. In Fig. 6 sind die aufgeweiteten Segmentflügel 21a gestrichelt dargestellt.

Das Rückenelement 7, welches sich über eine Lücke zwischen den beiden Segmentflügeln 21 wölbt (diese überbrückt), um eine Seitenwand des Arbeitskanals 8 zu bilden, kann ebenfalls nach radial außen oder innen elastisch und/oder plastisch aufgebogen/aufgeweitet bzw. zugebogen/verengt werden. Insbesondere können die Halteabschnitte 10 entsprechend gebogen werden. In Fig. 6 sind die aufgeweiteten Halteabschnitte 10a bzw. die aufgeweiteten Befestigungsbereiche 13a gestrichelt dargestellt. Somit kann die Wölbung des Rückenelements 7 an die Segmentflügel 21 bzw. an den Durchmesser des Arbeitskanals 8 angepasst werden, um die Lücke dazwischen auch bei einem verengten oder aufgeweiteten Arbeitskanal 8 sicher überbrücken zu können und vorzugsweise dem Durchmesser des Arbeitskanals 8 zu entsprechen.

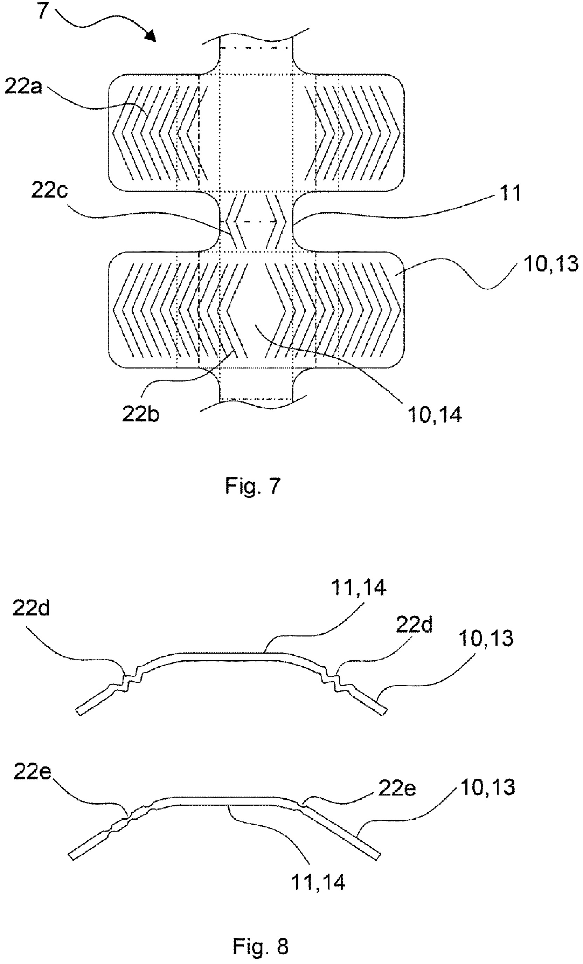

Fig. 7 und Fig. 8 zeigen Modifikationen einer Kanaleinstellstruktur 22a bis 22d des Rückenelements 7 (Verbindungsplatte), welche zusammen mit den aufbiegbaren Segmentflügeln 21 den Kanaleinstellmechanismus bilden oder ein Teil desselben sind. In Fig. 7 ist ein Abschnitt des Rückenelements 7 dargestellt, der mit einem Raster von, beispielsweise V-förmigen, Schlitzen 22a, 22b, 22c versehen ist, welches als die Kanaleinstellstruktur dient. Die V-förmigen Schlitze 22a, 22b, 22c sind derart im Rückenelement 7 angeordnet, dass eine Spitze der V-Form in Umfangsrichtung des Segments 4, wenn das Rückenelement 7 daran befestigt ist, nach außen weist (weg von den Deckbereichen 14). Alternativ kann die Spitze der V-Form auch nach innen, d. h. hin zu den Deckbereichen 14 in Umfangsrichtung des Segments 4, weisen. Alternativ können die Schlitze 22a, 22b, 22c eine beliebige Form annehmen, welche ein elastisches und/oder plastisches Biegen des Rückenelements 7 nach radial innen und/oder außen bezüglich der Endoskopachse ermöglichen/erleichtern.

Nach einer ersten Modifikation sind (ausschließlich) an den Befestigungsbereichen 13 des Rückenelements 7 Kanaleinstellstrukturen 22a vorgesehen, in diesem Beispiel die V-förmigen Schlitze, welche beispielhaft an dem in Fig. 7 oberen Halteabschnitt 10 dargestellt sind. Alternativ oder zusätzlich sind nach einer zweiten Modifikation an den Deckbereichen 14 Kanaleinstellstrukturen 22b (zum Beispiel die V-förmigen Schlitze) vorgesehen, welche beispielhaft an dem in Fig. 7 unteren Halteabschnitt 10 dargestellt sind. Alternativ oder zusätzlich können nach einer dritten Modifikation auch an den Scharnierabschnitten 11, wie in Fig. 7 beispielhaft mittig dargestellt, Kanaleinstellstrukturen 22c bereitgestellt sein.

Alternativ oder zusätzlich, wie in Fig. 8 gezeigt, kann ein Profilquerschnitt des Rückenelements 7, insbesondere der Halteabschnitte 10, vorzugsweise der Befestigungsbereiche 13, variiert werden, um die Kanaleinstellstruktur 22d, 22e bereitzustellen. Die Variation des Profilquerschnitts erstreckt sich vorzugsweise über den gesamten Halteabschnitt10 oder den gesamten Befestigungsbereich 13 und definiert entlang dieser Erstreckung eine Biegeachse für das Aufweiten/Verengen der Wölbung des Halteabschnitts 10. Beispielsweise kann der Halteabschnitt 10, insbesondere an einem Übergang des Befestigungsbereichs 13 zu dem Deckbereich 14 oder am Befestigungsbereiches 13 nahe des Deckbereichs 14, wie in Fig. 8 oben dargestellt, gewellt sein, um eine Kanaleinstellstruktur 22d nach einer vierten Modifikation bereitzustellen. Alternativ oder zusätzlich kann eine Materialdicke (Plattendicke/Blechdicke) des Halteabschnitts 10, insbesondere an dem Übergang des Befestigungsbereichs zu dem Deckbereich 14 oder am Befestigungsbereich nahe des Deckbereichs 14, wie in Fig. 8 unten dargestellt, an einer oder mehreren Stellen (falzartig) verringert sein, um eine Kanaleinstellstruktur 22e nach einer fünften Modifikation bereitzustellen. Eine Stelle mit verringerter Materialdicke wird durch eine in Endoskop-Längsrichtung verlaufende Rille gebildet. Vorzugsweise verlaufen mehrere solche Rillen parallel zueinander (siehe Fig. 8 unten links). Es reicht jedoch auch eine einzelne Rille 22e an einem der Halteabschnitte 10 bzw. Befestigungsbereiche 13 aus, um eine Kanaleinstellstruktur bereitzustellen (siehe Fig. 8 unten rechts). Die Rille oder Rillen 22e sind hier beispielhaft an einer radialen Innen- und Außenfläche derart angeordnet, dass jeweils eine radial äußere und eine radial innere Rille einander gegenüberliegen. Alternativ können diese auch versetzt zueinander angeordnet sein. Weiter alternativ kann/können die Rille/n nur radial innen oder nur radial außen vorgesehen sein.

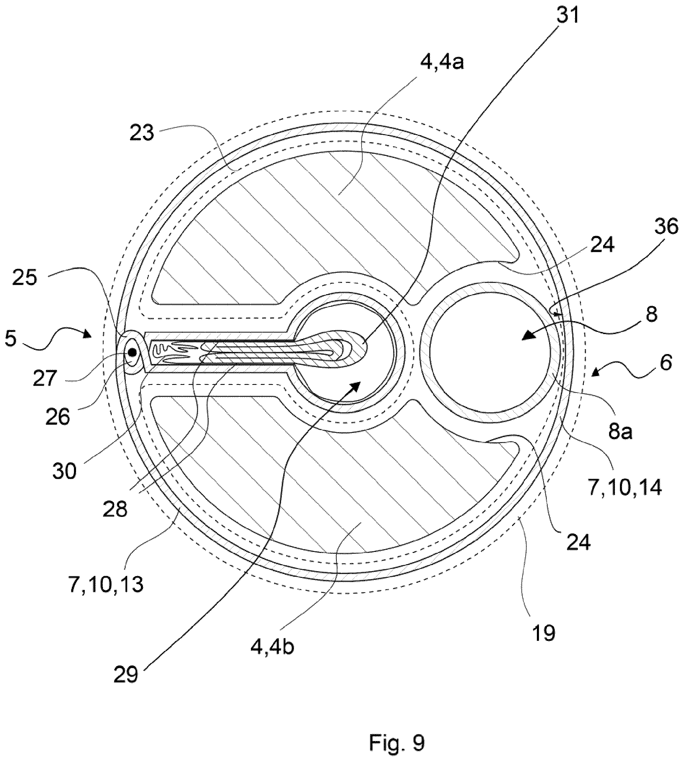

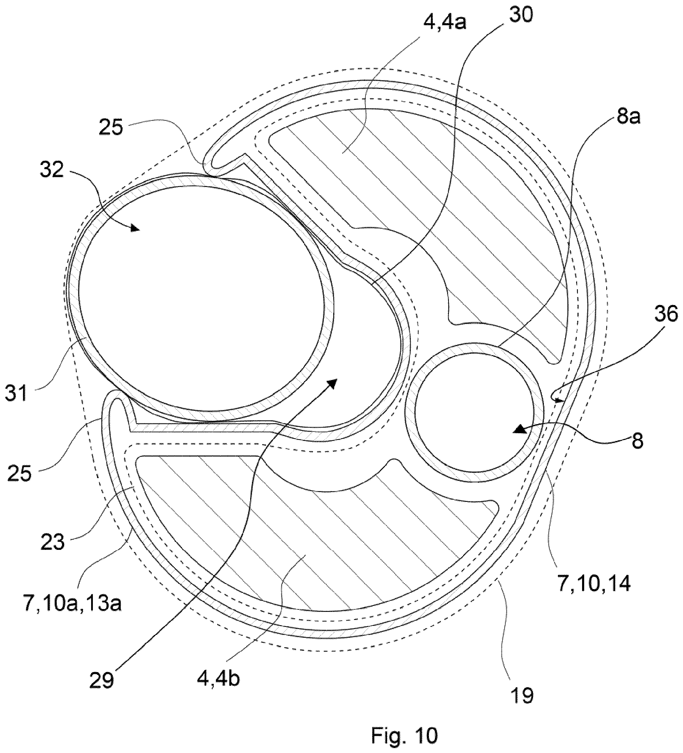

Fig. 9 und Fig. 10 zeigen einen Querschnitt durch einen Abklappmechanismus nach einer weiteren Ausführungsform. Nach dieser Ausführungsform ist das Rückenelement 7 (Verbindungsplatte) im Bereich der Halteabschnitte 10 (d.h., wo die Segmente 4 an dem Rückenelement 7 angebracht sind) als elastisches Hohlprofil ausgebildet, welches die Segmente 4 umgreift. Jedes Segment ist durch zwei vorzugsweise separate Teilsegmente 4a, 4b gebildet, welche jeweils spiegelsymmetrisch ausgebildet und an den Befestigungsbereichen 13 eines entsprechenden Haltebereichs 10 befestigt (z.B. geklebt, geschweißt, etc.) sind. Die Teilsegmente sind durch einen Spalt voneinander (vorzugsweise vollständig) getrennt. Optional ist zumindest entlang des (gesamten) Abklappmechanismus 2 zwischen den Segmenten 4 und den dem Rückenelement 7 eine Hüllschicht 23 bereitgestellt. Die Teilsegmente 4a, 4b bilden im Keilrückenabschnitt 6 radiale Rücksprünge 24 aus, welche einander derart gegenüberliegend angeordnet sind, um den sich am Keilrückenabschnitt 6 öffnenden Arbeitskanal 8 auszubilden. Im Arbeitskanal verläuft der Innenschlauch 8a.

Die Befestigungsbereiche 13 des Rückenelements greifen um die jeweiligen Teilsegmente 4a, 4b und bilden an der dem Arbeitskanal 8 radial gegenüberliegenden Seite bzw. am Keilspitzenabschnitt 5 jeweils eine Öse 25 aus. Die Ösen 25 der Befestigungsbereiche 13 liegen in einem in Fig. 9 dargestellten Auslieferungszustand in Endoskop-Längsrichtung betrachtet derart übereinander (überlagern einander), dass die Ösen 25 der Befestigungsbereiche 13 jedes Haltebereichs 10 zwischen sich einen Riegelkanal 26 ausbilden. Die Befestigungsbereiche 13 sind als Federelemente ausgebildet, die im Auslieferungszustand nach radial innen zusammengedrückt und somit vorgespannt sind. Im Auslieferungszustand ist ein Riegeldraht 27 oder ein anderes flexibles, schnurförmiges Element durch die einzelnen Ösen 25, d.h. durch den Riegelkanal 26, geführt und hält somit die einander jeweils gegenüberliegenden Befestigungsbereiche 13 am Keilspitzenabschnitt 5 gegen die Vorspannung der Befestigungsbereiche 13 zusammen.

Optional bilden die Befestigungsbereiche 13 Innenschenkel 28 aus, die sich im Wesentlichen parallel zueinander nach radial innen durch den Spalt erstrecken und innerhalb des Segments 4 bzw. zentral zwischen den Teilsegmenten 4a, 4b miteinander verbunden sind, um einen Aufnahmeraum 29 auszubilden. Die einander zugewandten Seiten der Innenschenkel 28 und der Aufnahmeraum 29 sind mit einer in Endoskop-Längsrichtung durch den (gesamten) Abklappmechanismus 2 verlaufenden, folienartigen (ggf. eingeklebten) Auskleidung 30 versehen, die am Keilspitzenabschnitt 5 zwischen den Innenschenkeln 28 gefaltet ist. Ferner ist an den einander zugewandten Seiten der Innenschenkel 28 bzw. an einem entsprechenden Bereich der Auskleidung 30 ein zusätzlicher, Endoskop-längsverlaufender Zusatzschlauch 31 eingelegt (an den Innenschenkeln 28 eingeklebt) und zwischen den Innenschenkeln 28 gefaltet und in dem Aufnahmeraum 29 aufgenommen. Wahlweise können der Zusatzschlauch 31 oder die Auskleidung 30 weggelassen werden.

Wird im Verlauf einer Behandlung ein zusätzlicher Arbeitskanal benötigt, beispielsweise um ein weitere Instrument oder einen Spülschlauch einführen zu können, wird der Riegeldraht 27 in Richtung proximal aus den Ösen 25 herausgezogen, öffnen/spreizen sich die Befestigungsbereiche 13a durch deren Vorspannung (Pacmanähnlich) nach radial außen, derart, dass die Innenschenkel 28 zwischen sich einen Winkel einschließen, wie in Fig. 10 dargestellt. Der keilspitzenseitige, gefaltete Bereich der Auskleidung 30 wird zwischen den Innenschenkeln 28 entfaltet oder gestreckt. Alternativ oder zusätzlich entfaltet sich der Zusatzschlauch 31 durch das Öffnen der Innenschenkel 28 und/oder dessen Eigenelastizität und bildet einen Zusatzarbeitskanal 32 aus. Der Overtube 19 ist dehnbar, um das Öffnen des Zusatzarbeitskanals 32 zu ermöglichen.

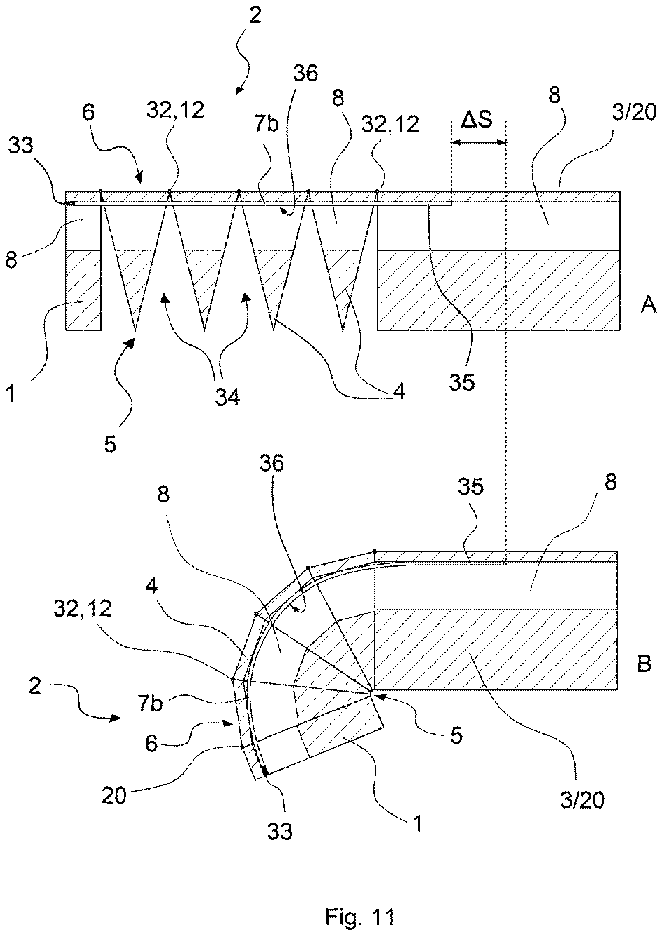

Fig. 11 zeigt eine Prinzipskizze eines Abklappmechanismus in einer Seitenansicht nach einer alternativen Ausgestaltung der Offenbarung im gestreckten (oben in Fig. 11 bzw. "A") und abgeklappten Zustand (unten in Fig. 11 bzw. "B"). Die in Endoskop-Längsrichtung zylindrische und in der Seitenansicht keilförmige Ausgestaltung der Segmente 4 sowie deren Anordnung zwischen einem Deflecting-Abschnitt 3 (oder Endoskopschaft 20) und einem Endoskopkopf (oder einem distalen Kopfabschnitt) 1 entsprechen im Wesentlichen der Ausgestaltung der Offenbarung nach Figuren 1 bis 10, ebenso wie die Betätigung über ein entsprechendes Betätigungselement, welches in Fig. 11 jedoch nicht dargestellt ist. Weitere Elemente wie z.B. ein Overtube o.Ä., die beiden Ausgestaltungen zu eigen sind, sind aus Übersichtsgründen nicht dargestellt.

Im Unterschied dazu sind die Segmente 4 jedoch am Keilrückenabschnitt 6 ungeschlitzt / undurchbrochen und sind am Keilrückenabschnitt 6 aneinander über Segmentgelenke 32 schwenkbar / abklappbar miteinander verbunden. Das heißt, die Durchgangsöffnungen, die zusammen den Arbeitskanal 8 ausbilden, sind derart in den jeweiligen Segmenten 4 eingebracht, dass die Wand jeder Durchgangsöffnung umlaufend geschlossen ist. D.h., anders als in der Ausführung nach Figuren 1 bis 10 weisen die Durchgangsöffnungen keinen seitlichen Durchbruch oder Schlitz auf. Anstelle eines als Verbindungsplatte ausgebildeten Rückenelements ist eine flexible Abgleitplatte oder eine flexible Abgleitbandeinrichtung 7b bereitgestellt, die innerhalb des Arbeitskanals 8 an einer dem Keilrückenabschnitt 6 zugewandten Position eingelegt ist. An einem distalen Bereich des Abklappmechanismus 2, vorzugsweise am Endoskopkopf / am distalen Kopfabschnitt 1, ist die Abgleitplatte/ Abgleitbandeinrichtung 7b an einer Fixierstelle 33, z.B. einer Klebe-, Schweiß- oder Lötstelle 33, in/an dem Arbeitskanal 8 (d.h. in/an einer Durchgangsöffnung) lagefestgelegt fixiert. Proximal von der Fixierstelle 33 erstreckt sich die Abgleitplatte/ Abgleitbandeinrichtung 7b lose / längsverschiebbar durch die Durchgangsöffnungen der Segmente 4 bzw. durch den Arbeitskanal 8 und überbrückt Lücken 34, die, wie in Fig. 11 oben (A) erkennbar, im gestreckten Zustand des Abklappmechanismus 2 zwischen den jeweils benachbarten Segmenten 4 ausgebildet sind. Ein proximales Ende 35 der Abgleitplatte/ Abgleitbandeinrichtung 7b liegt in dem Endoskopschaft 20 oder Deflecting-Abschnitt 3 (jeweils an dessen distalem Endbereich). Optional erstreckt sich die Abgleitplatte/ Abgleitbandeinrichtung 7b durch den Deflecting-Abschnitt 3 und das proximale Ende 35 liegt im dem Deflecting-Abschnitt 3 proximal vorgelagerten Endoskopfschaft 20.

Wird der Abklappmechanismus 2 abgeklappt/abgekrümmt, wie in Fig. 11 unten (B) dargestellt, kommen die Segmente 4 mit den jeweils benachbarten Segmenten 4 in Anlage und die Lücken 34 schließen sich (teilweise oder vollständig). Die Abgleitplatte/ Abgleitbandeinrichtung 7b wird distal an der Fixierstelle 33 lagefest gehalten, wodurch die Abgleitplatte/ Abgleitbandeinrichtung 7b gebogen wird und der Krümmung des Abklappmechanismus 2 im Wesentlichen folgt (in die Krümmung hineingedrückt wird). Da die Abgleitplatte/ Abgleitbandeinrichtung 7b lose in den proximal vom Endoskopkopf / distalen Kopfabschnitt 1 angeordneten Segmenten und im Deflecting 3 / Endoskopschaft 20 liegt, sowie durch eine Steifigkeit bzw. Elastizität der Abgleitplatte/ Abgleitbandeinrichtung 7b liegt diese im abgeklappten Zustand nicht vollständig an den Segmenten 4 an, sondern tangiert die Segmente 4 höchstens. Dadurch bildet die Abgleitplatte/ Abgleitbandeinrichtung 7b die am Übergang zwischen benachbarten Segmenten 4 entstehenden Winkel und/oder Lücken 34 nicht ab, sondern bildet eine (gleichmäßig) gewölbte Abgleitfläche 36, an welcher ein durch den Arbeitskanal 8 geschobenes Instrument gleichmäßig abgleiten kann.

Ferner verkürzt sich der Abklappmechanismus 2 durch das Abklappen im Bereich der Abgleitplatte/ Abgleitbandeinrichtung 7b, da die Lücken 34 teilweise oder vollständig geschlossen werden. Somit wird das proximale Ende 35 der Abgleitplatte/ Abgleitbandeinrichtung 7b beim Abklappen des Abklappmechanismus 2 um eine Einschubdistanz ΔS in den Endoskopschaft 20 / in das Deflecting 3 eingeschoben. Die Einschubdistanz ΔS entspricht im Wesentlichen der summierten Breite der Lücken 34 zwischen den Segmenten 4 im Bereich der dem Keilrückenabschnitt 6 zugewandten Arbeitskanalwände im gestreckten Zustand des Abklappmechanismus 2 sowie einer Verkürzung des durch die Segmente 4 verlaufenden Abschnitts der Abgleitplatte/ Abgleitbandeinrichtung 7b aufgrund der Wölbung der Abgleitplatte/ Abgleitbandeinrichtung 7b im Verhältnis zu den (in Endoskop-Längsrichtung) gerade verlaufenden Arbeitskanalwänden der Segmente 4.

Wird der Abklappmechanismus 2 wieder gestreckt, streckt sich die Abgleitplatte/ Abgleitbandeinrichtung 7b insbesondere aufgrund von deren Elastizität / Rückstellkraft.

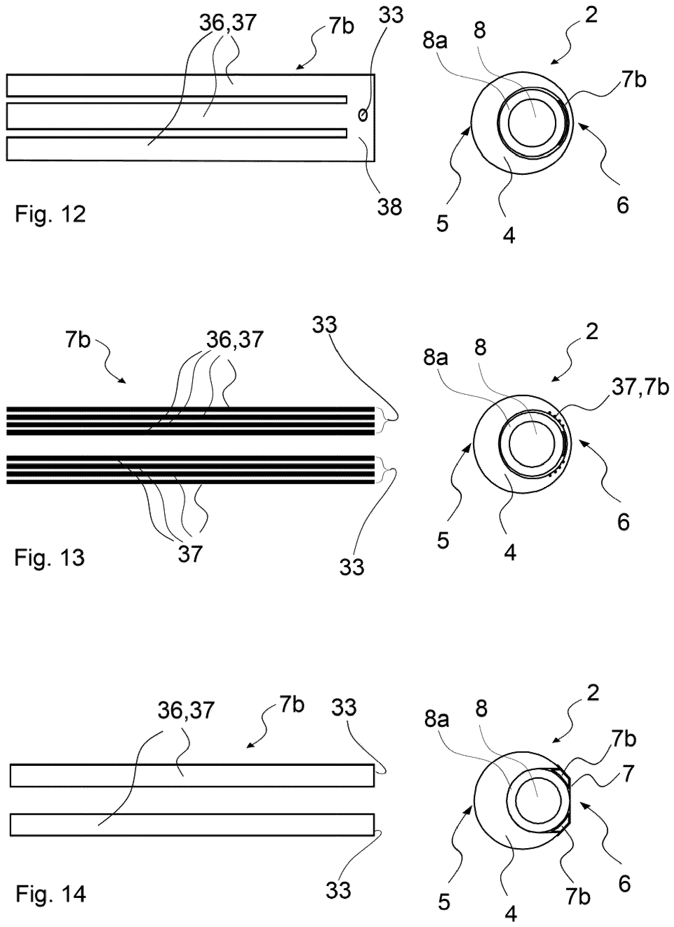

Fig. 12 zeigt eine bevorzugte Ausführungsform der Abgleitbandeinrichtung 7b (links) aus Fig. 11 und deren Lage im Querschnitt des Abklappmechanismus 2 (rechts) zwischen einem Innenschlauch 8a des Arbeitskanals 8 und einer Innenwand des Arbeitskanals 8 an der Keilrückenseite 6 der Segmente 4. Die Abgleitbandeinrichtung 7b ist gabelförmig ausgebildet und hat mehrere parallel verlaufende Längsstreifen als Längselemente 37, die an ihrem distalen Ende über einen Quersteg 38 miteinander verbunden sind. An einer Fixierstelle 33 des Querstegs 38 ist die Abgleitbandeinrichtung 7b mit dem distalen Bereich des Abklappmechanismus 2 verbunden. An einer dem Arbeitskanal 8 zugewandten Seite bilden die Längselemente 37 Abgleitflächen 36 für ein durch den Arbeitskanal 8 geschobenes Instrument aus.

Fig. 13 zeigt eine weitere bevorzugte Ausführungsform der Abgleitbandeinrichtung 7b (links) aus Fig. 11 und deren Lage im Querschnitt des Abklappmechanismus 2 (rechts) zwischen einem Innenschlauch 8a des Arbeitskanals 8 und einer Innenwand des Arbeitskanals 8 an der Keilrückenseite 6 der Segmente 4. Die Abgleitbandeinrichtung 7b wird durch mehrere parallel verlaufende Drähte 37 als Längselemente gebildet, die an ihrem distalen Ende jeweils eine Fixierstelle 33 ausbilden, über welche die Abgleitbandeinrichtung 7b mit dem distalen Bereich des Abklappmechanismus 2 verbunden ist. Insbesondere sind die Drähte 37 zumindest teilweise in Rillen in der Innenwand des Arbeitskanals 8 geführt. Die Drähte 37 können optional zusätzlich zu der rechteckigen/bandförmigen oder gabelförmigen Abgleitbandeinrichtung (hier in schematischer Darstellung zwischen zwei Gruppen der Drähte 37 angeordnet) bereitgestellt sein. An einer dem Arbeitskanal 8 zugewandten Seite bilden die Drähte 37 Abgleitflächen oder Abgleitlinien 36 für ein durch den Arbeitskanal 8 geschobenes Instrument aus.

Fig. 14 zeigt eine weitere bevorzugte Ausführungsform des Abklappmechanismus 2 der vorliegenden Offenbarung, welche im Wesentlichen der Ausführungsform nach Fig. 2 entspricht, mit nachfolgend erläuterten Unterschieden. Eine Breite des Schlitzes am Keilrückenabschnitt 6 der Segmente 4 entspricht einem Durchmesser des Arbeitskanals 8 (siehe Querschnittsansicht rechts). Ein Raum ist ausgebildet, welcher radial innen von einem Innenschlauch 8a des Arbeitskanals 8, radial außen von dem Verbindungs-/Rückenelement 7 und in Umfangsrichtung an einer dem Keilrückenabschnitt 6 abgewandten Seite von dem Segment 4 begrenzt wird. In diesem Raum ist eine Abgleitbandeinrichtung 7b derart eingelegt, dass sie sich (ähnlich wie die Abgleitbandeinrichtung 7b aus Fig. 11) entlang des Abklappmechanismus 2 erstreckt. Die Abgleitbandeinrichtung 7b ist links in einer Draufsicht dargestellt. Es bildet zwei parallel verlaufende Längselemente 37 aus, welche distal Fixierstellen 33 aufweisen, über welche die Längselemente 37 bzw. die Abgleitbandeinrichtung 7b mit dem distalen Bereich des Abklappmechanismus 2 verbunden ist. An einer dem Arbeitskanal 8 zugewandten Seite bilden die Längselemente 37 Abgleitflächen 36 für ein durch den Arbeitskanal 8 geschobenes Instrument aus.

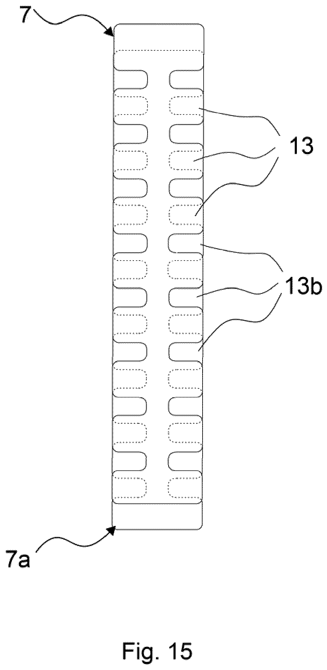

Fig. 15 zeigt einen Aspekt einer Modifikation der Ausführungen nach Fig. 1 bis Fig. 8, insbesondere nach Fig. 1. Genauer ist nach diesem Aspekt das Rückenelement/ die Verbindungsplatte 7 gemäß Fig. 3 (links, A) als ein erstes Rückenelement/ eine erste Verbindungsplatte 7 bereitgestellt. Ein zweites Rückenelement/ eine zweite Verbindungsplatte 7a (gestrichelt dargestellt, wo es/sie durch die erste Verbindungsplatte 7 verdeckt wird) ist bereitgestellt, welche derart in Endoskop-Längsrichtung versetzt zu der ersten Verbindungsplatte 7 angeordnet ist, dass die Laschen 13b der zweiten Verbindungsplatte 7a Zwischenräume zwischen den Laschen oder Befestigungsabschnitten 13 der ersten Verbindungsplatte 7 überlappen. Die Segmente 4 sind bevorzugt nur an den Laschen oder Befestigungsabschnitten 13 der ersten, im montierten Zustand radial äußeren Verbindungsplatte 7 angebracht oder sind alternativ bevorzugt wechselweise an den Laschen oder Befestigungsabschnitten 13 der ersten Verbindungsplatte 7 und an den Laschen 13b der zweiten Verbindungsplatte 7b angebracht.

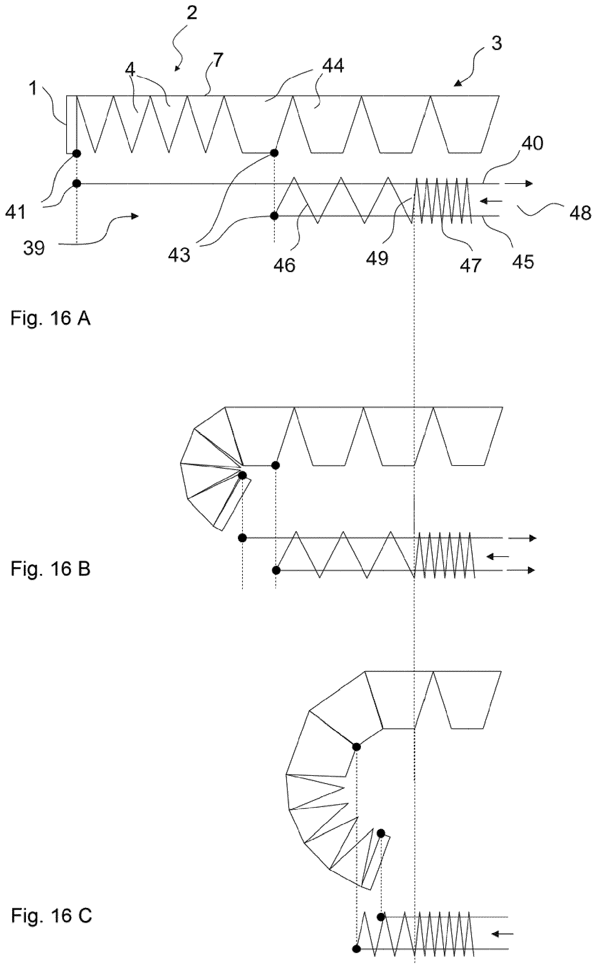

Fig. 16A bis Fig. 16C zeigen schematisch eine Betätigungsabfolge eines Betätigungsmechanismus zur Betätigung eines Endoskops nach einer der Ausführungsformen der Figuren 1 bis 15, wobei Bezugszeichen aus Übersichtsgründen nur in Fig. 16 A dargestellt sind. Aus Übersichtsgründen ist Abklappmechanismus 2 in dieser Darstellung nur schematisch dargestellt und weist ferner die Verbindungsplatte 7 und/oder die Abgleitbandeinrichtung 7b entsprechend einer der anderen beschriebenen Ausführungsformen auf. Im Falle der Verbindungsplatte 7 folgt diese im Wesentlichen den geraden Keilrückenabschnitten 6 der Segmente 4 bzw. Wirbelkörper 44 und ist nur an den dazwischenliegenden Scharnierabschnitten gekrümmt.

In Fig. 16A ist das Endoskop gemäß einer der vorstehenden Ausführungsformen in einer neutralen/ unbetätigten (gestreckten) Stellung dargestellt. Ein Betätigungsmechanismus 39 zum Betätigen/ Abkrümmen des Abklappmechanismus 2 und des Deflectings 3 ist parallel zu dem Endoskop dargestellt. Der Betätigungsmechanismus 39 hat einen Steuerdraht 40, dessen distales Ende an einem ersten Referenzpunkt 41 mit dem distalen Kopfabschnitt/ Endoskopkopf 1 verbunden (unmittelbar daran befestigt) ist.

Ferner hat der Betätigungsmechanismus 39 eine Betätigungsfeder 42, deren distales Ende an einem zweiten Referenzpunkt 43 mit einem am weitesten distal angeordneten Wirbelkörper 44 des Deflectings 3 verbunden (unmittelbar daran befestigt) ist. Ein distales Ende eines Sperrdrahts 45 des Betätigungsmechanismus 39 ist mit dem distalen Ende der Betätigungsfeder 42 verbunden (unmittelbar daran befestigt). Die Betätigungsfeder 42 hat einen distalen Federbereich 46 mit einer/m großen Steigung/ Windungsabstand und einen proximalen Federbereich 47 mit einer/m kleinen Steigung/Windungsabstand. Insbesondere ist ein Windungsabstand des proximalen Federbereichs 47 null, d. h. die Windungen der Betätigungsfeder 42 liegen aneinander an. Der Sperrdraht 45 sperrt/beschränkt eine Ausdehnung der Betätigungsfeder 42, insbesondere des distalen Federbereichs 46, und hält diese/n in einer komprimierten Stellung. Eine Stützfeder/Druckfeder 48 (in Figuren 16 A bis C symbolisch durch einen in Richtung distal weisenden Kraftpfeil dargestellt) stützt aus der proximalen Richtung die Betätigungsfeder 42.

Wird ausgehend von der neutralen/unbetätigten Stellung der Steuerdrahts 40 in proximale Richtung gezogen/betätigt (in Fig. 16 A durch einen in Richtung proximal weisenden Pfeil am Steuerdraht 40 dargestellt), wird der Abklappmechanismus 2, wie in Fig. 16 B dargestellt, abgeklappt/gekrümmt. Eine Vorspannkraft, welche durch den Sperrdraht 45 und/oder durch die Stützfeder/Druckfeder 48 auf den am weitesten distal angeordneten Wirbelkörper 44 des Deflectings 3 wirkt, verhindert eine Krümmung des Deflectings 3 aufgrund der Betätigung des Steuerdrahts 40.

Wird anschließend, wie in Fig. 16 B durch einen in Richtung proximal weisenden Pfeil am Sperrdraht 45 dargestellt, am Sperrdraht 45 innerhalb eines ersten Betätigungsbereichs in die proximale Richtung gezogen (wird dieser in der proximalen Richtung betätigt), wird zunächst der distale Federbereich 46 weiter komprimiert, wie in Fig. 16 C dargestellt, und werden somit die am weitesten distal angeordneten Wirbelkörper 44 des Deflectings 3 abgewinkelt. Insbesondere werden die Wirbelkörper 44 des Deflectings 3 abgewinkelt, welche sich distal von einem Übergangsbereich 49 zwischen dem proximalen Federbereich 47 und dem distalen Fehlerbereich 46 befinden. Im vorliegenden Beispiel wird gleichzeitig zu der Betätigung des Sperrdrahts 45 im ersten Betätigungsbereich der Steuerdraht 40 in die proximale Richtung gezogen (in Fig. 16 B durch einen in Richtung proximal weisenden Pfeil am Steuerdraht 40 dargestellt), um eine Längenänderung/Längenverschiebung des Steuerdrahts 40 aufgrund der Abwicklung des Deflectings 3 auszugleichen und die Krümmung des Abklappmechanismus 2 aufrechtzuerhalten.

Wird alternativ der Steuerdraht 40 während der Betätigung des Sperrdrahts 45 im ersten Betätigungsbereich nicht oder weniger weit die proximale Richtung gezogen, öffnet sich die Krümmung des Abklappmechanismus 2 ganz oder teilweise. Beispielsweise kann so eine Krümmung des Abklappmechanismus 2 in Verbindung mit den distalen Wirbelkörpern 44 des Deflectings 3 von 180° erreicht werden.

Wird anschließend, ausgehend von Fig. 16 C, der Sperrdraht 45 weiter in die proximale Richtung gezogen/betätigt, kommen die Windungen des distalen Federbereichs 46 in Anlage. Dies begrenzt den ersten Betätigungsbereich des Sperrdrahts 45. Wird anschließend der Sperrdraht von 45/ die Betätigungsfeder 42 weiter in der proximalen Richtung gezogen/betätigt (zweiter Betätigungsbereich), werden auch die am weitesten proximal angeordneten Wirbelkörper 44 des Deflectings 3 abgewinkelt (hier nicht dargestellt).

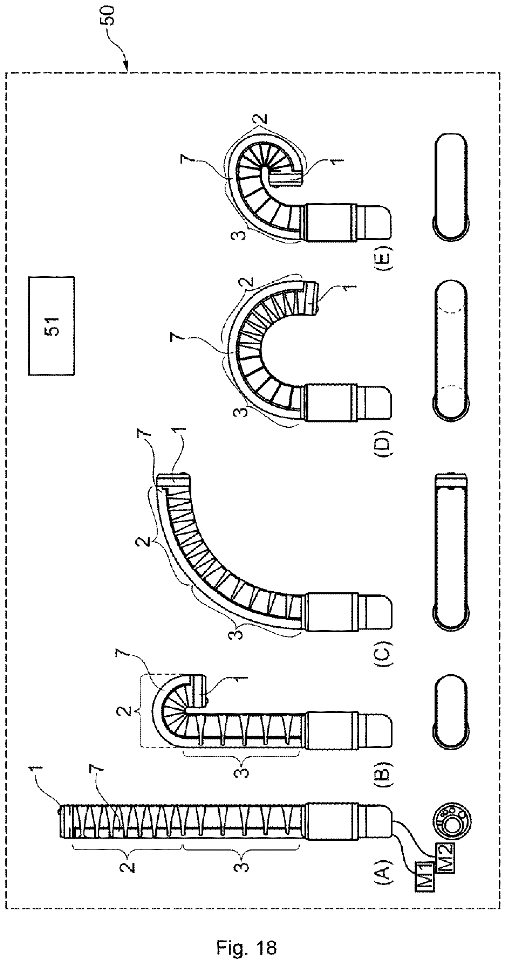

Fig. 18 zeigt ein Endoskop mit einem distalen Endoskop Kopf oder Kopfabschnitt 1, einem proximal dazu angeordneten Abklappmechanismus 2 und einem proximal dazu angeordneten Deflectingabschnitt (kurz: Deflecting) 3 in verschiedenen einstellbaren Modi oder Betriebszuständen. Von jedem Modus oder Betriebszustand ist eine Seitenansicht des Endoskops und eine Draufsicht auf das Endoskop aus einer Richtung, in welcher das Endoskop sich im gestreckten Zustand erstreckt, gezeigt. Insbesondere handelt es sich um ein Endoskop entsprechend einer der vorstehenden Figuren.

In einem neutralen Modus, welcher ganz links dargestellt und mit (A) gekennzeichnet ist, ist das Endoskop derart steuerbar, dass der Klappmechanismus 2 und das Deflecting 3, wie hier dargestellt, gestreckt sind. Optional können Sie sogar geringfügig überstreckt, d. h. leicht rückwärts in Richtung eines gegebenenfalls bereitgestellten Rückenelements 7 (siehe vorstehende Beschreibung) gebogen, sein.

In einem ersten Modus, gekennzeichnet mit (B), ist das Endoskop derart steuerbar, dass nur der Abklappmechanismus 2 gekrümmt ist, während das Deflecting 3 gestreckt beziehungsweise unbetätigt bleibt. Wie in der zugehörigen Draufsicht erkennbar ist, ist eine laterale Erstreckung des Endoskops in diesem Modus relativ gering.

In einem dritten Modus, hier gekennzeichnet mit (C) und (D), ist das Endoskop derart steuerbar, dass der Abklappmechanismus 2 und das Deflecting 3 synchron zueinander, d. h. im Wesentlichen gleichzeitig mit einem konstanten/ gleichen/ kontinuierlichen Krümmungsgrad krümmbar sind. In den zugehörigen Draufsichten ist erkennbar, dass eine laterale Erstreckung des Endoskops in diesem Modus relativ groß ist. Auf diese Weise kann das Endoskop in seinem distalen Bereich zum Beispiel eine 90°-Kurve (siehe Ansicht (C)) oder eine 180°-Kurve (siehe Ansicht (D)) erreichen. In letzterem Fall kann beispielsweise das Deflecting 3 maximal gekrümmt sein.

In einem vierten Modus, welcher ganz rechts dargestellt und mit (F) gekennzeichnet ist, ist das Deflecting 3 beispielsweise durch eine Steuerung im dritten Modus vollständig abgekrümmt und kann anschließend das Endoskop derart gesteuert werden, dass der Abklappmechanismus 2 weiter abkrümmbar ist, sodass eine hier dargestellte Stellung erreichbar ist, in welcher sowohl das Deflecting 3 als auch der Abklappmechanismus 2 maximal gekrümmt sind.

Das Endoskop kann Teil eines Behandlungssystems 50 sein, welches ferner ein Steuergerät/ eine Steuerungsvorrichtung 51 aufweist, welche dazu eingerichtet ist, das Endoskop wahlweise in einem der vorstehend beschriebenen Modi zu steuern.

Das Endoskop hat ferner zumindest einen Antrieb M1 zum Antreiben des Deflectings 3 und einen Antrieb M2 zum Antreiben des Abklappmechanismus 2, welche hier nur schematisch dargestellt sind. Die Steuervorrichtung 51 ist dazu eingerichtet, die Antriebe M1 und M2 je nach Modus unterschiedlich schnell und unabhängig voneinander anzutreiben.

### Bezugszeichenliste

- 1: Endoskopkopf / distaler Kopfabschnitt
- 2: Abklappmechanismus
- 3: Deflecting-Abschnitt
- 4: Segmente
- 4a, 4b: Teilsegmente
- 5: Keilspitzenabschnitt
- 6: Keilrückenabschnitt
- 7: Verbindungsplatte / Rückenelement
- 7a: Zweite/s Verbindungsplatte/ Rückenelement
- 7b: Flexible Abgleitplatte/ Abgleitbandeinrichtung
- 8: Arbeitskanal
- 8a: Innenschlauch
- 9: Nebenkanäle
- 10: Halteabschnitt
- 10a: Aufgeweiteter Halteabschnitt
- 11: Scharnierabschnitt
- 12: Scharnierachse
- 13: Befestigungsbereich/ Laschen
- 13a: Aufgeweiteter Befestigungsbereich
- 13b: Laschen des/r zweiten Rückenelements/ Verbindungsplatte
- 14: Deckbereich
- 15: Biegeachse
- 16: Betätigungselement
- 17: Rinnenmitte
- 18: Rinnenrand
- 19: Endoskophülle / Overtube
- 20: (passiver) Endoskopschaft
- 21: Segmentflügel
- 21a: Aufgeweiteter Segmentflügel
- 22a-e: Kanaleinstellstrukturen
- 23: Hüllschicht
- 24: Rücksprung
- 25: Öse
- 26: Riegelkanal
- 27: Riegeldraht
- 28: Innenschenkel
- 29: Aufnahmeraum
- 30: Auskleidung
- 31: Zusatzschlauch
- 32: Segmentgelenke
- 33: Klebe-, Schweiß- oder Lötstelle / Fixierstelle
- 34: Lücken zwischen Segmenten
- 35: Proximales Ende / proximaler Endbereich des Rückenelements
- 36: Abgleitfläche
- 37: Längsstreifen
- 38: Quersteg
- 39: Betätigungsmechanismus
- 40: Steuerdraht
- 41: Ersten Referenzpunkt
- 42: Betätigungsfeder
- 43: Zweiten Referenzpunkt
- 44: Wirbelkörper
- 45: Sperrdraht
- 46: Distaler Federbereich
- 47: Proximaler Federbereich
- 48: Stützfeder/Druckfeder
- 49: Übergangsbereich
- 50: Behandlungssystem
- 51: Steuerungsvorrichtung
- ΔS: Einschubdistanz

## Patentansprüche

1. Endoskop aufweisend einen Endoskopschaft, mit einem distalen manuell aktuierbaren Abklappmechanismus (2) zur kontrolliert abklapp- oder abwinkelbaren Halterung eines Endoskopkopfes oder distalen Kopfabschnitts (1) an einem dem Abklappmechanismus (2) proximal vorgelagerten Endoskopabschnitt (3, 20), wobei der Abklappmechanismus (2) eine Anzahl axial aufeinanderfolgender und mittels zumindest eines gemeinsamen Betätigungselements (16) aktiv gegeneinander winkelverstellbarer, im Wesentlichen zylindrischer Segmente (4) aufweist,
welche jeweils zwei im Wesentlichen keilförmig zueinander ausgerichtete axiale Stirnseiten und einen Zylindermantel aufweisen, der an einem Zylindermantelabschnitt mit maximaler axialer Länge einen Keilrückenabschnitt (6) definiert, und
welche in Endoskop-Längsrichtung jeweils zumindest eine Durchgangsöffnung aufweisen, derart, dass die Segmente (4) gemeinschaftlich einen Arbeitskanal (8) ausbilden,
**dadurch gekennzeichnet, dass** die Durchgangsöffnung des jeweiligen Segments (4) in dessen Keilrückenabschnitt (6) den Zylindermantel in Endoskop-Radialrichtung durchbricht und dabei eine Längsschlitz erzeugt, wobei
die Segmente (4) im Keilrückenabschnitt (6) durch eine flexible, vorzugsweise elastische, Verbindungsplatte (7) in Endoskop-Längsrichtung miteinander verbunden sind, welche unter Abdeckung der jeweiligen Längsschlitze an der jeweiligen Segmentaußenseite fixiert ist und zum einen eine im hierdurch definierten Arbeitskanal kontinuierlich verlaufende Abgleitfläche (36) für eingeführte medizinische Instrumente und zum anderen Scharnierabschnitte (11) zwischen den miteinander verbundenen Segmenten zum Abklappen zweier jeweils benachbarter Segmente (4) bildet.

2. Endoskop nach Anspruch 1, wobei die Verbindungsplatte (7) zumindest an den Scharnierabschnitten (11) federelastisch ausgebildet ist.

3. Endoskop nach Anspruch 2, wobei die Verbindungsplatte (7), insbesondere die Scharnierabschnitte (11), als ein Rückstellelement dienen, welches dazu ausgebildet ist, den Abklappmechanismus (2) aus einer abgeklappten Stellung in eine im Wesentlichen nach vorne ausgerichtete oder gestreckte Stellung zurückzustellen.

4. Endoskop nach einem der vorstehenden Ansprüche 2 oder 3, wobei die Scharnierabschnitte derart in Umfangsrichtung nach radial außen gewölbt oder gekrümmt sind, dass jeder Scharnierabschnitt eine in Endoskop-Längsrichtung verlaufende Rinne mit einem bestimmten, konstanten oder variablen, Rinnenradius bildet.

5. Endoskop nach einem der vorstehenden Ansprüche 1 bis 4, wobei die Verbindungsplatte (7) einen in Endoskop-Längsrichtung durchgehenden, glatten Mittenabschnitt bilden, welcher die Abgleitfläche (36) bildet, von dem symmetrisch beiderseits laschenförmige Befestigungsbereiche (13) abstehen, welche zur Befestigung der Segmente dienen und bezüglich des Mittenabschnitts aufeinander zu gebogen sind.

6. Endoskop nach Anspruch 5, wobei die Verbindungsplatte eine erste, radial äußere Verbindungsplatte (7) ist, der Abklappmechanismus (2) ferner eine zweite, radial innere Verbindungsplatte (7a) aufweist, welche einen sich in Endoskop-Längsrichtung erstreckenden zweiten Mittenabschnitt und davon seitlich vorstehende Laschen (13b) aufweist, und die zweite, radial innere Verbindungsplatte (7a) derart in Endoskop-Längsrichtung zu der ersten, radial äußeren Verbindungsplatte (7) versetzt angeordnet ist, dass die Laschen (13b) der zweiten, radial inneren Verbindungsplatte (7a) Zwischenräume zwischen den Befestigungsbereichen (13) der ersten, radial äußeren Verbindungsplatte (7) überdecken.

7. Endoskop nach einem der vorstehenden Ansprüche 1 bis 6, wobei der Abklappmechanismus (2) zur Halterung des Endoskopkopfes (1) am distalen Ende eines Endoskop-Deflectings (3) dient, das zwischen dem Abklappmechanismus und dem Endoskopschaft (20) zwischengefügt ist und mittels eines eigenen, separaten Betätigungselements abkrümmbar ist.

8. Endoskop nach Anspruch 7, wobei ein Biegeradius des Abklappmechanismus (2), wenn dieser vollständig abgeklappt ist, kleiner ist als ein Biegeradius des Endoskop-Deflectings (3), wenn dieses vollständig abgekrümmt ist.

9. Endoskop nach einem der vorstehenden Ansprüche, wobei ein Kanaleinstellmechanismus bereitgestellt ist, der zur Vergrößerung und/oder Verkleinerung eines Arbeitskanaldurchmessers konfiguriert ist, und der dadurch bereitgestellt wird, dass die die Segmente (4) im Keilrückenabschnitt (6) sowie die Verbindungsplatte (7) nach radial innen und/oder außen bezüglich der Endoskop-Längsrichtung aufbiegbar und/oder zubiegbar konfiguriert sind.

10. Endoskop nach Anspruch 9, wobei die Verbindungsplatte (7) Kanaleinstellstrukturen (22a-e) aufweist, welche die Biegesteifigkeit der Verbindungsplatte (7) lokal verringern, derart, dass die Verbindungsplatte (7) definiert nach radial innen und/oder außen bezüglich der Endoskop-Längsrichtung aufbiegbar und/oder zubiegbar konfiguriert ist.

11. Endoskop nach einem der vorstehenden Ansprüche, wobei jedes Segment (4) zwei Teilsegmente (4a, 4b) aufweist, die über die Verbindungsplatte (7) miteinander verbunden sind, welche als eine Spreizfeder konfiguriert und dazu ausgebildet ist, um einen Spalt zwischen den Teilsegmenten (4a, 4b) wahlweise derart aufzuspreizen, dass dazwischen ein Raum für einen Zusatz-Arbeitskanal (32) bereitgestellt ist.

12. Endoskop aufweisend einen Endoskopschaft, mit einem distalen manuell aktuierbaren Abklappmechanismus (2) zur kontrolliert abklapp- oder abwinkelbaren Halterung eines Endoskopkopfes oder distalen Kopfabschnitts (1) an einem dem Abklappmechanismus (2) proximal vorgelagerten Endoskopabschnitt (3, 20), wobei der Abklappmechanismus (2) eine Anzahl axial aufeinanderfolgender und mittels zumindest eines gemeinsamen Betätigungselements (16) aktiv gegeneinander winkelverstellbarer, im Wesentlichen zylindrischer Segmente (4) aufweist,
welche jeweils zwei im Wesentlichen keilförmig zueinander ausgerichtete axiale Stirnseiten und einen Zylindermantel aufweisen, der an einem Zylindermantelabschnitt mit maximaler axialer Länge einen Keilrückenabschnitt (6) definiert, an welchem die Segmente (4) aneinander angelenkt sind, und
welche in Endoskop-Längsrichtung jeweils zumindest eine Durchgangsöffnung aufweisen, derart, dass die Segmente (4) gemeinschaftlich einen Arbeitskanal (8) mit einer Arbeitskanalwand ausbilden,
**dadurch gekennzeichnet, dass** in den Arbeitskanal (8) ein flexibles, sich in der Endoskop-Längsrichtung entlang mehrerer Segmente (4), vorzugsweise entlang des gesamten Abklappmechanismus (2), erstreckende Abgleitbandeinrichtung (7b) derart eingelegt ist, dass die Abgleitbandeinrichtung (7b) einen keilrückenseitigen Abschnitt der Arbeitskanalwand der jeweiligen Segmente (4) bedeckt und eine durchgängige, glatte Abgleitfläche (36) für in den Arbeitskanal (8) eingeführte medizinische Instrumente bildet.

13. Endoskop nach Anspruch 12, wobei die Abgleitbandeinrichtung (7b) an einem distalen Endbereich des Abklappmechanismus (2), insbesondere an dem Endoskopkopf oder distalen Kopfabschnitt (1) oder an einem am weitesten distal angeordneten Segment (4), fixiert ist und in der Endoskop-Längsrichtung verschiebbar in den Durchgangsöffnungen einer Anzahl von weiter proximal angeordneten Segmenten (4) liegt, derart, dass die Abgleitbandeinrichtung (7b) beim Abkrümmen des Abklappmechanismus (2) in dem Arbeitskanal (8) verrutschen kann, um eine Dehnung oder Stauchung der Abgleitbandeinrichtung (7b) in Endoskop-Längsrichtung im Wesentlichen zu vermeiden.

14. Endoskop nach einem der Ansprüche 12 bis 13, wobei die Abgleitbandeinrichtung (7b) elastisch ist und in einer Ruhestellung im Wesentlichen gerade verläuft, sodass die Abgleitbandeinrichtung (7b) beim Rückstellen des Abklappmechanismus aus einer abgeklappten Stellung in eine im Wesentlichen nach vorne ausgerichtete oder gestreckte Stellung in die gerade, ungebogene Ruhestellung strebt.

15. Endoskop nach einem der Ansprüche 12 bis 14, wobei die Abgleitbandeinrichtung (7b) mehrere Längselemente (37) aufweist, welche sich in Endoskop-Längsrichtung, insbesondere parallel zu einander, erstrecken.

## Claims

1. An endoscope having an endoscope shaft, with a distal manually actuable folding mechanism (2) for the controlled folding or bending mounting of an endoscope head or distal head section (1) on an endoscope section (3, 20) located proximally upstream of the folding mechanism (2), wherein the folding mechanism (2) has a number of axially successive substantially cylindrical segments (4) which can be actively angularly adjusted relative to one another by means of at least one common actuating element (16)
which each have two axial end faces aligned substantially wedge-shaped with respect to one another and a cylinder jacket which defines a wedge back section (6) at a cylinder jacket section with maximum axial length, and
which each have at least one through opening in the longitudinal direction of the endoscope in such a way that the segments (4) jointly form a working channel (8),
**characterised in that** the through-opening of the respective segment (4) in its wedge back section (6) breaks through the cylinder jacket in the endoscope radial direction and thereby produces a longitudinal slot, wherein
the segments (4) in the wedge back section (6) are connected to one another in the longitudinal direction of the endoscope by a flexible, preferably elastic, connecting plate (7) which is fixed to the respective outer side of the segment while covering the respective longitudinal slits and forms, on the one hand, a sliding surface (36) for inserted medical instruments which runs continuously in the working channel defined thereby and, on the other hand, hinge sections (11) between the segments connected to one another for folding down two respective neighbouring segments (4).

2. The endoscope according to claim 1, wherein the connecting plate (7) is designed to be resilient at least at the hinge sections (11).

3. The endoscope according to claim 2, wherein the connecting plate (7), in particular the hinge portions (11), serve as a return element which is designed to return the folding mechanism (2) from a folded-down position to a substantially forwardly orientated or extended position.

4. The endoscope according to one of the preceding claims 2 or 3, wherein the hinge portions are curved or bent radially outwards in circumferential direction in such a way that each hinge portion forms a groove extending in the longitudinal direction of the endoscope with a certain, constant or variable, groove radius.

5. The endoscope according to one of the preceding claims 1 to 4, wherein the connecting plate (7) forms a smooth centre section which is continuous in the longitudinal direction of the endoscope and forms the sliding surface (36), from which tab-shaped fastening regions (13) protrude symmetrically on both sides, which serve to fasten the segments and are bent towards each other with respect to the centre section.

6. The endoscope according to claim 5, wherein the connecting plate is a first, radially outer connecting plate (7), the folding mechanism (2) further comprises a second, radially inner connecting plate (7a) which has a second centre portion extending in the longitudinal direction of the endoscope and tabs (13b) projecting laterally therefrom, and the second, radially inner connecting plate (7a) has a second centre portion extending in the longitudinal direction of the endoscope and tabs (13b) projecting laterally therefrom, radially inner connecting plate (7a) is arranged offset in the longitudinal direction of the endoscope relative to the first, radially outer connecting plate (7) in such a way that the tabs (13b) of the second, radially inner connecting plate (7a) cover intermediate spaces between the fastening regions (13) of the first, radially outer connecting plate (7).

7. The endoscope according to one of the preceding claims 1 to 6, wherein the folding mechanism (2) serves to hold the endoscope head (1) at the distal end of an endoscope deflector (3), which is interposed between the folding mechanism and the endoscope shaft (20) and can be bent by means of its own, separate actuating element.

8. The endoscope according to claim 7, wherein a bending radius of the folding mechanism (2), when the latter is completely folded down, is smaller than a bending radius of the endoscope deflector (3), when the latter is completely bent down.

9. The endoscope according to any of the preceding claims, wherein a channel adjustment mechanism is provided which is configured to increase and/or decrease a working channel diameter, and which is provided in that the segments (4) in the wedge back portion (6) as well as the connecting plate (7) are configured to be bendable and/or bendable radially inwards and/or outwards with respect to the endoscope longitudinal direction.

10. The endoscope according to claim 9, wherein the connecting plate (7) has channel adjustment structures (22a-e) which locally reduce the bending stiffness of the connecting plate (7) such that the connecting plate (7) is configured such that it can be bent open and/or bent closed in a defined manner radially inwards and/or outwards with respect to the longitudinal direction of the endoscope.

11. The endoscope according to one of the preceding claims, wherein each segment (4) comprises two partial segments (4a, 4b) which are connected to each other via the connecting plate (7), which is configured as an expanding spring and is designed to selectively expand a gap between the partial segments (4a, 4b) in such a way that a space for an additional working channel (32) is provided therebetween.

12. The endoscope having an endoscope shaft, with a distal manually actuable folding mechanism (2) for the controlled folding or bending mounting of an endoscope head or distal head section (1) on an endoscope section (3, 20) located proximally in front of the folding mechanism (2), wherein the folding mechanism (2) has a number of axially successive substantially cylindrical segments (4) which can be actively angularly adjusted relative to one another by means of at least one common actuating element (16),
which each have two axial end faces aligned substantially wedge-shaped relative to one another and a cylindrical jacket which defines a wedge back section (6) at a cylindrical jacket section with maximum axial length, at which the segments (4) are articulated to one another, and
which each have at least one through-opening in the longitudinal direction of the endoscope in such a way that the segments (4) jointly form a working channel (8) with a working channel wall,
**characterised in that** a flexible slide-off strip device (7b) extending in the longitudinal direction of the endoscope along several segments (4), preferably along the entire folding mechanism (2), is inserted into the working channel (8) in such a way that the slide-off strip device (7b) covers a wedge-backed portion of the working channel wall of the respective segments (4) and forms a continuous, smooth slide-off surface (36) for medical instruments inserted into the working channel (8).

13. The endoscope according to claim 12, wherein the sliding band device (7b) is fixed to a distal end region of the folding mechanism (2), in particular to the endoscope head or distal head section (1) or to a segment (4) arranged furthest distally, and lies displaceably in the longitudinal direction of the endoscope in the through-openings of a number of segments (4) arranged further proximally, such that the sliding band device (7b) can slip in the working channel (8) during bending of the folding mechanism (2) in order to substantially avoid stretching or compression of the sliding band device (7b) in the longitudinal direction of the endoscope.

14. The endoscope according to one of claims 12 to 13, wherein the slide-off band device (7b) is elastic and extends substantially straight in a rest position, so that the slide-off band device (7b) strives into the straight, unbent rest position when the fold-down mechanism is reset from a folded-down position into a substantially forwardly orientated or stretched position.

15. The endoscope according to one of claims 12 to 14, wherein the slide-off band device (7b) comprises a plurality of longitudinal elements (37) which extend in the longitudinal direction of the endoscope, in particular parallel to each other.

## Revendications

1. Endoscope présentant une tige d'endoscope, comprenant un mécanisme de rabattement (2) distal actionnable manuellement destiné au support rabattable ou inclinable commandé d'une tête d'endoscope ou d'une section de tête distale (1) au niveau d'une section d'endoscope (3, 20) placée de manière proximale en amont par rapport au mécanisme de rabattement (2), dans lequel le mécanisme de rabattement (2) présente un certain nombre de segments (4) sensiblement cylindriques axialement successifs et réglables angulairement de manière active les uns par rapport aux autres au moyen d'au moins un élément d'actionnement (16) commun,
qui présentent respectivement deux faces frontales axiales sensiblement en forme de coin orientées l'une par rapport à l'autre et une enveloppe cylindrique qui définit une section arrière de coin au niveau d'une section d'enveloppe cylindrique avec une longueur axiale maximale, et
qui présentent dans la direction longitudinale de l'endoscope respectivement au moins une ouverture de passage, de telle sorte que les segments (4) forment ensemble un canal de travail (8),
**caractérisé en ce que** l'ouverture de passage du segment (4) respectif perce dans sa section arrière de coin (6) l'enveloppe cylindrique dans la direction radiale de l'endoscope et produit ainsi une fente longitudinale, dans lequel
les segments (4) sont reliés les uns aux autres dans la section arrière de coin (6) par une plaque de liaison (7) souple, de préférence élastique dans la direction longitudinale de l'endoscope, qui est fixée au niveau de la face extérieure respective du segment en recouvrant les fentes longitudinales respectives et constitue, d'une part, une surface de glissement (36) s'étendant en continu dans le canal de travail ainsi défini pour des instruments médicaux qui y sont introduits et, d'autre part, des sections formant charnières (11) entre les segments reliés entre eux pour rabattre deux segments (4) respectivement adjacents.

2. Endoscope selon la revendication 1, dans lequel la plaque de liaison (7) est formée de manière élastique au moins au niveau des sections formant charnières (11).

3. Endoscope selon la revendication 2, dans lequel la plaque de liaison (7), en particulier les sections formant charnières (11) servent d'élément de rappel, qui est formé pour ramener le mécanisme de rabattement (2) d'une position rabattue à une position orientée sensiblement vers l'avant ou étirée.

4. Endoscope selon l'une quelconque des revendications 2 ou 3, dans lequel les sections formant charnières sont bombées ou courbées radialement vers l'extérieur dans la direction périphérique, de telle sorte que chaque section formant charnière constitue une gouttière s'étendant dans la direction longitudinale de l'endoscope avec un rayon de gouttière déterminé, constant ou variable.

5. Endoscope selon l'une quelconque des revendications 1 à 4, dans lequel la plaque de liaison (7) constitue une section centrale lisse, continue dans la direction longitudinale de l'endoscope, qui constitue la surface de glissement (36), à partir de laquelle des zones de fixation (13) en forme de languette font saillie symétriquement des deux côtés, qui servent de fixation aux segments et sont courbées les unes sur les autres par rapport à la section centrale.

6. Endoscope selon la revendication 5, dans lequel la plaque de liaison est une première plaque de liaison (7) radialement extérieure, le mécanisme de rabattement (2) présente en outre une seconde plaque de liaison (7a) radialement intérieure qui présente une seconde section centrale s'étendant dans la direction longitudinale de l'endoscope et des languettes (13b) faisant saillie latéralement depuis celle-ci, et la seconde plaque de liaison (7a) radialement intérieure est disposée de manière décalée dans la direction longitudinale de l'endoscope par rapport à la première plaque de liaison (7) radialement extérieure, de telle sorte que les languettes (13b) de la seconde plaque de liaison (7a) radialement intérieure recouvrent des espaces intermédiaires entre les zones de fixation (13) de la première plaque de liaison (7) radialement extérieure.

7. Endoscope selon l'une quelconque des revendications 1 à 6, dans lequel le mécanisme de rabattement (2) sert au support de la tête d'endoscope (1) au niveau de l'extrémité distale d'un déflecteur d'endoscope (3) qui est intercalé entre le mécanisme de rabattement et la tige d'endoscope (20) et peut être courbé au moyen d'un élément d'actionnement propre, distinct.

8. Endoscope selon la revendication 7, dans lequel un rayon de courbure du mécanisme de rabattement (2), lorsque celui-ci est entièrement rabattu, est plus petit qu'un rayon de courbure du déflecteur d'endoscope (3), lorsque celui-ci est entièrement courbé.

9. Endoscope selon l'une quelconque des revendications précédentes, dans lequel un mécanisme de réglage de canal est prévu, qui est configuré pour l'augmentation et/ou la diminution d'un diamètre de canal de travail, et qui est prévu en ce que les segments (4) dans la section arrière de coin (6), ainsi que la plaque de liaison (7) sont configurés de manière à pourvoir être pliés et/ou dépliés radialement vers l'intérieur et/ou vers l'extérieur par rapport à la direction longitudinale de l'endoscope.

10. Endoscope selon la revendication 9, dans lequel la plaque de liaison (7) présente des structures de réglage de canal (22a-e), qui réduisent localement la résistance à la flexion de la plaque de liaison (7), de telle sorte que la plaque de liaison (7) est configurée de manière à pouvoir être pliée et/ou dépliée radialement vers l'intérieur et/ou l'extérieur par rapport à la direction longitudinale de l'endoscope.

11. Endoscope selon l'une quelconque des revendications précédentes, dans lequel chaque segment (4) présente deux segments partiels (4a, 4b) qui sont reliés entre eux par le biais de la plaque de liaison (7), qui est configurée comme un ressort d'écartement et formée pour écarter sélectivement une fente entre les segments partiels (4a, 4b), de telle sorte qu'un espace est prévu entre eux pour un canal de travail supplémentaire (32).

12. Endoscope présentant une tige d'endoscope, comprenant un mécanisme de rabattement (2) distal actionnable manuellement destiné au support rabattable ou inclinable commandé d'une tête d'endoscope ou d'une section de tête distale (1) au niveau d'une section d'endoscope (3, 20) placée de manière proximale en amont par rapport au mécanisme de rabattement (2), dans lequel le mécanisme de rabattement (2) présente un certain nombre de segments (4) sensiblement cylindriques axialement successifs et réglables angulairement de manière active les uns par rapport aux autres au moyen d'au moins un élément d'actionnement (16) commun,
qui présentent respectivement deux faces frontales axiales sensiblement en forme de coin orientées l'une par rapport à l'autre et une enveloppe cylindrique, qui définit une section arrière de coin au niveau d'une section d'enveloppe cylindrique avec une longueur axiale maximale, au niveau de laquelle les segments (4) sont articulés les uns aux autres, et
qui présentent dans la direction longitudinale de l'endoscope respectivement au moins une ouverture de passage, de telle sorte que les segments (4) forment ensemble un canal de travail (8) avec une paroi de canal de travail,
**caractérisé en ce qu'** un appareil de bande de glissement (7b) flexible, s'étendant dans la direction longitudinale de l'endoscope le long de plusieurs segments (4), de préférence le long de l'ensemble du mécanisme de rabattement (2) est inséré dans le canal de travail (8) de telle sorte que l'appareil de bande de glissement (7b) recouvre une section côté arrière de coin de la paroi de canal de travail des segments (4) respectifs et forme une surface de glissement (36) continue, lisse pour les instruments médicaux introduits dans le canal de travail (8).

13. Endoscope selon la revendication 12, dans lequel l'appareil de bande de glissement (7b) est fixé au niveau d'une zone d'extrémité distale du mécanisme de rabattement (2), en particulier au niveau de la tête d'endoscope ou la section de tête distale (1) ou au niveau d'un segment (4) disposé le plus distalement possible et se trouve de manière mobile dans la direction longitudinale de l'endoscope dans les ouvertures de passage d'un certain nombre de segments (4) disposés de manière plus proximale possible, de telle sorte que l'appareil de bande de glissement (7b) peut glisser dans le canal de travail (8) lors du pliage du mécanisme de rabattement (2) pour éviter sensiblement un étirement ou une compression de l'appareil de bande de glissement (7b) dans la direction longitudinale de l'endoscope.

14. Endoscope selon l'une quelconque des revendications 12 à 13, dans lequel l'appareil de bande de glissement (7b) est élastique et s'étend de manière sensiblement rectiligne dans une position de repos, de sorte que, lorsque le mécanisme de rabattement est ramené d'une position rabattue à une position orientée sensiblement vers l'avant ou étendue, l'appareil de bande de glissement (7b) tend vers la position de repos rectiligne, non courbée.

15. Endoscope selon l'une quelconque des revendications 12 à 14, dans lequel l'appareil de bande de glissement (7b) présente plusieurs éléments longitudinaux (37) qui s'étendent en particulier parallèlement les uns aux autres dans la direction longitudinale de l'endoscope.
